(19) Europäisches Patentamt
European Patent Office
Office européen des brevets

(11) Numéro de publication: **0 018 894**
**B1**

# (12) FASCICULE DE BREVET EUROPEEN

(45) Date de publication du fascicule de brevet: **17.02.82**

(21) Numéro de dépôt: **80400557.7**

(22) Date de dépôt: **24.04.80**

(51) Int. Cl.[3]: **C 07 C 69/743**, C 07 C 69/612, A 01 N 53/00, A 01 N 37/10, A 61 K 31/215, C 07 C 67/08, C 07 D 307/54, C 07 D 333/24, C 07 C 121/48

(54) **Esters de dérivés de l'alléthrolone et d'acides carboxyliques, leur procédé de préparation et les compositions les renfermant.**

(30) Priorité: **26.04.79 FR 7910651**

(43) Date de publication de la demande: **12.11.80 Bulletin 80/23**

(45) Mention de la délivrance du brevet: **17.02.82 Bulletin 82/7**

(84) Etats contractants désignés: **AT BE CH DE FR GB IT LI LU NL SE**

(56) Documents cités: **FR-A-2 355 797**

(73) Titulaire: **ROUSSEL-UCLAF, 35, boulevard des Invalides, F-75007 Paris (FR)**

(72) Inventeur: **Martel, Jacques, 15, rue Douvillez, F-93140 Bondy (FR)**
Inventeur: **Tessier, Jean, 30, rue Jean Moulin, F-94300 Vincennes (FR)**
Inventeur: **Teche, André, 51, avenue Joliot Curie, F-92000 Nanterre (FR)**

(74) Mandataire: **Douetteau, Pierre et al, c/o ROUSSEL-UCLAF 102, route de Noisy Boite postale 9, F-93230 Romainville (FR)**

**EP 0 018 894 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Esters de dérivés de l'alléthrolone et d'acides carboxyliques, leur procédé des préparation et les compositions les renfermant

La présente invention concerne des esters de dérivés de l'alléthrolone et d'acides carboxyliques, leur procédé de préparation et les compositions les renfermant.

L'invention a pour objet sous toutes leurs formes isomères possibles, les composés de formule générale (I'):

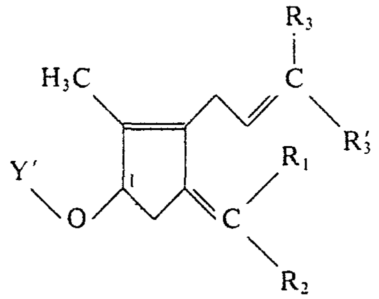

(I')

dans laquelle $R_1$ et $R_2$, identiques ou différents, représentent un groupement carbamoyle ou bien des groupements $R'_1$ et $R'_2$, lesquels, identiques ou différents, représentent un atome d'hydrogène, un atome d'halogène, un radical alcoyle comportant de 1 à 6 atomes de carbone, un radical aryle comportant de 6 à 10 atomes de carbone, un radical aralcoyle comportant des 7 à 13 atomes de carbone, un groupement alcoyloxycarbonyle comportant des 2 à 5 atomes de carbone ou un groupement cyano, $R_3$ et $R'_3$, identiques ou différents, représentent un atome d'hydrogène, un halogène ou un radical alcoyle saturé ou insaturé, comportant de 1 à 3 atomes de carbone, Y' représente ou bien un groupement:

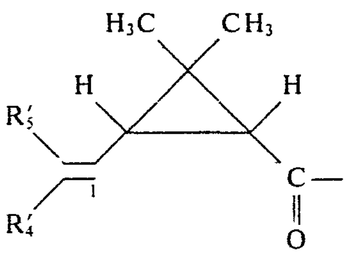

(A')

dans lequel soit $R'_4$ et $R'_5$, identiques ou différents, représentent un atome d'hydrogène, un radical alcoyle comportant de 1 à 4 atomes de carbone, un atome de fluor, de chlore ou de brome, soit $R'_4$ et $R'_5$ représentent ensemble avec l'atome de carbone auquel ils sont liés, un homocycle carboné ou un hétérocycle, comportant de 3 à 7 chaînons, ces cycles pouvant être éventuellement insaturés, ou bien un hétérocycle de structure:

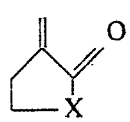

dans lequel X représente un atome d'oxygène ou de soufre, soit l'un des groupes $R'_4$ ou $R'_5$ représente un groupement cyano, un groupement

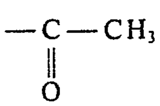 un groupement 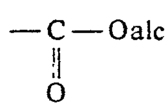

alc représentant un radical alcoyle renfermant de 1 à 4 atomes de carbone, lorsque l'autre représente un atome d'hydrogène, soit $R'_4$ représente un groupement cyano et $R'_5$ un radical phényle, les substituants du cycle cyclopropanique pouvant être de configuration cis ou trans, racémique ou optiquement active, la double liaison en position 1 de la chaîne latérale vinylique pouvant être de configuration (E) ou (Z) ou bien Y' représente un groupement:

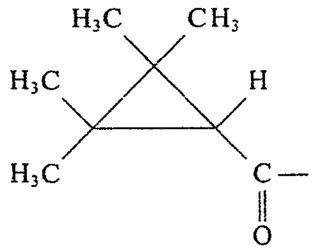

(B)

ou bien Y' représente un groupement:

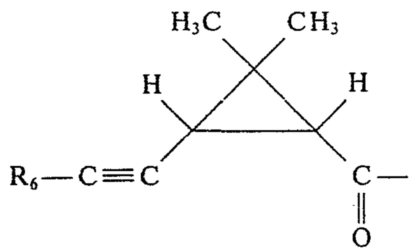

(C)

dans lequel $R_6$ représente un atome d'hydrogène, un radical alcoyle comportant de 1 à 4 atomes de carbone ou un groupement alcoxycarbonyle comportant de 2 à 5 atomes de carbone, les substituants du cycle cyclopropanique pouvant être de configuration cis ou trans, racémique ou optiquement active, ou bien Y' représente un groupement:

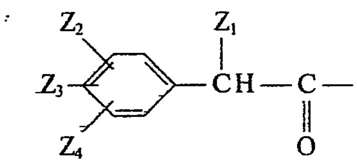

(D)

dans lequel $Z_1$ représente un radical alcoyle comportant de 1 à 4 atomes de carbone, $Z_2$, $Z_3$ et $Z_4$, identiques ou différents, représentent un atome d'hydrogène, un radical alcoyle comportant de 1 à 4 atomes de carbone, un radical alcoyloxyle comportant de 1 à 4 atomes de carbone, un radical alcoylthio comportant de 1 à 4 atomes de carbone, ou un atome d'halogène, le carbone asymétrique du groupement (D) pouvant être de configuration S, R ou racémique et le carbone asymétrique en position 1 du dérivé de l'alléthrolone pouvant être de configuration R, S ou racémique:

Parmie les composés de formule générale I', on peut citer notamment les composés répondant à la formule générale I:

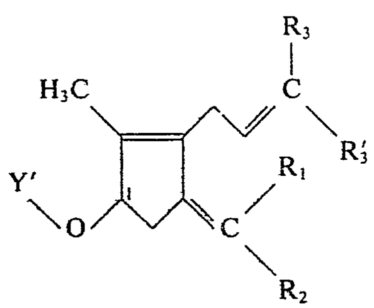

(I')

dans laquelle $R_1$, $R_2$, $R_3$ sont définis comme précédemment et Y représente ou bien un groupement:

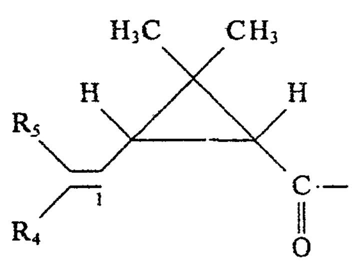

(A)

dans lequel, soit $R_4$ et $R_5$, identiques ou différents, représentent un atome d'hydrogène, un radical alcoyle comportant de 1 à 4 atomes de carbone, un atome de fluor, de chlore ou de brome, soit $R_4$ et $R_5$

représentant ensemble avec l'atome de carbone auquel ils sont liés, un homocycle carboné ou un hétérocycle comportant de 3 à 7 chaînons, ces cycles pouvant être éventuellement insaturés, ou bien un hétérocycle de structure:

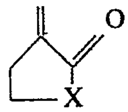

dans lequel X représente un atome d'oxygène ou de soufre, soit l'un des groupes $R_4$ ou $R_5$ représente un groupement cyano, un groupement

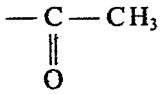 un groupement 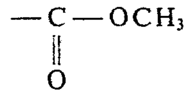

lorsque l'autre représente un atome d'hydrogène, les substituants du cycle cyclopropanique pouvant être de configuration cis ou trans, racémique ou optiquement active, la double liaison en position 1 de la chaîne latérale vinylique pouvant être de configuration (E) ou (Z), ou bien Y représente un groupement:

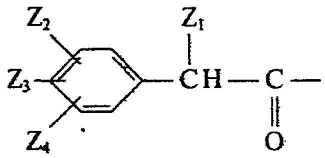 (D)

tel que défini précédemment, le carbone asymétrie en position 1 du dérivé de l'alléthrolone pouvant être de configuration R, S ou racémique.

L'invention a plus particulièrement pour objet les composés de formule générale I, dans lesquels Y représente un groupement:

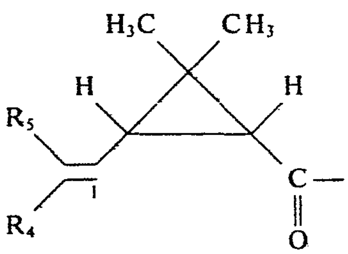 (A)

les composés de formule générale I, dans lesquels Y représents un groupement:

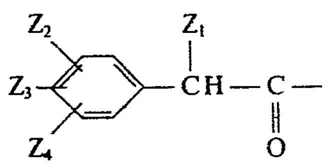 (D)

les composés de formule générale I, dans lesquels Y représente un groupement A, $R_1$ et $R_2$, représent un atome d'halogène, $R_3$ et $R'_3$ représentent un atome d'hydrogène et $R_4$ et $R_5$ représentent un atome d'halogène, les composés de formule générale I, dans lesquels Y représente un groupement A, $R_1$, $R_2$, $R_3$ et $R'_3$ représentent de l'hydrogène et $R_4$ et $R_5$ représentent ensemble avec l'atome de carbone auquel ils sont liés, un homocycle carboné, les composés de formule générale I, dans lesquels Y représente un groupement A, $R_1$, $R_2$, $R_3$ et $R'_3$ représentent de l'hydrogène et $R_4$ et $R_5$ représentent un atome d'halogene.

L'invention a notamment pour objet des composés de formule (I) décrits dans la partie expérimentale et plus particulièrement

le (1R,trans) 2,2-diméthyl 3-cyclopentylidène méthyl cyclopropane-1-carboxylate de (1S)2-méthyl 3-allyl 4-méthylène cyclopent-2-èn 1-yle,

le (1R,trans)2,2-diméthyl 3-(2,2-difluoroéthényl)cyclopropane-1-carboxylate de (1S)2-méthyl 3-allyl 4-méthylène cyclopent-2-èn 1-yle,

le (1R,trans)2,2-diméthyl 3-(2,2-dichloroéthényl)cyclopropane-1-carboxylate de (1S)2-méthyl 3-allyl 4-méthylène cyclopent-2-èn 1-yle,

le (1R,trans) 2,2-diméthyl 3-/2(Z)cyano éthényl/cyclopropane-1-carboxylate de (1S)2-méthyl 3-allyl 4-méthylène cyclopent-2-èn 1-yle,

le (1R,trans) 2,2-diméthyl 3-/2-fluoro 2-chloro éthényl/cyclopropane-1-carboxylate de

(1S)2-méthyl 3-allyl 4-méthylène cyclopent-2-èn 1-yle,
le (1R,trans) 2,2-diméthyl 3-cyclobutylidène méthyl cyclopropane-1-carboxylate de
(1S)2-méthyl 3-allyl 4-méthylène cyclopent-2-èn 1-yle et
le (1R,cis) 2,2-diméthyl 3-éthynyl cyclopropane-1-carboxylate de
(1S)2-méthyl 3-allyl 4-méthylène cyclopent-2-èn 1-yle.

Dans les composés de l'invention, $R_1$ et $R_2$ représentent, notamment un atome d'hydrogène, de fluor, de chlore ou de brome, un radical méthyle, éthyle, propyle, isopropyle, butyle, linéaire ou ramifié, pentyle, linéaire ou ramifié, hexyle, linéaire ou ramifié, un groupement alcoyloxycarbonyle dans lequel le radical alcoyle représente un radical méthyle, éthyle, propyle ou isopropyle, butyle, linéaire ou ramifié, un groupement carbamoyle, un radical phényle, naphthyle, un groupement cyano: $R_3$ et $R'_3$, représentent notamment un atome d'hydrogène, un radical méthyle, éthyle, propyle, isopropyle, un radical vinyle, propen-1-yle, allyle; $R'_4$ et $R'_5$, représentent notamment un atome d'hydrogène, un radical méthyle, éthyle, propyle ou isopropyle, un atome de fluor, de chlore ou de brome ou $R'_4$ et $R'_5$ représentent ensemble avec l'atome de carbone auquel ils sont liés, soit un cyclopropyle, un cyclobutyle, un cyclopentyle, un cyclohexyle ou un cycloheptyle, soit un hétérocycle de 3 à 7 chaînons, éventuellement insaturé et en particulier ceux de structure:

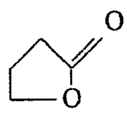 ou de structure 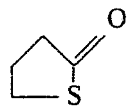

$Z_1$ représente notamment un radical méthyle, éthyle, propyle, isopropyle, butyle linéaire ou ramifié, $Z_2$, $Z_3$ et $Z_4$ représentent un atome d'hydrogène, un radical méthyle, éthyle, propyle, isopropyle, butyle linéaire ou ramifié, un radical alcoyloxyle dont le groupement alcoyle est un méthyle, un éthyle, un propyle, un isopropyle, un butyle linéaire ou ramifié, un radical alcoylthio dans lequel le radical alcoyle est un méthyle, un éthyle, un propyle, un isopropyle ou un butyle linéaire ou ramifié, un atome de fluor, de chlore, de brome ou d'iode.

L'invention a également pour objet un procédé de préparation des composés de formule générale (I), caractérisé en ce que l'on fait réagir un composé de formule générale II, de configuration (S), (R) ou racémique:

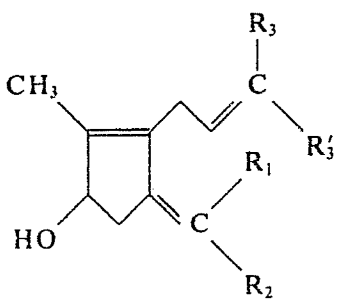

(II)

dans laquelle $R_1$, $R_2$, $R_3$, et $R'_3$ conservent les significations précitées, ou un dérivé fonctionnel de ce composé (II), avec un acide carboxylique de formule générale (III):

$$Y'—OH \qquad (III)$$

dans laquelle Y' conserve la signification précitée, ou avec un dérivé fonctionnel de cet acide III, tel qu'un halogénure, und anhydride, und anhydride mixte ou un sel de base minérale ou organique.

Selon un des modes d'exécution de ce procédé, le dérivé fonctionnel de l'acide est le chlorure et l'estérification est effectuée au sein du benzène en présence de pyridine.

Le dérivé fonctionnel du composé (II) est notamment un sel métallique tel qu'un sel alcalin ou un sel d'argent ou bien un sel de base organique.

L'invention a aussi pour objet un procédé de préparation des composés de formule générale (I'), caractérisé en ce que l'on soumet, un composé de formule générale IV:

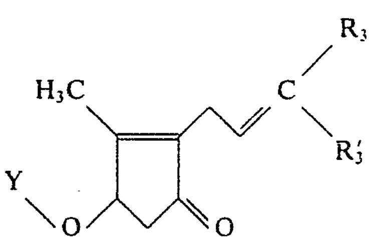

(IV)

dans laquelle $R_3$, $R'_3$ et Y' conservent les significations précitées, à l'action d'un réactif de formule:

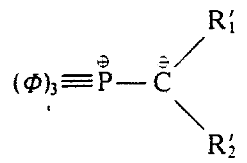

dans laquelle $R'_1$ et $R'_2$ conservent les significations précitées, puis, le cas échéant, fait réagir, lorsque $R'_1$ et/ou $R'_2$ représentent un alcoyloxycarbonyle, le composé I' correspondant, comportant un ou deux groupements esters, avec de l'ammoniac, pour obtenir le composé de formule I' correspondant, comportant un ou deux groupements carbamoyles.

Selon l'invention, le réactif de formule:

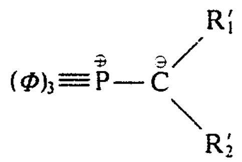

est obtenu:

- soit par action d'une base forte au sein d'un solvant, sur un halogénure de triphényl phosphonium substitué,
- soit, lorsque $R'_1$ et $R'_2$ représentent un atome d'halogène par action de la triphényl phosphine, d'une base forte et d'un haloforme au sein d'un solvant.

Un mode d'exécution commode du procédé de l'invention consiste à utiliser comme sel de phosphonium, un chlorure de triphényl phosphonium substitué, comme base forte, le butyl lithium et comme solvant l'éther.

D'une manière générale, on peut cependant utiliser une base forte choisie dans le groupe constitué par les hydrures alcalins, les amidures alcalins, les alcoolates alcalins et les alcoyllithiens et un solvant choisi dans le groupe constitué par l'éther éthylique, le diméthyl sulfoxyde, le tétrahydrofuranne le diméthoxyéthane, les alcanols, l'éther monométhylique du diéthylèneglycol et l'éther diéthylique du diéthylène glycol.

On connaissait déjà des esters d'alléthrolone et d'acides cyclopropane carboxyliques doués de propriétés insecticides (comme par exemple, le d-trans chrysanthémate de dl-alléthrolone ou bioalléthrine). Ces composés étaient doués d'une activité knock-down intéressante mais leur activité létale était relativement faible.

En remplaçant dans les esters d'alléthrolone et d'acides carboxyliques, l'alléthrolone par des dérivés résultant de l'attaque du groupement carbonyle par divers réactifs, la société demanderesse a maintenant trouvé, avec les composés (I'), une classe d'esters, doués de propriétés insecticides, acaricides, ixodicides, nématicides particulièrement intéressantes, dont les représentants sont doués à la fois de propriétés de knock-down et de pouvoir létal élevés, ce qui rend l'utilisation de ces composés particulièrement avantageuse.

Les propriétés insecticides des composés de l'invention peuvent être mises en évidence par des tests sur mouches domestiques et sur moustiques. Ces tests montrent que les esters de l'invention sont doués d'un pouvoir de knock-down élevé et d'un pouvoir létal important. Les propriétés insecticides des composés de l'invention peuvent également être mises en évidence notamment sur Spodoptera Littoralis, sur Epilachna Varivestris, Sitophilus Granarius, Tribolium Castaneum, Blatella Germanica et Aphis Fabae.

Ces tests sont décrits plus loin dans la partie expérimentale.

L'invention a également pour objet les compositions insecticides caractérisées en ce qu'elles contiennent comme matière active un au moins des composés de formule générale (I').

L'invention a plus particulièrement pour objet les compositions insecticides caractérisées en ce qu'elles contiennent, outre, le ou les principes actifs, au moins un agent synergisant, les compositons insecticides, caractérisées en ce qu'elles contiennent comme matière active au moins un composé de formule I dans lequel Y représente un groupement:

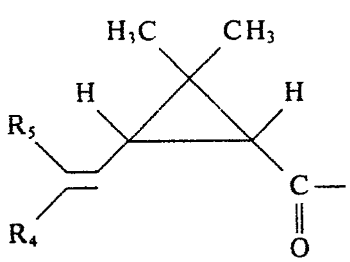

(A)

les compositions insecticides caractérisées en ce qu'elles contiennent comme matière active au moins un composé de formule I dans lequel Y représente un groupement:

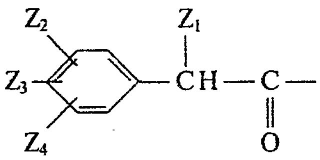

(D)

les compositions insecticides caractérisées en ce qu'elles contiennent comme matière active au moins un composé de formule I dans lequel Y représente un groupement A, $R_1$ et $R_2$ représentent un atome d'halogène, $R_3$ et $R'_3$ représentent un atome d'hydrogène et $R_4$ et $R_5$ représentent un atome d'halogène, les compositions insecticides, caractérisées en ce qu'elles contiennent comme matière active au moins un composé de formule I dans lequel Y représente un groupement A, $R_1$, $R_2$, $R_3$ et $R'_3$ représentent de l'hydrogène et $R_4$ et $R_5$ représentent ensemble avec l'atome de carbone auquel ils sont liés, un homocycle carboné, les compositions insecticides caractérisées en ce qu'elles contiennent comme matière active au moins un composé de formule I dans lequel Y représente un groupement A, $R_1$, $R_2$, $R_3$ et $R'_3$ représentent de l'hydrogène et $R_4$ et $R_5$ représentent un atome d'halogène, les compositions insecticides caractérisées en ce qu'elles contiennent un au moins des principes actifs choisis dans le groupe constitué par les composés dont les noms suivent:

- le (1R,trans) 2,2-diméthyl 3-cyclopentylidène méthyl cyclopropane-1-carboxylate de (1S)2-méthyl 3-allyl 4-méthylène cyclopent-2-èn 1-yle,
- le (1R,trans) 2,2-diméthyl 3-(2,20difluoroéthényl)cyclopropane-1-carboxylate de (1S)2-méthyl 3-allyl 4-méthylène cyclopent-2-èn 1-yle,
- le (1R,trans) 2,2-diméthyl 3-(2,2-dichloroéthényl)cyclopropane-1-carboxylate de (1S)2-méthyl 3-allyl 4-méthylène cyclopente-2-èn 1-yle,
- le (1R,trans) 2,2-diméthyl 3-/2(Z)cyano éthényl/cyclopropane-1-carboxylate de (1S)2-méthyl 3-allyl 4-méthylène cyclopent-2-èn 1-yle,
- le (1R,trans) 2,2-diméthyl 3-/2-fluoro 2-chloro éthényl/cyclopropane-1-carboxylate de (1S)2-méthyl 3-allyl 4-méthylène cyclopent-2-èn 1-yle,
- le (1R,trans) 2,2-diméthyl 3-cyclobutylidène méthyl cyclopropane-1-carboxylate de (1S)2-méthyl 3-allyl 4-méthylène cyclopent-2-èn 1-yle,
- le (1R,cis) 2,2-diméthyl 3-éthynyl cyclopropane-1-carboxylate de (1S)2-méthyl 3-allyl 4-méthylène cyclopent-2-èn 1-yle.

Dans les compositions de l'invention, la ou les matières actives peuvent être additionnées éventuellement d'un ou plusieurs autres agents pesticides. Ces pompositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le pricipe actif, ces compositions contiennent, en général, un véhicule et/ou un agent tensioactif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre, telle que le talc, les argiles, les silicates, le Kieselguhr ou un solide combustible, tel que la poudre de tabu (ou marc de pyrèthre).

Ainsi que mentionneé précédemment, les compositions de l'invention peuvent contenir un agent synergisant des pyréthrinoïdes.

Pour exalter l'activité insecticide des composés de l'invention, on peut ainsi les additionner des synergistes classiques utilisés en pareil cas, tel que le 1-(2,5,8-trioxa dodécyl 2-propyl 4,5-méthylènedioxy)-benzène (ou butoxyde de pipéronyle), la N-(2-éthyl heptyl)bicyclo/2,2,1-/ 5 heptèn-2,3-dicarboximide, le pipéronyl-bis-2-(2'-n-butoxy éthoxy) éthyl acétal (ou tropical).

Les compositions insecticides selon l'invention contiennent, de préférence, entre 0,005% et 10% en poids de matière active.

Il a été trouvé par ailleurs que les produits de formule (I') possèdent d'intéressantes propriétés acaricides.

Des tests sur Tetranychus Urticae permettent de démontrer l'activité acaricide des composés de formule (I').

Ces tests montrent que les composés (I') possèdent une double action dans la lutte contre les acariens. Outre l'action létale classique ces composés sont doués d'une action répulsive particulièrement intéressante sous l'angle écologique.

L'invention a donc pour objet les compositions acaricides, caractérisées en ce qu'elles contiennent comme matière active, un au moins des composés de formule générale (I').

L'invention a également pour objet les compositions répulsives vis-à-vis des acariens parasites des végétaux, caractérisées en ce qu'elles contiennent comme matière active un au moins des composés (I').

Les compositions acaricides selon l'invention peuvent éventuellement être additionnees d'un ou plusieurs autres agents pesticides et d'un agent synergisant.

Les compositions acaricides peuvent se présenter sous forme de poudre, granulés, suspensions, émulsions, solutions.

Pour l'usage acaricide, on utilise de préférence des poudres mouillables, pour pulvérisation foliaire, contenant de 1 à 80% en poids de matière active ou des liquides pour pulvérisation foliaire contenant de 1 à 500 g/l de matière active. On peut également employer des poudres pour poudrages foliaires, contenant des 0,05 à 10% en poids de matière active.

Les composés de formule (I') se sont également avérés doués d'intéressantes propriétés nématicides.

Des tests sur Panagrellus Silusiae permettent de démontrer l'acitivité nématicide des composés de formule (I').

L'invention a donc pour objet les compositions nématicides caractérisées en ce qu'elles contiennent comme matière active, un au moins des composés de formule générale (I').

Pour l'usage nématicide, on utilise de préférence des liquides pour traitement des sols contenant de 300 à 500 g/l de principe actif.

Les composés acarides et nématides selon l'invention sont utilisés, de préférence, à des doses comprises entre 1 et 100 g de matière active à l'hectare.

Les composése de formule (I') sont également doués de propriétés antifongiques.

Des tests zur Aerobacter Aerogenes, Pseudomonas Aeruginosa, Botrytis Cinerea, Fusarium Roseum permettent de démontrer l'activité antifongique du composé de formule (I').

L'invention a donc pour objet les compositions antifongiques, caractérisées en ce qu'elles contiennent comme matière active un au moins des composés de formule générale (I').

Pour l'usage antifongique, on utilise de préférence des poudres pour pulvérisation foliaire, contenant des 25 à 95% en poids de matière active ou des poudres pour poudrage filiaire, contenant de 2,5 à 99% en poids de matière active.

La société demanderesse a enfin trouvé que les produits de formule (I') possèdent des propriétés antiacariennes qui permettent d'utiliser ces produits en tant que médicament vétérinaire dans la lutte contre les acariens parasites des animaux et notamment dans la lutte contre lex ixodidés et les sarcoptidés parasites des animaux.

L'activité acaricide des composés de formule (I') peut être démontrée par un test sur Rhipicephalus Sanguinens chez le chien.

Les composés de formule (I') peuvent être utilisés chez l'animal pour lutter notamment contre toutes sortes de gales, telles que la gale sarcoptique, la gale psoroptique et la gale chorioptique.

Les composés de formule (I') permettent encore de lutter contre toutes sortes de tiques comme, par exemple, l'espèce Boophilus, l'espèce Hyalomnia, l'espèce Amblyoma et l'espèce Rhipicephalus.

L'invention a donc également pour objet les compositions pharmaceutique à usage vétérinaire utilisées dans la lutte contre les affections provoquées par les acariens, caractérisées en ce qu'elles contiennent, comme matière active, un au moins des composés de formule générale (I').

Les compositions pharmaceutiques à usage vétérinaire, selon l'invention peuvent être utilisées par voie externe.

Les compositions pharmaceutique à usage vétérinaire, selon l'invention peuvent être également utilisées par voie parentérale ou digestive.

Les compositions pharmaceutiques à usage vétérinaire, selon l'invention peuvent être avantageusement additionnées d'un agent synergisant des pyréthrinoïdes.

Enfin, il peut être commode pour l'usage vétérinaire d'utiliser des composés (I') en mélange avec des aliments composés équilibrés pour animaux.

On pourra, par exemple, employer des aliments composés pour animaux qui renferment 0,01 à 2% en poids de 2,2-diméthyl 3S-(2,2-difluoroéthényl)cyclopropane-1R-carboxylate de 1(S)-2-méthyl 3-allyl 4-méthylène cyclopent-2-èn 1-yle.

L'invention a donc pour objet les compositons destinées à l'alimentation animale, caractérisées en ce qu'elles sont constituées par un aliment composé, équilibré, pour animal et qu'elles renferment, en outre, un au moins des composés de formule générale (I').

L'invention a également pour objet les associations douées d'activité insecticide, acaricide, fongicide, ou nématicide, caractérisées en ce qu'elles contiennent comme matière active, d'une part un au moins des composés de formule générale (I'), et d'autre part, un au moins des esters pyréthrinoïdes choisis dans le groupe constitué par les esters d'alléthrolones, d'alcool 3,4,5,6-tétrahydrophthalimido méthylique, d'alcool 5-benzyl 3-furyl méthylique, d'alcool 3-Phénocy benzylique et d'alcools $\alpha$-cyano 3-phénoxy benzyliques des acides chryanthémiques, par les esters d'alcool 5-benzyl 3-furyl méthylique des acides 2,2-diméthyl 3-(2-oxo 3-tétrahydrothiophénylidène méthyl)cyclopropane-1-carboxyliques, par les esters d'alcool 3-phénoxy benzylique et d'alcools $\alpha$-cyano 3-phénoxy benzyliques des acides 2,2-diméthyl 3-(2,2-dichlorovinyl)cyclopropane-1-carboxyliques, par les esters d'alcools $\alpha$-cyano 3-phénoxy benzyliques d'acides 2,2-diméthyl 3-(2,2-dibromovinyl)cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophthalimidométhylique, d'alcool 5-benzyl 3-furyl méthylique, d'alcool 3-phénoxy benzylique et d'alcools $\alpha$-cyano 3-phénoxy benzyliques des acides 2,2-diméthyl 3-(1,2,2,2-tétrahalo)cyclopropane-1-carboxyliques, dans lesquels ›halo‹ représente un atome de fluor, de chlore ou de brome, étant entendu que les composés I' peuvent exister sous toutes

leurs formes stéréoisomères possibles de même que les copules acides et alcools des esters pyréthrinoïdes ci-dessus.

Les associations selon l'invention présentent notamment l'intérêt soit de permettre de combattre, par la polyvalence de leur action, une gamme de parasites, plus étendue, soit de manifester, dans certains cas, un effet de synergie.

Les composés de formule générale (II) utilisés au départ d'un des procédés de préparation des composés (I') sont décrits dans une demande de brevet déposée en France le même jour que la présente demande, par la société demanderesse et intitulée »Dérivés de l'alléthrolone et procédé de préparation«.

Les composés (II) peuvent, par exemple, être préparés en soumettant les composés (V):

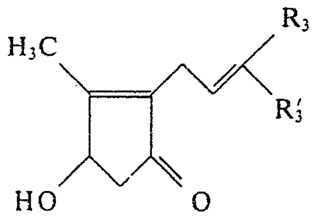

(V)

à l'action d'un réactif de formule:

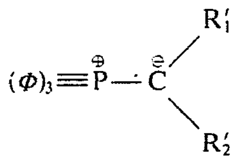

après blocage éventuel de l'hydroxyle sous forme d'ester ou sous forme d'éther.

Les composés (II) dans lesquels $R_1$ et/ou $R_2$ représentent des groupements carbamoyles peuvent être obtenus en faisant réagir un composé (II) dont le groupement hydroxyle peut être bloqué, et, dans lequel $R'_1$ et/ou $R'_2$ représentent un groupement alcoyloxycarbonyle, avec d'ammoniac, puis, en débloquant éventuellement le groupement hydroxyle.

Des exemples de préparations sont donnés plus loin dans la partie expérimentale.

Les composés (III) sont, en général, décrits dans la littérature, ou peuvent être préparés par des procédés décrits dans la littérature. Leurs dérivés fonctionnels sont accesibles par des procédés connus de l'homme de l'art.

Des exemples de préparations sont donnés plus loin dans la partie expérimentale.

Les composés (IV) utilisés au départ d'un des procédés de préparation des composés (I') sont en général décrits dans la littérature. Ils peuvent être préparés par estérification des acides carboxyliques convenables, par l'alléthrolone ou par un dérivé de l'alléthrolone substitué sur la chaîne allylique.

Les exemples siuvants illustrent l'invention sans la limiter.

### Exemple 1

(1R,cis) 2,2-diméthyl 3-(2-méthyl 1-propényl) cyclopropane-1-carboxylate de (1S)2-méthyl 3-allyl 4-méthylène cyclopent-2-èn 1-yle

On mélange à température ambiante, 6,5 g de chlorure de l'acide ((1R,cis) 2,2-diméthyl 3-(2-méthyl 1-propényl) cyclopropane-1-carboxylique, 20 $cm^3$ de benzène, 4 $cm^3$ de pyridine puis en 30 minutes, on ajoute 5 g de 1S-hydroxy 2-méthyl 3-allyl 4-méthylène cyclopent-2-ène en solution dans 10 $cm^3$ de benzène. On refroidit à 20°C et agite pendant 20 heures à cette température. On verse dans l'eau, décante extrait au benzène la phase aqueuse, lave à l'eau les phases benzéniques réunies, les sèche sur sulfate de magnésium, filtre et concentre sous pression réduite. On purifie le résidu par chromatographie sur silice en éluant au mélange cyclohexane-acétate d'éthyle (9-1) concentant 1‰ de triéthylamine et obtient 9,41 g de produit attendu.

$[\alpha]_D = -32° \pm 1°$ (c = 1,3% éthanol)

Analyse: $C_{20}H_{28}O_2$:

| | | |
|---|---|---|
| Calculé: | C 79,95, | H 9,39%, |
| Trouvé: | C 79,8, | H 9,5% |

Spectre IR (chloroforme)

- Absorption à 1720 $cm^{-1}$ attribuée au groupement C=O (ester)
- Absorption à 1639 $cm^{-1}$ attribuée au groupement C=C
- Absorption à 993—916 $cm^{-1}$ attribuée au groupement $-C=CH_2$
- Absorption à 864 $cm^{-1}$ attribuée au groupement $>=CH_2$.

La 1S-hydroxy 2-méthyl 3-allyl 4-méthylène cyclopent-2-ène utilisée au départ de l'exemple 1 peut être préparée de la manière suivante:

On mélange sous agitation et sous atmosphère inerte 250 $cm^3$ d'éther, 26,4 $cm^3$ de ter-butanol et 100 g de bromure de triphényl méthyl phosphonium, puis on ajoute en 4 fois et en 10 minutes, 31,4 g de ter-butylate de potassium et laisse le mélange réactionnel pendant 5 heures à température ambiante. On refroidit à O, +5°C et ajoute en 20 minutes, 31,9 g de 1S-hydroxy 2-méthyl 3-allyl 4-oxo cyclopent-2-ène dissous dans 30 $cm^3$ d'éther. On maintient le mélange réactionnel pendant 2 heures à O, +5°C puis le ramène à température ambiante pendant 16 heures. On verse sur une solution aqueuse saturée de phosphate monosodique, décante, extrait la phase aqueuse à l'éther, sèche les phases organiques sur sulfate de magnésium, les filtre et les concentre à sec. On reprend le résidu à l'éther, agite quelques minutes et filtre. On concentre le filtrat sous pression réduite et chromatographie le résidu sur silice en éluant au mélange cyclohexane-acétate d'éthyle (60—40) contenant 2‰ de triéthylamine et obtient 26,05 g de produit attendu. Par recristallisation dans l'essence G (eb.: 40—70°C) on obtient un produit dont les constantes sont les suivantes F: 23°C.

$[\alpha]_D = -110° \pm 2°$ (c = 0,8% chloroforme).

Spectre IR (chloroforme)

- Absorption à 3586 $cm^{-1}$ attribuée à la fonction —OH
- Absorption à 865 $cm^{-1}$ attribuée au groupement $=CH_2$.

## Exemple 2

### (1R,trans)2,2-diméthyl 3-(2-méthyl 1-propényl) cyclopropane-1-carboxylate de (1S)2-méthyl 3-allyl 4-méthylène cyclopent-2-èn 1-yle

On met en suspension, sous atmosphère inerte, 26 g de bromure de triphényl méthylphosphonium dans 150 $cm^3$ d'éther, ajoute en 20 minutes à température ambiante 50 $cm^3$ de butyllithium 1,4 N et agite 30 minutes à température ambiante. On refroidit à +5°C et ajoute en 25 minutes, 10,9 g de (1R,trans)2,2-diméthyl-3-(2-méthyl 1-propényl)cyclopropane-1-carboxylate de (1S)2-méthyl 3-allyl 4-oxo cyclopent-2-èn-1-yle dissous dans 60 $cm^3$ d'éther, laisse en contact pendant 30 minutes à +5°C et verse sur une solution glacée de phosphate monosodique. On extrait la phase aqueuse à l'éther, lave les phases organiques à l'eau jusqu'à neutralité, les réunit, les sèche sur sulfate de magnésium et les concentre à sec sous pression réduite. On purifie le résidu par chromatographie sur silice en éluant au mélange cyclohexane-acétate d'éthyle-triéthylamine (90-10-0,1) et recueille 1,03 g de produit attendu:

Spectre IR (chloroforme)

- Absorption à 1715 $cm^{-1}$ attribuée au groupement C=O (ester)
- Absorption à 1639 $cm^{-1}$ attribuée au groupement C=C
- Absorption à 867 $cm^{-1}$ attribuée au groupement $>C=CH_2$.

Spectre RMN ($CDCl_3$ — 60 MHz)

- Pic à 1,20 p. p. m. (doublet J: 7,5 Hz) attribué aux hydrogènes des groupement $-CH_3$ en position 2 du cyclopropane.
- Pic à 1,7 p. p. m. attribué aux hydrogènes des méthyles en bout de la chaîne en position 3 du cyclopropane.
- Pic à 1,78 p. p. m. attribué aux hydrogènes du groupement $-CH_3$ en position 2 du cyclopentène.

Exemple 3
(1R,trans)2,2-diméthyl 3-(2-méthyl 1-propényl)cyclopropane-1-carboxylate de (1S)2-méthyl 3-allyl 4-méthylène cyclopent-2-èn 1-yle

On mélange à 20°C, 7 g de chlorure d'acide (1R,trans)2,2-diméthyl 3-(2-méthyl 1-propényl)cyclopropane-1-carboxylique, 20 $cm^3$ de benzène, 5 $cm^3$ de pyridine et ajoute en 10 minutes une solution de 5 g de 1S-hydroxy 2-méthyl 3-allyl 4-méthylène cyclopent-2-èn dans 5 $cm^3$ de benzène, refroidit à 20—25°C et agite pendant 4 heures à cette température. On ajoute ensuite 50 $cm^3$ d'eau, agite pendant 10 minutes, décante, extrait au benzène, lave les phases organiques réunies à l'eau, les sèche sur sulfate de magnésium, filtre, amène le filtrat à sec sous pression réduite. On purifie le résidu par chromatographie sur silice en éluant au mélange cyclohexane-acétate d'éthyle (9-1) contenant 1‰ de triéthylamine et obtient 8,76 g de produit attendu identique à celui obtenu à l'exemple 2. (Ces 2 produits présentent les mêmes constantes).

Exemple 4
(1R,cis)2,2-diméthyl 3-cyclopentylidène méthylcyclopropane-1-carboxylate de (1S)2-méthyl 3-allyl 4-méthylène cyclopent-2-èn 1-yle

On mélange à 20°C, 4 g de 1S-hydroxy 2-méthyl 3-allyl 4-méthylène cyclopent-2-ène, 10 $cm^3$ de benzène, 5 $cm^3$ de pyridine et ajoute en 15 minutes 30 $cm^3$ d'une solution benzénique de chlorure de l'acide (1R,cis)2,2-diméthyl 3-cyclopentylidène méthyl cyclopropane-1-carboxylique (préparé au départ de 7 g d'acide) et agite pendant 20 heures à 20°C. On verse ensuite dans 30 $cm^3$ d'eau, agite pendant 10 minutes, extrait au chlorure de méthylène, lave la phase organique à l'eau, puis sèche les phases organiques réunies sur sulfate de mangésium, filtre et amène le filtrat à sec sous pression réduite. On purifie résidu par chromatographie sur silice en éluant au mélange cyclohexane-acétate d'éthyle (95-5) contenant 1‰ de triéthylamine et obtient 4,1 g de produit attendu.

Analyse: ($C_{22}H_{30}O_2$):

| | | |
|---|---|---|
| Calculé: | C 80,93, | H 9,26%, |
| Trouvé: | C 81,2, | H 9,4%. |

Spectre IR (chloroforme)

— Absorption à 1715 $cm^{-1}$ attribuée au groupement >C=O (ester)
— Absorption à 1635 $cm^{-1}$ attribuée au groupement >C=O<
— Absorption à 918—995 $cm^{-1}$ attribuée au groupement >C=$CH_2$.

Exemple 5
(1R,trans)2,2-diméthyl 3-cyclopentylidène méthylcyclopropane-1-carboxylate de (1S)2-méthyl 3-allyl 4-méthylène cyclopent-2-èn 1-yle

A une suspension de 5 g de 1S-hydroxy 2-méthyl 3-allyl 4-méthylène cyclopent-2-èn dans 15 $cm^3$ de benzène et 2,96 $cm^3$ de pyridine, on ajoute en 10 minutes en maintenant la température à 20°C, 7,08 g de chlorure de l'acide (1R,trans)2,2-diméthyl 3-cyclopentylidène méthyl cyclopropane-1-carboxylique dissous dans 3 $cm^3$ de benzène, laisse en contact sous agitation pendant 17 heures toujours à 20°C. On ajoute 20 $cm^3$ d'eau, agite 10 minutes, décante, extrait la phase aqueuse au benzène, lave les phases organiques à l'eau, extrait les eaux de lavage au benzène, sèche les phases organiques réunies sur sulfate de magnésium, filtre et amène à sec le filtrat sous pression réduite. On purifie le résidu par chromatographie sur silice en éluant au mélange cyclohexane-acétate d'éthyle (95-5) contenant 1‰ de triéthylamine et obtient 8,24 g de produit attendu.

$[\alpha]_D^{20} = -87° \pm 1°$ (c = 0,9% éthanol)

Spectre IR (chloroforme)

— Absorption à 1715 $cm^{-1}$ attribuée au groupement>C=O (ester)
— Absorption à 1635 $cm^{-1}$ attribuée au groupement C=C
— Absorption à 865 $cm^{-1}$ attribuée au groupement >C=$CH_2$.

Exemple 6

(1R,cis)2,2-diméthyl 3-(2-oxo 3-tétrahydrofurannylidène méthyl)cyclopropane-1-carboxylate de (1S)2-méthyl 3-allyl 4-méthylène cyclopent-2-èn 1-yle

A une suspension de 7,6 g de chlorure d'acide (1R,cis)2,2-diméthyl 3-(2-oxo 3-tétrahydrofurannylidène méthyl)cyclopropane-1-carboxylique dans 30 $cm^3$ de benzène et 2,6 $cm^3$ de pyridine, on ajoute à +10°C en 15 minutes, 4,40 g de 1S-hydroxy 2-méthyl 3-allyl 4-méthylène cyclopent-2-ène dissous dans 10 $cm^3$ de benzène en maintenant la température à 25°C et laisse en contact à 20°C pendant 16 heures.

On ajoute ensuite 20 $cm^3$ d'eau, agite en présence de 20 $cm^3$ de chlorure méthylène, décante, extrait au chlorure de méthylène, lave les phases organiques à l'eau, les sèche sur sulfate de magnésium, filtre et amène le filtrat à sec sous pression réduite. On obtient un produit gélatineux que l'on purifie par empâtage dans l'essence G (eb.: 40—70°C), puis par une chromatographie du produit cristallisé sur silice en éluant au mélange benzène-acétate d'éthyle (9-1). On obtient 4,01 g de produit attendu: F: 93°C.

Analyse: $C_{21}H_{26}O_4$:

| | | |
|---|---|---|
| Calculé: | C 73,66, | H 7,66%, |
| Trouvé: | C 73,4, | H 7,5%. |

Spectre IR (chloroforme)

— Absorptions à 1750—1720 $cm^{-1}$ attribuées aux groupements C=O (lactone et ester)
— Absorptions à 1667—1633 $cm^{-1}$ attribuées aux groupements C=C
— Absorptions à 992—916 $cm^{-1}$ attribuées au groupement $-CH=CH_2$.

Exemple 7

(1R,trans)2,2-diméthyl 3-(2-oxo 3-tétrahydrofurannylidène méthyl)cyclopropane-1-carboxylate de (1S)2-méthyl 3-allyl 4-méthylène cyclopent-2-èn 1-yle

On met en suspension, à 20°C, 3 g de 1S-hydroxy 2-méthyl 3-allyl 4-méthylène cyclopent-2-ène dans 10 $cm^3$ de benzène et 3 $cm^3$ de pyridine, puis ajoute en 15 minutes sans dépasser 25°C 5,4 g du chlorure de l'acide (1R,trans)2,2-diméthyl 3-(2-oxo 3-tétrahydrofurannylidène méthyl)cyclopropane-1-carboxylique en solution dans 50 $cm^3$ de benzène et laisse pendant 20 heures à 20°C sous agitation. On ajoute ensuite 30 $cm^3$ d'eau, agite 10 minutes, décante, extrait la phase aqueuse au benzène, lave les phases organiques à l'eau, les sèche sur sulfate de magnésium, filtre et amène le filtrat à sec sous pression réduite. On purifie le résidu par chromatographie sur silic en éluant au mélange cyclohexane-acétate d'éthyle (7-3) contenant 1‰ de triéthylamine et obtient 2,61 g de produit attendu.

$[\alpha]_D = -28,5° \pm 2,5°$ (c = 0,5%, éthanol)

Analyse ($C_{21}H_{26}O_4$):

| | | |
|---|---|---|
| Calculé: | C 73,65, | H 7,65%, |
| Trouvé: | C 73,9, | H 7,7%. |

Spectre IR (chloroforme)

— Absorptions à 1753 $cm^{-1}$—1718 $cm^{-1}$ attribués aux fonctions C=O (lactone et ester)
— Absorptions à 1673 $cm^{-1}$ — 1633 $cm^{-1}$ attribués au groupement C=C
— Absorptions à 992—916 $cm^{-1}$ attribuées au groupement $-CH=CH_2$.

Exemple 8

(1R,cis)2,2-diméthyl 3-(2-oxo 3-tétrahydrothiénylidène méthyl)cyclopropane-1-carboxylate de (1S)2-méthyl 3-allyl 4-méthylène cyclopent-2-èn 1-yle

A une suspension de 5 g de 1S-hydroxy 2-méthyl 3-allyl 4-méthylène cyclopent-2-ène dans 10 $cm^3$ de benzène et 5 $cm^3$ de pyridine, on ajoute sous agitation, en 15 à 20 minutes, 50 $cm^3$ d'une solution benzénique de chlorure d'acide (1R,cis)2,2-diméthyl 3-(2-oxo 3-tétrahydrothiénylidène méthyl)cyclopropane-1-carboxylique (préparé au départ de 8,5 g d'acide) en maintenant la température à 20—25°C

et laisse le mélange réactionnel sous agitation pendant 18 heures à température ambiante. On introduit ensuite 50 $cm^3$ d'eau, agite 15 minutes, décante, extrait la phase aqueuse par 50 $cm^3$ de chlorure de méthylène, lave les phases organiques à l'eau, les sèche sur sulfate de sodium, filtre et amène le filtrat à sec sous pression réduite. On purifie le résidu par chromatographie sur silice en éluant au mélange benzène-acétate d'éthyle (9-1) contenant 1‰ de triéthylamine et obtient 7,8 g de produit attendu qui cristallise de l'éther de pétrole. F = 55°C.

$[\alpha]_D^{20} = -12° \pm 2°$ (c = 0,8%, éthanol)

Analyse ($C_{21}H_{26}O_3S$):

| | | | |
|---|---|---|---|
| Calculé: | C 70,35, | H 7,31, | S 8,94% |
| Trouvé: | C 70,3, | H 7,4, | S 8,6% |

Spectre RMN ($CDCl_3$, 60 MHz)

- Pic à 1,29 p. p. m. (doublet J: 5,5 Hz) attribué aux hydrogènes des méthyles géminés.
- Pic à 1,73 p. p. m. attribué aux hydrogènes du groupement $-CH_3-C=C$.
- Pics de 1,58 à 3,58 p. p. m. attribués auxh hydrogènes du cyclopropane, du groupement $-CH_2-CH=CH_2$ et du groupement $CH_2S$.
- Pics de 4,67 à 6,17 p. p. m. attribués aux hydrogènes du groupement $-CH=CH_2$ et en position 1 du cyclopropane.

## Exemple 9

(1R,trans)2,2-diméthyl 3-(2-oxo 3-tétrahydrothiénylidène méthyl cyclopropane-1-carboxylate de (1S)2-méthyl 3-allyl 4-méthylène cyclopent-2-èn 1-yle

Dans une suspension de 5 g de 1S-hydroxy 2-méthyl 3-allyl 4-méthylène cyclopent-2 ène dans 2 $cm^3$ de pyridine et 20 $cm^3$ de benzène, on introduit en 15 à 20 minutes en maintenant à 20—25°C, 50 $cm^3$ d'une solution benzénique de chlorure de l'acide (1R,trans)2,2-diméthyl 3-(2-oxo 3-tétrahydrothiénylidène méthyl)cyclopropane-1-carboxylique (préparé au départ de 8,5 g d'acide), agite pendant 18 heures à température ambiante. On ajoute 50 $cm^3$ d'eau, agite 10 minutes, décante, extrait la phase aqueuse au chlorure de méthylène, lave les phases organiques réunies à l'eau, les sèche sur sulfate de magnésium, filtre et amène le filtrat à sec sous pression réduite. On purifie le résidu par chromatographie sur silice en éluant au mélange cyclohexaneacétate d'éthyle (8-2) contenant 1‰ de triéthylamine pour obtenir 7,47 g de produit pur.

$[\alpha]_D^{20} = -61,5° \pm 1,5°$ (c = 0,8%, éthanol)

Analyse ($C_{21}H_{26}O_3S$):

| | | | |
|---|---|---|---|
| Calculé: | C 70,35, | H 7,31, | S 8,94% |
| Trouvé: | C 70,2, | H 7,6, | S 8,6% |

Spectre RMN (60 MHz, $CDCl_3$)

- Pic à 1,28 p. p. m. (doublet J: 3,5 Hz) attribué aux méthyles géminés.
- Pic à 1,78 p. p. m. attribué aux hydrogènes du groupement $CH_3-C=$.
- Pic à 2,64 p. p. m. (doublet J: 13 Hz) attribué aux hydrogènes du cyclopropane et du groupement $-CH_2-S$.
- Pics de 4,67 à 5,25 p. p. m. attribués aux hydrogènes des groupements $=CH_2$.
- Pic à 6,2 p. p. m. (doublet J: 10 Hz) attribué aux hydrogènes éthyléniques.
- Pics de 5,5 à 6,33 p. p. m. attribués aux hydrogènes des groupements $CH=CH_2$ et $COO-CH-$.

## Exemple 10

(1R,cis)2,2-diméthyl 3-(2-cyano éthényl) cyclopropane-1-carboxylate de (1S)2-méthyl 3-allyl 4-méthylène cyclopent-2-èn-1-yle(isomère E et Z)

A une suspension de 6,1 g de 1S-hydroxy 2-méthyl 3-allyl 4-méthylène cyclopent-2-ène, dans 8 $cm^3$ de pyridine et 60 $cm^3$ de benzène, on ajoute sous agitation à température ambiante, une solution de

chlorure de l'acide (1R,cis)2,2-diméthyl 3-(2-cyano éthényl) cyclopropane-1-carboxylique (isomères E+Z) dans 10 $cm^3$ de benzène et met sous agitation pendant 1 heure. On verse ensuite dans l'eau, extrait à l'éther isopropylique, lave les pnases organiques réunies à l'eau, les sèche sur sulfate de sodium et les amène à sec sous pression réduite. On chromatographie l'huile résiduelle sur silice en éluant au benzène à 1‰ de triéthylamine et obtient:

– d'une part 2,5 g d'isomère Z

$[\alpha]_D = +66,5° \pm 2,5°$ (c = 0,6%, benzène)

Analyse ($C_{19}H_{23}NO_2$):

| | | | |
|---|---|---|---|
| Calculé: | C 76,73, | H 7,80, | N 4,71% |
| Trouvé: | C 76,6, | H 7,8, | N 4,6% |

Spectre IR (chloroforme)

– Absorption à 1714 $cm^{-1}$ attribuée au groupement C=O
– Absorption à 1612–2220 $cm^{-1}$ attribuée au groupement

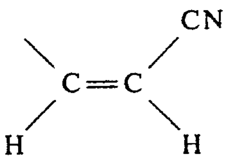

– Absorption à 1635 $cm^{-1}$ attribuée au groupement C=C
– Absorption à 917 $cm^{-1}$ attribuée au groupement $CH_2$=CH
– Absorption à 866–874 $cm^{-1}$ attribuée au groupement $CH_2$=C<
– d'autre part 1,9 g d'isomère E.

$[\alpha]_D^{20} = -58° \pm 2,5°$ (c = 0,7%, benzène)

Analyse ($C_{19}H_{23}NO_2$):

| | | | |
|---|---|---|---|
| Calculé: | C 76,73, | H 7,79, | N 4,71% |
| Trouvé: | C 76,3, | H 7,9, | N 4,4% |

Spectre IR (chloroforme)

– Absorption à 2225 $cm^{-1}$ attribuée au groupement –C≡N
– Absorption à 1718 $cm^{-1}$ attribuée au groupement C=O
– Absorption à 1381 $cm^{-1}$ attribuée aux méthyles géminés
– Absorption à 1633 $cm^{-1}$ attribuée au groupement C=C
– Absorption à 910–917 $cm^{-1}$ attribuée au groupement $CH_2$=CH.

Exemple 11

(d) α-isopropyl α-(4-chlorophényl) acétate de (1S)2-méthyl 3-allyl 4-méthylène cyclopent-2-èn-1-yle

Dans une suspension de 1S-hydroxy 2-méthyl 3-allyl 4-méthylène cyclopent-2-ène dans 30 $cm^3$ de benzène et 5 $cm^3$ de pyridine, on introduit à 20°C sous agitation, 4,6 g de chlorure de l'acide (d) α-isopropyl α-(4-chlorophényl) acétique dissous dans 5 $cm^3$ de benzène et agite pendant 16 heures à 20°C. On verse le mélange réactionnel dans l'eau, extrait à l'éther isopropylique, sèche les phases organiques réunies sur sulfate de sodium, filtre et amène le filtrat à sec sous pression réduite. On obtient une huile que l'on chromatographie sur silice en éluant au mélange benzène-cyclohexane (4-6) contenant 1‰ de triéthylamine et recueille 4,8 g de produit attendu pur.

$[\alpha]_D^{20} = -81,5° \pm 2°$ (c = 0,8% benzène)

Analyse ($C_{21}H_{25}ClO_2$):

| | | | |
|---|---|---|---|
| Calculé: | C 73,13, | H 7,31, | Cl 10,28%, |
| Trouvé: | C 73,2, | H 7,4, | Cl 10,2%. |

Spectre RMN ($CDCl_3$, 60 MHz)

- Pics à 0,69 p. p. m. et à 1,05 p. p. m. (2 doublets J: 6,5 Hz) attribués aux hydrogènes des méthyles du groupement isopropyle.
- Pic à 1,58 p. p. m. attribué aux hydrogènes du groupement $-CH_3$ en position 2 du cyclopentène.
- Pic à 3,14 p. p. m. (doublet J: 10,5 Hz) attribué à l'hydrogène en $\alpha$ du groupement $-COO$.
- Pics de 5,33 à 6,17 p. p. m. attribué à l'hydrogène en position 2 du groupement allyle.
- Pic de 4,73 à 5,07 p. p. m. attribué aux hydrogènes des groupements $=CH_2$ en position 3 du groupement allyle et en position 4 du cyclopentène.

Exemple 12

(1R,trans)2,2-diméthyl 3-(2,2-difluoroéthényl)cyclopropane-1-carboxylate de (1S)2-méthyl 3-allyl 4-méthylène cyclopent-2-èn 1-yle

A une suspension de 3 g de 1S-hydroxy 2-méthyl 3-allyl 4-méthylène cyclopent-2-ène dans 30 $cm^3$ de benzène et 4,5 $cm^3$ de pyridine, on ajoute sans dépasser 30°C et sous agitation, 3,88 g de chlorure d'acide (1R,trans)2,2-diméthyl 3-(2,2-difluoroéthényl)cyclopropane-1-carboxylique en solution dans 5 $cm^3$ de benzène, agite pendant 4 heures à 20°C, puis verse dans l'eau. On décante, extrait la phase aqueuse à l'éther isopropylique, lave les extraits organiques réunis à l'eau, les sèche sur sulfate de sodium, filtre et amène à sec sous pression réduite. On purifie l'huile résiduelle par rectification sous pression réduite et obtient 2,3 g de produit pur. Eb (0,1 mm Hg): 113—114°C.

$[\alpha]_D^{20} = -67° \pm 2,5°$ (c = 0,6% benzène)

Analyse ($C_{18}H_{22}F_2O_2$):

| | | | |
|---|---|---|---|
| Calculé: | C 70,11, | H 7,19, | F 12,32%, |
| Trouvé: | C 69,8, | H 7,2, | F 12,5%. |

Spectre IR (chloroforme)

- Absorptions à 1746—1716 $cm^{-1}$ attribuées aux groupements: C=O (ester) et

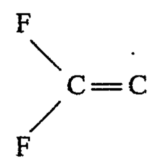

- Absorption à 1636 $cm^{-1}$ attribuée aux groupements C=C du cyclopentène et $C=CH_2$
- Absorptions à 920—991 $cm^{-1}$ attribuées au groupement $CH=CH_2$.

Exemple 13

(1R,cis)2,2-diméthyl 3-(2-fluoro 2-chloroéthényl)cyclopropane-1-carboxylate de (1S)2-méthyl 3-allyl 4-méthylène cyclopent-2-èn-1-yle

On introduit sous agitation à 20°C, une solution de 4,2 g de chlorure d'acide (1R,cis)2,2-diméthyl 3-(2-fluoro 2-chloroéthényl) cyclopropane-1-carboxylique dans 10 $cm^3$ de benzène, dans un mélange de 3 g de 1S-hydroxy 2-méthyl 3-allyl 4-méthylène cyclopent-2-ène, de 20 $cm^3$ de benzène et de 5,5 $cm^3$ de pyridine, agite le mélange réactionnel pendant 16 heures à 20°C puis verse dans l'eau. On décante, extrait la phase aqueuse à l'éther isopropylique, sèche les phases organiques réunies sur sulfate de sodium, filtre et amène à sec sous pression réduite. On purifie l'huile résiduelle par distillation et obtient 3,4 g de produit attendu pur. Eb 0,1 mm Hg: 125°C.

$[\alpha]_D^{20} = -10° \pm 2°$ (c = 0,5%, benzène)

Analyse ($C_{18}H_{22}ClFO_2$):

| | | | | |
|---|---|---|---|---|
| Calculé: | C 66,55, | H 6,83, | F 5,85, | Cl 10,92% |
| Trouvé: | C 65,9, | H 6,9, | F 6.0, | Cl 11,4%. |

Spectre IR (chloroforme)

- Absorption à 1716 $cm^{-1}$ attribuée au groupement C=O de l'ester
- Absorption à 1675 $cm^{-1}$ attribuée au groupement

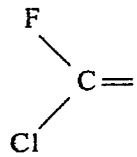

- Absorption à 1636 $cm^{-1}$ attribuée au groupement C=C
- Bandes à 870—918—993 $cm^{-1}$ attribuées aux groupements $CH_2=C<$ et $CH_2=CH-$.

Exemple 14
(1R,cis)2,2-diméthyl 3-(2,2-dichloroéthényl)cyclopropane-1-carboxylate de (1S)2-méthyl 3-allyl 4-méthylène cyclopent-2-èn 1-yle

On mélange 4,40 g de 1S-hydroxy 2-méthyl 3-allyl 4-méthylène cyclopent-2-ène, 15 $cm^3$ de benzène et 2,6 $cm^3$ de pyridine, puis on ajoute à la solution obtenue refroidie à 10°C, 7 g de chlorure d'acide (1R,cis)2,2-diméthyl 3-(2,2-dichloroéthényl)cyclopropane-1-carboxylique et laisse le mélange réactionnel sous agitation à 20°C pendant 16 heures.

On ajoute 20 $cm^3$ d'eau, agite 5 minutes, dilué par 20 $cm^3$ de chlorure de méthylène, décante, extrait la phase aqueuse au chlorure de méthylène, lave les phases organiques réunies à l'eau, les sèche sur sulfate de magnésium, filtre et amène le filtrat à sec sous pression réduite. On purifie le résidu par chromatographie sur silice en éluant au mélange cyclohexaneacétate d'éthyle (95-5) contenant 0,5‰ de triéthylamine. On obtient 4,97 g de produit attendu.

$[\alpha]_D^{20} = -27,5° \pm 1,5°$ (c = 0,9%, éthanol)

Analyse ($C_{18}H_{22}O_2Cl_2$):

| | | | |
|---|---|---|---|
| Calculé: | C 63,35, | H 6,49, | Cl 20,77% |
| Trouvé: | C 63,4, | H 6,6, | Cl 20,9%. |

Spectre IR (chloroforme)

- Epaulement à 1724—1713 $cm^{-1}$ attribué au groupement C=O (ester)
- Absorption à 1633—1619 $cm^{-1}$ attribué au groupement C=C
- Absorptions à 995—919 $cm^{-1}$ attribuées au groupement $-HC=CH_2$.

Exemple 15
(1R,trans)2,2-diméthyl 3-(2,2-dichloroéthényl)cyclopropane-1-carboxylate de (1S)2-méthyl 3-allyl 4-méthylène cyclopent-2-èn 1-yle

On ajoute, en 15 minutes, 7 g de chlorure d'acide (1R,trans)2,2-diméthyl 3-(2,2-dichloroéthényl)cyclopropane-1-carboxylique dissous dans 5 ml de benzène à une solution de 4,40 g de (1S)hydroxy 2-méthyl 3-allyl 4-méthylène cyclopent-2-ène dans 15 $cm^3$ de benzène et 2,6 $cm^3$ de pyridine, puis laisse sous agitation pendant 16 heures. On ajoute alors 20 $cm^3$ d'eau, agite 5 minutes, décante, extrait la phase aqueuse au benzène, lave les phases organiques à l'eau, les sèche sur sulfate de magnésium, filtre et amène à sec sous pression réduite. On purifie l'huile résiduelle par chromatographie sur silice en éluant au mélange cyclohexaneacétate d'éthyle (95-5) contenant 1‰ de triéthylamine et recueille 6,99 g de produit attendu pur.

$[\alpha]_D^{20} = -45,5° \pm 1,5°$ (c = 1%, éthanol)

Analyse ($C_{18}H_{22}O_2Cl_2$):

| | | | |
|---|---|---|---|
| Calculé: | C 63,35, | H 6,49, | Cl 20,77%, |
| Trouvé: | C 63,3, | H 6,6, | Cl 20,3%. |

Spectre IR (chloroforme)

- Absorption à 1717 $cm^{-1}$ attribuée au groupement C=O (ester)
- Absorptions à 1633—1618 $cm^{-1}$ attribuées au groupement C=C
- Absorptions à 990—917 $cm^{-1}$ attribuées au groupement $-CH=CH_2$.

Exemple 16

(1R,cis)2,2-diméthyl 3-(2,2-dibromoéthényl)cyclopropane-1-carboxylate de (1S)2-méthyl 3-allyl 4-méthylène cyclopent-1-èn 1-yle

On met en suspension 3,49 g de 1S-hydroxy 2-méthyl 3-allyl 4-méthylène cyclopent-2-ène, dans 15 $cm^3$ benzène et 2,06 $cm^3$ de pyridine, puis ajoute en 10 minutes en maintenant à 25°C, 7,45 g de chlorure d'acide (1R,cis)2,2-diméthyl 3-(2,2-dibromoéthényl)cyclopropane-1-carboxylique dissous dans 2 $cm^3$ de benzène et laisse sous agitation pendant 20 heures à 20°C. On ajoute ensuite 20 $cm^3$ d'eau, agite 10 minutes, ajoute 20 $cm^3$ de chlorure de méthylène, agite encore dix minutes, décante, extrait la phase aqueuse au chlorure de méthylène, lave les phases organiques à l'eau, les sèche sur sulfate de magnésium, filtre et amène à sec le filtrat sous pression réduite. On purifie le résidu par 2 chromatographies successives en éluant au mélange benzène-acétate d'éthyle (7-3) contenant 1 ‰ de triéthylamine puis au mélange cyclohexane-acétate d'éthyle (95-5) contenant 1 ‰ de triéthylamine et recueille 4,983 g de produit attendu pur.

$[\alpha]_D^{20} = -3,50° \pm 2°$ (c = 0,7%, chloroforme)

Analyse ($C_{18}H_{22}O_2Br_2$):

| | | | |
|---|---|---|---|
| Calculé: | C 50,25, | H 5,15, | Br 37,15%, |
| Trouvé: | C 50,5, | H 5,2, | Br 36,8%. |

Dichroïsme circulaire (dioxanne)

- Maximum à 347 nm $\Delta\varepsilon$: +0,005
- Maximum à 330 nm $\Delta\varepsilon$: +0,010
- Maximum à 320 nm $\Delta\varepsilon$: +0,012
- Maximum à 250 nm $\Delta\varepsilon$: −3,16
- Maximum à 221 nm $\Delta\varepsilon$: −3,92.

Exemple 17

(1R,trans)2,2-diméthyl 3-(2,2-dibromoéthényl)cyclopropane-1-carboxylate de (1S)2-méthyl 3-allyl 4-méthylène cyclopent-2-èn-1-yle

A une solution de 3 g de 1S-hydroxy 2-méthyl 3-allyl 4-méthylène cyclopent-2-ène, dans 5 $cm^3$ de benzène et 5 $cm^3$ de pyridine, maintenue à 20—25°C, on ajoute en 15 minutes, 9,3 g de chlorure d'acide (1R,trans)2,2-diméthyl 3-(2,2-dibromoéthényl) cyclopropane-1-carboxylique dissous dans 30 $cm^3$ de benzène, agite pendant 20 heures à 20°C, ajoute 50 $cm^3$ d'eau et agite encore 15 minutes. On décante, extrait la phase aqueuse au benzène, lave les phases organiques réunies à l'eau, les sèche sur sulfate de magnésium, filtre et amène le filtrat à sec sous pression réduite. On chromatographie le résidu sur silice en éluant au mélange cyclohexane-acétate d'éthyle (95-5) contenant 1‰ de triéthylamine et obtient 4,05 g de produit cherché pur.

$[\alpha]_D^{20} = -41,5° \pm 2°$ (éthanol)

Analyse ($C_{18}H_{22}O_2Br_2$):

| | | | |
|---|---|---|---|
| Calculé: | C 50,25, | H 5,15, | Br 37,15%, |
| Trouvé: | C 50,9, | H 5,3, | Br 36,9%. |

Spectre RMN ($CDCl_3$, 60 MHz)

- Pic à 1,24 p. p. m. (doublet J: 5,5 Hz) attribué aux hydrogènes des $CH_3$ en position 2 du cyclopropane.
- Pic à 1,64 p. p. m. (doublet J: 5 Hz) attribué à l'hydrogène en position 1 du cyclopropane.
- Pic à 1,8 p. p. m. attribué aux hydrogènes du $CH_3$ en position 2 du cyclopentène.
- Pics à 2,12 p. p. m. et 2,24 p. p. m. (2 doublets J: 6 Hz) attribué à l'hydrogène en position 3 du cyclopropane.
- Pics de 4,67 à 5,17 p. p. m. attribués aux hydrogènes des groupements $=CH_2$ en position 3 du radical allyl et aux hydrogènes du groupement méthylène en position 4 du cyclopentène.
- Pics de 5,5 à 6,33 p. p. m. attribués à l'hydrogène en position 2 du radical allyle.
- Pic à 5,58 p. p. m. attribué à l'hydrogène en position 1 du cyclopentène.
- Pic à 6,17 p. p. m. (doublet J: 7,5 Hz) attribué à l'hydrogène en position 1 du radical éthényl dibromé.

## Exemple 18

### (1R,trans)2,2-diméthyl 3-(2-méthyl 1-propényl)cyclopropane-1-carboxylate de (1RS)2-méthyl 3-(3-méthyl 2-buténvl)4-méthylène cyclopent-2-èn 1-yle

On introduit en 15 minutes environ en maintenant la température à 25°C, 3,61 g de chlorure d'acide (1R,trans)2,2-diméthyl 3-(2-méthyl 1-propényl)cyclopropane-1-carboxylique dissous dans 5 $cm^3$ de benzène, dans un mélange de 3 g de 1RS-hydroxy 2-méthyl 3-(3-méthyl 2-buténvl) 4-méthylène cyclopent-2-ène, de 10 $cm^3$ de benzène et de 1,64 $cm^3$ de pyridine, laisse le mélange réactionnel sous agitation et sous azote pendant 16 heures à 20°C.

On ajoute ensuite 20 $cm^3$ d'eau, agite 10 minutes, ajoute 20 ml de chlorure de méthylène, agite 10 minutes, décante, extrait la phase aqueuse au chlorure de méthylène, lave les phases organiques à l'eau, les sèche sur sulfate de magnésium, filtre et amène le filtrat à sec sous pression réduite. On purifie le résidu par chromatographie sur silice en éluant au mélange cyclohexane-acétate d'éthyle (95-5) contenant 0,5‰ de triéthylamine et recueille 3,564 g de produit cherché.

$[\alpha]_D^{20} = -7,5° \pm 1°$ (c = 1,2%, éthanol)

Analyse ($C_{22}H_{32}O_2$):

| | | |
|---|---|---|
| Calculé: | C 80,44, | H 9,82%, |
| Trouvé: | C 80,3, | H 9,7%. |

Spectre RMN ($CDCl_3$, 60 MHz)

- Pic à 1,19 p. p. m. (doublet J: 8,5 Hz) attribué aux hydrogènes des $-CH_3$ en position 2 du cyclopropanol.
- Pic à 1,37 p. p. m. (doublet J: 5 Hz) attribué à l'hydrogène en position 1 du cyclopropane.
- Pic à 1,7 p. p. m. attribué aux hydrogènes des $-CH_3$ terminaux de la chaîne diméthyl allyl du cyclopentène.
- Pic à 1,78 p. p. m. attribué aux hydrogènes du $-CH_3$ en position 2 du cyclopentène.
- Pic à 2,89 p. p. m. attribué aux hydrogènes en position 1 de la chaîne diméthyl allyl du cyclopentène.
- Pics de 4,67 à 5,25 p. p. m. attribués aux hydrogènes éthyléniques.
- Pic à 5,63 p. p. m. attribué à l'hydrogène en position 1 du cyclopentène.

Le 1RS-hydroxy 2-méthyl 3-(3'-méthyl 2-butényl) 4-méthylène cyclopent-2-ène utilisé au départ de l'exemple 17 peut être préparé comme suit:

## Stade A

### 7-méthyl 3-oxo 6-octénoate d'éthyle

On introduit sous agitation et à −60°C, 1,4 g de nitrate ferrique dans 1200 $cm^3$ d'ammoniac liquide, laisse cus agitation pendant 5 minutes, ajoute 2 g de sodium (toujours à −60°C) et poursuit l'agitation pendant 10 minutes.

On introduit ensuite 104 g de sodium en 2 heures 30 minutes à −55°C ± 5°C et agite de nouveau à cette température pendant 1 heure. Au bout de ce temps, on ajoute au mélange réactionnel en 30 minutes et sans dépasser −30°C, 300 g d'acétyl acétate d'éthyle puis 1000 $cm^3$ d'éther refroidi à −20°C et laisse sous agitation pendant 5 minutes. Puis on introduit en 30 minutes sans dépasser −25°C, 289 g de 1-chloro 3-méthyl 2-butène, laisse en contact à température ambiante pendant 16 heures, ajoute 1000 $cm^3$ d'éther et progressivement sans dépasser +15°C, une solution de 250 $cm^3$ d'acide acétique dans 1000 $cm^3$ d'eau. On décante, extrait la phase aqueuse à l'éther, lave les phases organiques réunies avec de l'acide chorhydrique 2 N puis à l'eau, sèche sur sulfate des sodium et amène à sec sous pression réduite. On obtient par rectification du résidu sous pression réduite, 175,5 g de produit attendu. Eb (3 mm Hg): 98 à 107°C.

Spectre RMN ($CKCl_3$, 60 MHz)

– Pic à 1,28 p. p. m. (triplet J: 7 Hz) } attribué aux hydrogènes du groupement $-COOC_2H_5$
– Pic à 29 p. p. m. (quadruplet J: 7,5 Hz) }
– Pic à 3,62 p. p. m. attribué aux hydrogènes du groupement $-CH_2$ en position 2.
– Pic à 1,64 p. p. m. (doublet J: 3 Hz) attribué aux hydrogènes du $-CH_3$ en position 7.

Stade B

3-hydroxy 9-méthyl 8-décène 2,5-dione

On met en suspension 175 g de 7-méthyl 3-oxo 6-octénoate d'éthyle dans 875 $cm^3$ d'eau, puis ajoute en 1 heure environ sans dépasser 33°C, 97 $cm^3$ d'hydroxyde de sodium 10 N et laisse sous agitation pendant 20 heures à température ambiante. On ajoute au mélange réactionnel 25 $cm^3$ d'acide acétique pour amener à pH = 7, puis on introduit en 3 heures à 20—25°C, en maintenant le pH à 7396 g de pyruvaldéhyde en solution aqueuse (16,8% en poids) et laisse sous agitation pendant 20 heures à température ambiante (durant ce temps, on a consommé 30 $cm^3$ d'acide acétique pour maintenir le pH à 7). Puis on ajoute 1000 $cm^3$ de chlorure de méthylène, agite pendant 10 minutes, décante, extrait la phase aqueuse au chlorure de méthylène, sèche les phases organiques sur sulfate des sodium et amène à sec sous pression réduite. On obtient 175 g de produit attendu.

Stade C

1RS-hydroxy 2-méthyl 3-(3'-méthyl 2'-butényl 4-oxo cyclopent-2-ène

On fait barboter de l'azote pendant 1 heure dans 875 $cm^3$ d'hydroxyde de sodium 1 N puis introduit 175 mg d'hydroquinone, amène la température à +3°C et introduit en 1 heure à 2° ± 1°C, 175 g de 3-hydroxy 9-méthyl 8-décène 2,5-dione. On laisse le mélange réactionnel sous agitation pendant 2 heures 30 minutes à 2° ± 1°C, introduit ensuite, toujours sous agitation, 80 $cm^3$ d'acide chorhydrique concentré, agite pendant 30 minutes en laissant remonter la température à 20°C. On ajoute alors 200 g de chlorure de sodium, agite pendant 10 minutes, introduit 1000 $cm^3$ de chlorure de méthylène, décante, extrait la phase aqueuse avec du chlorure de méthylène, sèche les phases organiques réunies sur sulfate de sodium et amène à sec sous pression réduite. On obtient un résidu que l'one rectifie sous pressions réduite et recueille 53,4 g de produit cherché. Eb 0,5 mm Hg: 143 à 148°C.

Spectre RMN ($CDCl_3$, 60 MHz)

– Pic à 1,72 p. p. m. attribué aux hydrogènes des groupements $-CH_3$ en bout de la chaîne diméthyl allyle.
– Pic à 2,87 p. p. m. (doublet J: 7 Hz) attribué aux hydrogènes en position 1 de la chaîne diméthyl allyle.
– Pic à 4,7 p. p. m. attribué à l'hydrogène au pied du groupement −OH.
– Pic à 5,03 p. p. m. (triplet J: 7 Hz) attribué à l'hydrogène en position 2 de la chaîne diméthyl allyle.
– Pic à 2,67 p. p. m. attribué à l'hydrogène de la fonction −OH.
– Pics de 2,08 à 2,92 p. p. m. attribués au groupement $=CH_2$ en position 4 du cyclopentène.

Stade D

1RS-hydroxy 2-méthyl 3-(3-méthyl 2-butényl) 4-méthylène cyclopent-2-ène

On met en suspension, sous agitation, 29,7 g de bromure de triphényl méthyl phosphonium dans 100 ml d'éther et 7,84 ml de ter-butanol, puis on ajoute en 30 minutes en 6 fois, 9,93 g de ter-butylate de potassium et laisse sous agitation et sous atmosphère inerte pendant 5 heures à 20°C. On refroidit à 0°C et ajoute en 20 minutes 10 g de 1RS-hydroxy 2-méthyl 3-(3'-méthyl 2'-butényl) 4-oxo cyclopent-2-ène dissous dans 10 ml d'éther. On maintient le milieu réactionnel à 0°C pendant 16 heures sous agitation et sous atmosphère inerte, laisse revenir à 20°C, agite de nouveau pendant 3 heures, verse dans une solution aqueuse saturée de phosphate monosodique et agite pendant 15 minutes. On décante, extrait à l'éther, sèche les phases organiques réunies sur sulfate de magnésium, filtre et amène à sec le filtrat sous pression réduite. On reprend le résidu à l'éther et agite pendant 10 minutes à 0°C, essore le précipité et le lave à l'éther. On amène à sec le filtrat sous pression réduite et obtient 19,54 g de produit attendu brut que l'on purifie par chromatographie sur silice en éluant au mélange benzène-acétate d'éthyle (7-3) contenant 1‰ de triéthylamine. On recueille 7,244 g de produit pur de Rf: 0,55.

Spectre IR (chloroforme)

- Absorption complexe à 3605–3580 $cm^{-1}$ caractéristique du groupement –OH
- Absorption à 1630 $cm^{-1}$ caractéristique de >C=C<
- Absorption à 865 $cm^{-1}$ caractéristique de >=$CH_2$.

Spectre RMN ($CHCl_3$, 60 MHz)

- Pic à 1,7 p. p. m. attribué aux hydrogènes des groupements –$CH_3$ en bout de chaîne latérale.
- Pic à 1,83 p. p. m. attribué aux hydrogènes du groupement –$CH_3$ en position 2.
- Pic à 1,98 p. p. m.attribué à l'hydrogène du groupement –OH en position 1.
- Pics de 2,83 à 2,95 p. p. m. attribués aux hydrogènes du groupement –$CH_2$ en position 1 de la chaîne latérale.
- Pic à 5,03 p. p. m. (triplet J: 7 Hz) attribué aux hydrogènes en position 3 de la chaîne latérale.
- Pics de 4,42 à 4,83 p. p. m. attribué à l'hydrogène en $\alpha$ du groupement –OH et du méthylène en position 4.

Exemple 19

(1R,trans)2,2-diméthyl 3-cyclopentylidène méthyl cyclopropane-1-carboxylate de (1RS)2-méthyl 3-(3-méthyl 2-buténγl) 4-méthylène cyclopent-2-èn-1-yle

A une suspension de 3,08 g de chlorure d'acide (1R,trans)2,2-diméthyl 3-cyclopentylidène méthyl cyclopropane-1-carboxylique dans 10 $cm^3$ de benzène et 1,22 $cm^3$ de pyridine, on ajoute en 10 minutes en maintenant à 25°C, 2 g de (1RS)-hydroxy 2-méthyl 3-(3-méthyl 2-butényl) 4-méthylène cyclopent-2-ène dissous dans 5 $cm^3$ de benzène et laisse le mélange réactionnel sous agitation et sous azote pendant 16 heures à 20°C. On ajoute ensuite 20 $cm^3$ d'eau, agite 10 minutes, ajoute 20 $cm^3$ de chlorure de méthylène, agite 5 minutes, décante, extrait la phase aqueuse au chlorure de méthylène, lave les phases organiques réunies à l'eau, extrait les eaux de lavage au chlorure de méthylène, sèche les phases organiques réunies sur sulfate de magnésium, filtre et amène le filtrat à sec sous pression réduite. On purifie le résidu par chromatographie sur silice en éluant au mélange cyclohexane-acétate d'éthyle (95-5) contenant 0,5‰ de triéthylamine et recueille 2,448 g de produit cherché.

$[\alpha]_D = -19° \pm 1,5°$ (c = 0,9%, éthanol)

Analyse ($C_{24}H_{34}O_2$):

| | | |
|---|---|---|
| Calculé: | C 81,31, | H 9,66% |
| Trouvé: | C 81,4, | H 9,6% |

Spectre RMN ($CDCl_3$, 60 MHz)

- Pic à 1,20 p. p. m. (doublet J: 7,5 Hz) attribué aux hydrogènes des –$CH_3$ en position 2 du cyclopropane.
- Pic à 1,41 p. p. m. (doublet J: 5,5 Hz) attribué à l'hydrogène en position 1 du cyclopropane.
- Pic à 1,7 p. p. m. attribué aux hydrogènes des –$CH_3$ terminaux de la chaîne diméthyl allyle du cyclopentène.
- Pic à 1,78 p. p. m. attribué aux hydrogènes du –$CH_3$ en position 2 du cyclopentène.
- Pics de 1,83 à 2,23 p. p. m. attribués aux hydrogènes en position 3 du cyclopropane.
- Pic à 2,27 p. p. m. attribué aux hydrogènes en position 2 et 5 du cyclopentylidène.
- Pics de 4,75 à 5,17 p. p. m. attribués aux hydrogènes de groupement =$CH_2$ en position 4 du cyclopentène.

– Pic à 5 p. p. m. attribué à l'hydrogène en position 2 de la chaîne diméthyl allylique du cyclopentène.
– Pic à 5,62 p. p. m. attribué à l'hydrogène en position 1 du cyclopentène.

Exemple 20

(1R,trans)2,2-diméthyl 3-(2-oxo 3-tétrahydrofurannylidène méthyl)cyclopropane-1-carboxylate de (1RS)2-méthyl 3-(3-méthyl 2-buténγl) 4-méthylène cyclopent-2-èn-1-yle

On mélange 2 g de 1RS-hydroxy 2-méthyl 3-(3-méthyl 2-butényl) 4-méthylène cyclopent-2-ène, 5 $cm^3$ de benzène et 1,22 $cm^3$ de pyridine, puis on ajoute en 15 minutes 3,85 g de chlorure d'acide (1R,trans)2,2-diméthyl 3-(2-oxo 3-tétrahydrofurannylidène méthyl) cyclopropane-1-carboxylique dissous dans 30,6 $cm^3$ de benzène en maintenant la température à 25°C et laisse le mélange réactionnel sous agitation et sous azote pendant 16 heures. On ajoute ensuite 30 $cm^3$ d'eau, agite 5 minutes, ajoute 10 $cm^3$ de chlorure de méthylène, agite 5 minutes, décante, extrait la phase aqueuse au chlorure de méthylène, lave les phases organiques réunies à l'eau, les sèche sur sulfate de magnésium, filtre et amène à sec le filtrat sous pression réduite. On purifie le résidu par chromatographie sur silice en éluant au mélange benzène-acétate d'éthyle (7-3) contenant 0,5‰ de triéthylamine et recueille 2,933 g de produit cherché.

$[\alpha]_D^{20} = +1,5° \pm 1,5°$ (c = 0,7%, éthanol)

Analyse ($C_{23}H_{30}O_4$):

| | | |
|---|---|---|
| Calculé: | C 74,56, | H 8,16% |
| Trouvé: | C 73,7, | H 8,1% |

Spectre IR (chloroforme)

– Absorptions à 1753–1718 $cm^{-1}$ attribuées aux groupements C=O (lactone et ester)
– Absorptions à 1675–1633 $cm^{-1}$ attribuées aux groupements C=C
– Absorptions à 992–916 $cm^{-1}$ attribuées aux groupements $-CH=CH_2$.

Exemple 21

(1R,cis)2,2-diméthyl 3-(2,2-dibromoéthényl)cyclopropane-1-carboxylate de (1RS)2-méthyl 3-(3-méthyl 2-buténγl) 4-méthylène cyclopent-2-èn-1-yle

A une suspension de 2,35 g de chlorure d'acide (1R,cis)2,2-diméthyl 3-(2,2-dibromoéthényl) cyclopropane-1-carboxylique dans 10 $cm^3$ de benzène et 0,65 $cm^3$ de pyridine, on ajoute en 15 minutes, sans dépasser 20–25°C, 1,02 g de 1RS-hydroxy 2-méthyl 3-(3-méthyl 2-butényl) 4-méthylène cyclopent-2-ène dissous dans 5 $cm^3$ de benzène, laisse sous agitation à 20°C pendant 16 heures. On ajoute ensuite 30 $cm^3$ d'eau, agite pendant 10 minutes, ajoute 20 $cm^3$ de chlorure de méthylène, agite pendant 10 minutes, décante, extrait la phase aqueuse au chlorure de méthylène, lave les phases organiques réunies à l'eau, les sèche sur sulfate de magnésium, filtre et amène à sec le filtrat sous pression réduite. On purifie le résidu par chromatographie sur silice en éluant au mélange cyclohexane-acétate d'éthyle (95-5) contenant 0,5‰ de triéthylamine et obtient 1,87 g de produit cherché.

$[\alpha]_D^{20} = -10° \pm 1°$ (c = 1%, éthanol)

Analyse ($C_{20}H_{26}O_2Br_2$):

| | | | |
|---|---|---|---|
| Calculé: | C 52,42, | H 5,72, | Br 34,87% |
| Trouvé: | C 52,7, | H 5,9, | Br 39,8% |

Spectre RMN ($CDCl_3$, 60 MHz)

– Pic à 1,26 p. p. m. (doublet J: 2 Hz) attribué aux hydrogènes des $-CH_3$ en position 2 du cyclopropane.
– Pic à 1,7 p. p. m. attribué aux hydrogènes des $-CH_3$ en bout de chaîne diméthyl allyle en position 3 du cyclopentène.
– Pic à 1,78 p. p. m. attribué aux hydrogènes du $-CH_3$ en position 2 du cyclopentène.

- Pic à 2,92 p. p. m. (doublet J: 7 Hz) attribué aux hydrogènes en position 4 sur la chaîne diméthyl allyle du cyclopentène.
- Pics de 4,67 à 5 p. p. m. attribués aux hydrogènes du méthylène en position 4 du cyclopentène.
- Pic à 5,08 p. p. m. (triplet J: 7 Hz) attribué à l'hydrogène en position 2 de la chaîne latérale du cyclopentène.
- Pic à 5,6 p. p. m. attribué à l'hydrogène en position 1 du cyclopentène.
- Pic à 6,8 p. p. m. (doublet J: 7 Hz) attribué au dernier hydrogène éthylènique de la chaîne dibromovinylique.

### Exemple 22

(1R,trans)2,2-diméthyl 3-(cyclopentylidène methyl cyclopropane-1-carboxylate de (1S)2-méthyl 3-allyl 4-dicyano méthylène cyclopent-2-èn 1-yle

On mélange sous agitation 5 g de 1R,trans)2,2-diméthyl 3-cyclopentylidène méthyl cyclopropane-1-carboxylate de (1S)2-méthyl 3-allyl 4-oxo cyclopent-2-èn-1-yle, 20 $cm^3$ de benzène, ajoute 2 g de malonitrile, 580 mg d'acétate d'ammonium et 2,4 $cm^3$ d'acide acétique. On porte le mélange réactionnel au reflux pendant 18 heures, ajoute 3 g d'acetate d'ammonium et 2,5 $cm^3$ d'acide acétique, continue le reflux pendant 44 heures, ajoute de l'eau et extrait à l'éther. On lave les phases éthérées à l'eau, puis par une solution saturée de bicarbonate de sodium, puis à l'eau, sèche les phases organiques réunies sur sulfate de magnésium, filtre et concentre le filtrat à sec sous pression réduite. On chromatographie le résidu sur silice en éluant au mélange benzène-acétate d'éthyl-triéthylamine (95-5-0,1) et recueille 1,296 g de produite cherché.

$[\alpha]_D^{20} = -130° \pm 2,5°$ (c = 0,6%, éthanol)

Analyse ($C_{24}H_{28}O_2N_2$):

| | | | |
|---|---|---|---|
| Calculé: | C 76,56, | H 7,5, | N 7,4% |
| Trouvé: | C 76,5, | H 7,7, | N7,1% |

Spectre IR (chloroforme)

- Absorption à 2229 $cm^{-1}$ attribuée aux fonctions C≡N
- Absorption à 1725 $cm^{-1}$ attribuée au groupement C=O (ester)
- Absorption de 1640—1617 $cm^{-1}$ attribuée aux groupements C=C.

### Exemple 23

(1R,trans)2,2-diméthyl 3-cyclopentylidène méthyl cyclopropane-1-carboxylate de (1S)2-méthyl 3-allyl 4-dichlorométhylène cyclopent-2-èn 1-yle

On mélange sous atmosphère inerte, 128 $cm^3$ d'heptane, 6 $cm^3$ de tert-butanol et 7,08 g de tert-butylate de potassium, ajoute 11,1 g de triphénylphosphine, refroidit à −20°C, introduit sous agitation en 1 heure 15 minutes, 7,56 g de chloroforme dissous dans 36 $cm^3$ d'heptane et laisse le mélange réactionnel pendant 20 heures à −20°C sous agitation. On obtient 160 $cm^3$ de solution d'ylide dont on prélève 80 $cm^3$ que l'on conserve à −20°C.

On ajoute aux 80 $cm^3$ de solution restante, à −20°C en 20 minutes, 2,3 g de (1R,trans)2,2-diméthyl 3-cyclopentylidène méthyl cyclopropane-1R-carboxylate de (1S)2-méthyl 3-allyl 4-oxo cyclopent-2-èn-1-yle en solution dans 15 $cm^3$ de tétrahydrofuranne, laisse remonter la température à 25°C et laisse en contact pendant 6 heures. On refroidit à −20°C, introduit les 80 $cm^3$ de solution d'ylide conservée à −20°C, laisse revenir à température ambiante et abandonne pendant 16 heures sous agitation et sous atmosphère inerte. On filtre, lave à l'éther, verse le filtrat sur une solution glacée de phosphate monosodique, agite, décante, extrait la phase aqueuse à l'éther, lave les phases organiques réunies à l'eau jusqu'à pH = 7, les sèche sur sulfate de magnésium, filtre et concentre à sec le filtrat sous pression réduite. On chromatographie le résidu sur silice en éluant au benzène contenant 1‰ de triéthylamine et recueille 2,22 g de produit attendu.

$[\alpha]_D^{20} = -89° \pm 2,5°$ (c = 0,65%, éthanol)

Analyse ($C_{22}H_{28}O_2Cl_2$):

| | | | |
|---|---|---|---|
| Calculé: | C 66,83, | H 7,14, | Cl 17,94%, |
| Trouvé: | C 66,9, | H 7,2, | Cl 17,7%. |

Spectre RMN ($CDCl_3$, 60 MHz)

- Pic à 1,21 p. p. m. (doublet J: 7 Hz) attribué aux hydrogènes des $-CH_3$ en position 2 du cyclopropane.
- Pic à 1,42 p. p. m. (doublet J: 5,5 Hz) attribué à l'hydrogène en position 1 du cyclopropane.
- Pic à 1,78 p. p. m. attribué aux hydrogènes en position 3 de la chaîne allyle.
- Pics de 4,83 à 5,12 p. p. m. attribués aux hydrogènes du méthylène en position 4 du cyclopentène et à l'hydrogène éthylénique en $\alpha$ du cyclopropane.
- Pic de 5,33 à 5,17 p. p. m. attribué à l'hydrogène en position 2 de la chaîne allyle et en position 1 du cyclopentène.

Exemple 24

(1R,cis)2,2-diméthyl 3-(2,2-difluoroéthényl) cyclopropane-1-carboxylate de (1S)2-méthyl 3-allyl 4-dichlorométhylène cyclopent-2-èn 1-yle

On refroidit à $-20$°C un mélange de 128 $cm^3$ d'heptane, 6 $cm^3$ de tert-butanol, 7,08 g de tert-butylate de potassium, et 11,1 g de triphénylphosphine, puis introduit en 1 heure toujours à $-20$°C, une solution de 7,56 g de chloroforme dans 36 $cm^3$ d'heptane et laisse le mélange réactionnel pendant 20 heures sous agitation à $-20$°C. On obtient ainsi 160 $cm^3$ de solution d'ylide dont 80 $cm^3$ sont conservés à $-20$°C. Dans les 80 $cm^3$ de solution restante, on coule à $-20$°C sous agitation et sous atmosphère d'azote, en 20 minutes environ, 2,2 g de (1R,cis)2,2-diméthyl 3-(2,2-difluoroéthényl) cyclopropane-1-carboxylate de (1S)2-méthyl 3-allyl 4-oxo cyclopent 2-èn-1-yle dissous dans 15 $cm^3$ de tétrahydrofurane, laisse remonter la température à 25°C et laisse en contact à cette température pendant 6 heures. On refroidit ensuite à $-20$°C, introduit rapidement les 80 $cm^3$ de solution d'ylide conservée à $-20$°C, laisse revenir à température ambiante et abandonne sous agitation et sous atmosphère inerte pendant 16 heures. On filtre, lave à l'éther, ajoute du phosphate monosodique au filtrat, agite, décante, extrait la phase aqueuse à l'éther, sèche les phases organiques réunies sur sulfate de sodium, filtre et amène le filtrat à sec pression réduite. Après empâtage à l'éther, filtration et évaporation, on obtient une huile que l'on chromatographie sur silice en éluant au mélange benzène-cyclohexane (4-6) contenant 1‰ de triéthylamine et recueille 2 g de produit attendu.

$[\alpha]_D^{20} = -28° \pm 1,5°$ (c = 0,8%, benzène)

Analyse ($C_{18}H_{20}Cl_2F_2O_2$):

| | | | | |
|---|---|---|---|---|
| Calculé: | C 57,30, | H 5,35, | Cl 18,8, | F 10,07%, |
| Trouvé: | C 56,9, | H 5,4, | Cl 19,0, | F 10,2%. |

Spectre IR (chloroforme)

- Absorption à 1742 $cm^{-1}$ attribuée au groupement:

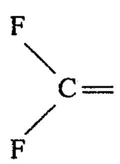

- Absorption à 1720 $cm^{-1}$ attribuée au groupement:

  $C{=}O$ (ester)

- Absorption à 1647 $cm^{-1}$ attribuée au groupement:

  $CF_2{=}CH$

– Absorption à 1603 $cm^{-1}$ attribuée au groupement:

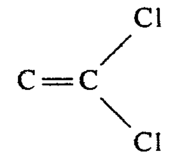

Exemple 25
(1R,trans)2,2-diméthyl 3-(2,2-difluoroéthényl)cyclopropane-1-carboxylate de (1S)2-méthyl 3-allyl 4-dichlorométhylène cyclopent-2-èn 1-yle

On mélange 2,3 g de (1S)hydroxy 2-méthyl 3-allyl 4-dichlorométhylène cyclopent-2-ène, 30 $cm^3$ de benzène et 3 $cm^3$ de pyridine, puis on introduit sous agitation à 25–30°C, 1,95 g de chlorure de l'acide (1R,trans)2,2-diméthyl 3-(2,2-difluoroéthényl) cyclopropane-1-carboxylique dissous dans 15 $cm^3$ de benzène et laise sous agitation pendant 16 heures à 20°C. On verse dans l'eau, extrait à l'éther isopropylique, sèche sur sulfate de sodium, filtre et amène le filtrat à sec sous pression réduite. On purifie le résidu par chromatographie sur silice en éluant au mélange benzène-cyclohexane (4-6) contenant 1‰ de triéthylamine et obtient 2,1 g de produit attendu.

$[\alpha]_D^{20} = -77,5° \pm 1,5°$ (c = 1%, benzène)

Analyse ($C_{18}H_{20}Cl_2F_2O_2$):

| | | | | |
|---|---|---|---|---|
| Calculé: | C 57,30, | H 5,34, | Cl 18,80, | F 10,07% |
| Trouvé: | C 57,3, | H 5,2, | Cl 19,8, | F 9,6% |

Spectre IR (chloroforme)

– Absorption à 1745 $cm^{-1}$ attribée au groupement:

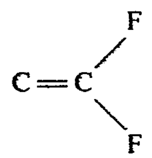

– Absorption à 1720 $cm^{-1}$ attribuée au groupement: C=O (ester)
– Absorptions à 1638–1605 $cm^{-1}$ attribuées aux groupements C=C et C=C conjugué.

Le (1S)hydroxy 2-méthyl 3-allyl 4-dichlorométhylène cyclopent-2-ène utilisé au départ de l'exemple 25 peut être obtenu de la manière qui suit.

Stade A
(1S)-acétoxy 2-méthyl 3-allyl 4-oxo cyclopent-2-éne

On dissout 2 g de (1S)-hydroxy 2-méthyl 3-allyl 4-oxo cyclopent-2-ène dans 15 ml de chlorure de méthylène puis ajoute 9,15 ml de triéthylamine et 3,1 ml d'anhydride acétique. Après 30 minutes de réaction, on verse sur une solution aqueuse saturée de phosphate monosodique, extrait à l'éther, lave à l'eau, sèche sur sulfate de magnésium, filtre et amène le filtrat à sec. On obtient 3,049 g de produit attendu.

Spectre RMN ($CDCl_3$, 90 MHz)

– Pics à 1,67 et 2,02 p. p. m. attribués aux hydrogènes du radical $-CH_3$ en position 2 et du groupement acétoxy en position 1.
– Pics de 2,11 à 3,01 p. p. m. attribués aux hydrogènes en position 5 du cycle et en position 1 de la chaîne allylique.
– Pics de 4,94 à 5,09 p. p. m. attribués aux hydrogènes en 3 de la chaîne allylique.
– Pics de 5,55 à 6 p. p. m. attribués à l'hydrogène en position 2 de la chaîne allylique.

Stade B

(1S)-acétoxy 2-méthyl 3-allyl 4-dichloro méthylène cyclopent-2-ène

On met sous agitation à température ambiante, un mélange de 111 g de triphényl phosphine broyé, 1000 $cm^3$ d'heptane, 60 $cm^3$ de ter-butanol et 70,8 g de ter-butylate de potassium. On refroidit à −20°C et introduit en 1 heure 30 minutes, sous agitation une solution de 75,6 g de chloroforme dans 60 $cm^3$ d'heptane et maintient l'agitation pendant 5 heures à −20°C et laisse 16 heures au repos à cette température. On obtient une solution d'ylide dont on prélève environ la moitié que l'on conserve sous azote à −20°C. A la moitié restante on ajoute à −20°C sous atmosphère inerte et sous agitation 26 g de (1S)-acétoxy 2-méthyl 3-allyl 4-oxo cyclopent-2-ène en solution dans 140 $cm^3$ de tétrahydrofuranne, laisse revenir à température ambiante et agite 5 heures à cette température. On introduit alors à −20°C, la 2ème partie d'ylide conservée sous azote à −20°C, laisse revenir la température à 20°C et agite pendant 16 heures à cette température et sous atmosphère inerte. On filtre, ajoute au filtrat une solution aqueuse de phosphate monosodique, extrait à l'éther, sèche les phases organiques réunies sur sulfate de sodium, filtre et amène à sec sous pression réduite. On purifie le résidu par chromatographie sur silice en éluant au mélange benzène-acétate d'éthyle (8-2) contenant 1‰ de triéthylamine et recueille 14 g de produit attendu.

Stade C

(1S)-hydroxy 2-méthyl 3-allyl 4-dichloro méthylène cyclopent-2-ène

On dissout 14 g du produit obtenu au stade B dans 350 $cm^3$ d'éthanol et ajoute sous agitation 9,3 g de carbonate de sodium dans 195 $cm^3$ d'eau, ajoute un peu de dioxanne, agite pendant 4 jours à 20°C. On amène à sec, reprend le résidu à l'eau, extrait à l'éther isopropylique, sèche la phase organique sur sulfate de sodium, filtre et amène à sec sous pression réduite. On recueille 10,9 g de produit attendu.

Spectre IR (chloroforme)

- Absorptions à 3600—3587 $cm^{-1}$ attribuées à la fonction −OH
- Absorptions à 1635—1600 $cm^{-1}$ attribuées au groupement C=C.

Spectre RMN ($CDCl_3$, 60 MHz)

- Pics de 1,77 à 3,17 p. p. m. attribués aux hydrogènes en position 5 du cycle.
- Pic à 1,83 p. p. m. attribué au groupement $-CH_3$ en position 2 du cycle.
- Pic à 3,27 p. p. m. (doublet J: 7 Hz) attribué aux hydrogènes en position 1 de la chaîne allylique.
- Pics de 4,5 à 4,67 p. p. m. attribués à l'hydrogène en position 1 du cycle.
- Pics de 4,83 à 5,25 p. p. m. attribués aux hydrogènes en position 3 de la chaîne allylique.
- Pics de 5,5 à 6,33 p. p. m. attribués à l'hydrogène en position 2 de la chaîne allylique.

Exemple 26

(1R,cis)2,2-diméthyl 3-(2-fluoro 2-chloroéthényl)cyclopropane-1-carboxylate de (1S)2-méthyl 3-allyl 4-dichlorométhylène cyclopent-2-èn 1-yle

Dans un mélange de 2,3 g de (1S)-hydroxy 2-méthyl 3-allyl 4-dichlorométhylène cyclopent-2-ène, de 23 $cm^3$ de benzène et de 4 $cm^3$ de pyridine, on ajoute goutte à goutte à 25—30°C sous agitation, 2,1 g de chlorure de l'acide (1R,cis)2,2-diméthyl 3-(2-fluoro 2-chloroéthényl)cyclopropane-1-carboxylique dissous dans 10 $cm^3$ de benzène, maintient l'agitation pendant 16 heures puis verse dans l'eau. On extrait la phase aqueuse à l'éther, sèche les phases organiques réunies sur sulfate de sodium, filtre et amène le filtrat à sec sous pression réduite. On purifie le résidu par chromatographie sur silice en éluant au mélange benzène-cyclohexane (4-6) contenant 1‰ de triéthylamine et recueille 2,2 g de produit attendu.

$[\alpha]_D^{20} = -13,5° \pm 2°$ (c = 0,42%, benzène)

Analyse ($C_{18}H_{20}Cl_3FO_2$):

| | | | | |
|---|---|---|---|---|
| Calculé: | C 54,91, | H 5,12, | Cl 27,01, | F 4,83% |
| Trouvé: | C 55,1, | H 5,2, | Cl 26,8, | F 4,9% |

Spectre IR (chloroforme)

– Absorption à 1720 $cm^{-1}$ attribuée au groupement C=O (ester)
– Absorption à 1675 $cm^{-1}$ attribuée au groupement:

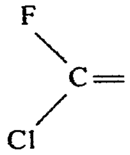

– absorptions à 1675–1637–1605 $cm^{-1}$ attribuées aux groupements:

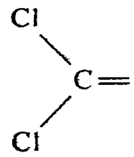 C=C conjugué et C=C

Exemple 27
(1R,cis)2,2-diméthyl 3-(2,2-dibromoéthényl)cyclopropane-1-carboxylate de (1S)2-méthyl 3-allyl 4-dichlorométhylène cyclopent-2-èn 1-yle

On mélange 2,3 g de (1S)-hydroxy 2-méthyl 3-allyl 4-dichlorométhylène cyclopent-2-ène, 30 $cm^3$ de benzène et 3,5 $cm^3$ de pyridine, puis ajoute goutte à goutte à 25–30°C, 3,2 g de chlorure de l'acide (1R,cis)2,2-diméthyl 3-(2,2-dibromoéthényl)cyclopropane-1-carboxylique dissous dans 15 $cm^3$ de benzène puis agite à 20°C pendant 16 heures. On précipite le mélange dans l'eau, décante, extrait la phase aqueuse à l'éther isopropylique, sèche les phases organiques réunies sur sulfate de sodium, filtre et amène sec sous pression réduite. On chromatographie le résidu sur silice en éluant au mélange benzène-cyclohexane (4-6) contenant 2% de triéthylamine et obtient 2,2 g de produit attendu.

$[\alpha]_D^{20} = -6° \pm 2°$ (c = 0,7%, benzène)

Analyse ($C_{18}H_{20}Cl_2Br_2O_2$):

| | | | | |
|---|---|---|---|---|
| Calculé: | C 43,31, | H 4,04, | Br 32,03, | Cl 14,21% |
| Trouvé: | C 43,8, | H 4,0, | Br 31,8, | Cl 14,1% |

Spectre IR (chloroforme)

– Absorption à 1721 $cm^{-1}$ attribuée au groupement C=O (ester)
– Absorptions à 1640–1607 $cm^{-1}$ attribuées aux groupements, C=C et C=C conjugué
– Absorption à 997 $cm^{-1}$ attribuée au groupement $C=CH_2$.

Exemple 28
(1R,trans)2,2-diméthyl 3-(2,2-difluoroéthényl)cyclopropane-1-carboxylate de (1S)2-méthyl 3-allyl 4-méthylène cyclopent-2-èn 1-yle

On refroidit à +10°C une solution de 3 g de (1S)-hydroxy 2-méthyl 3-allyl 4-méthylène cyclopent-2-ène dans 30 $cm^3$ de benzène et 9 $cm^3$ de pyridine, ajoute en 5 minutes 3,88 g de chlorure d'acide (1R,trans)2,2-diméthyl 3-(2,2-difluoroéthényl)cyclopropane-1-carboxylique, agite pendant 4 heures à 20–25°C puis verse sur un mélange eau-glace. On décante, extrait la phase aqueuse à l'éther isopropylique, lave les phases organiques réunies à l'eau, les sèche sur sulfate de sodium et concentre à sec sous pression réduite.

On chromatographie l'huile obtenue sur silice en éluant au mélange cyclohexane-benzène (6-4) à 1‰ de triéthylamine et obtient 5,6 g de produit attendu pur.

Analyse ($C_{18}H_{22}F_2O_2$):

| | | | |
|---|---|---|---|
| Calculé: | C 70,11, | H 7,19, | F 12,32% |
| Trouvé: | C 70,4, | H 7,3, | F 12,2% |

Spectre IR (chloroforme)

- Absorption à 1742 $cm^{-1}$ attribuée au groupement $F_2C{=}CH-$
- Absorption à 1713 $cm^{-1}$ attribuée au groupement C=O (ester)
- Absorption à 1635 $cm^{-1}$ attribuée au groupement $CH_2{=}C<$.

### Exemple 29
### (d) α-isopropyl α-(4-chlorophényl)acétate de (1S)2-méthyl 3-allyl 4-dichlorométhylène cyclopent-2-èn 1-yle

On introduit goutte à goutte, à 25—30°C et sous agitation 2,3 g de chlorure de l'acide (d) α-isopropyl α-(4-chlorophényl)acétique dissous dans 10 $cm^3$ de benzène dans un mélange de 2,3 g de (1S)-hydroxy 2-méthyl 3-allyl 4-dichlorométhylène cyclopent-2-ène, 23 $cm^3$ de benzène et 4 $cm^3$ de pyridine, agite pendant 16 heures à température ambiante puis verse dans l'eau. On extrait la phase aqueuse à l'éther isopropylique, sèches les phases organiques réunies sur sulfate de sodium, filtre et amène le filtrat à sec sous pression réduite. On chromatographie le résidu sur silice en éluant au mélange benzène-cyclohexane (4-6) contenant 1‰ triéthylamine et recueille 2,7 g des produit attendu.

$[\alpha]_D^{20} = -105° \pm 3°$ (c = 0,46% benzène)

Analyse ($C_{21}H_{23}Cl_3O_2$):

| | | | |
|---|---|---|---|
| Calculé: | C 60,96, | H 5,60, | Cl 25,76% |
| Trouvé: | C 61,0, | H 5,6, | Cl 25,8% |

Spectre IR (chloroforme)

- Absorption à 1728 $cm^{-1}$ attribuée au groupement C=O (ester)
- Absorption à 1638 $cm^{-1}$ attribuée au groupement C=C
- Absorptions à 1604—1494 $cm^{-1}$ attribuées au groupement C=C conjugué et noy aux aromatiques.

### Exemple 30
### (1R,cis)2,2-diméthyl 3-(2,2-dichloroéthényl)cyclopropane-1-carboxylate de (1S)2-méthyl 3-allyl 4-dichlorométhylène cyclopent-2-èn-1-yle

Dans une solution de 2,3 g de (S) dichlorométhylène alléthrolone dans 30 $cm^3$ de benzène et 5 $cm^3$ de pyridine, on introduit à 20°C, une solution de 2,2 g de chlorure de l'acide (1R,cis)2,2-diméthyl 3-(2',2'-dichloroéthényl) cyclopropane carboxylique dans 5 $cm^3$ de benzène, agite pendant 16 heures à 20°C, verse le mélange réactionnel dans l'eau, extrait à l'éther éthylique et après les traitements habituels concentre à sec, chromatographie sur gel de silice en éluant avec un mélange de benzène et de cyclohexane (7-3) contenant 1‰ de triéthylamine et obtient 1,9 g de composé attendu.

$[\alpha]_D^{20} = -9°$ (c = 0,2% benzène)

Spectre de RMN (deutéro chloroforme)

- Pics à 1,26—1,27 p. p. m. attribués aux hydrogènes des $-CH_3$ en position 2 du cyclopropane.
- Pics à 1,78—1,80 p. p. m. attribués aux hydrogènes du méthyle en position 2 du cyclopentène.
- Pics à 4,78—5,16 p. p. m. attribués aux hydrogènes du méthylène de la chaîne allylique en position 3 du cyclopentène.
- Pics à 5,47—5,58 p. p. m. attribués à l'hydrogène en position 1 du cyclopentène.
- Pics à 5,50—6,33 p. p. m. attribués à l'hydrogène en position 2 de la chaîne allylique du cyclopentène.
- Pics à 6,20—6,30 p. p. m. attribués à l'hydrogène en position 1 du radical dichlorovinyl en position 3 du cyclopropyle.

Exemple 31

(1R,cis)2,2-diméthyl 3-/(E) 3-oxo 1-buténylicyclopropane carboxylate de (1S)2-méthyl 3-allyl 4-méthylène cyclopent-2-èn 1-yle

On mélange 3,3 g de chlorure de l'acide (1R,cis)2,2-diméthyl 3-/(E) 3-oxo 1-buténylicyclopropane carboxylique, 2,71 g de (1S)hydroxy 2-méthyl 3-allyl 4-méthylène cyclopent-2-ène et 50 $cm^3$ de benzène puis ajoute à +5°C, 1,5 $cm^3$ de pyridine.

On maintient sous agitation à +5°C pendant 15 minutes puis à 20°C pendant deux heures. On verse dans un mélange de 20 $cm^3$ d'acide chlorhydrique 2 N et 100 $cm^3$ d'eau à +5°C, décante puis extrait à l'éther. On lave les phases éthérées à l'eau, les sèche et évapore le solvant.

On obtient 7 g de produit attendu que l'on chromatographie sur silice en éluant au mélange cyclohexane-acétate d'éthyle: 95-5 à 1 pour mille de triéthylamine.

On obtient 1,75 g de produit attendu.

$[\alpha]_D^{20} = -47° \pm 2°$ (c = 0,5%, benzène)

Analyse ($C_{20}H_{26}O_3$) (314,42):

| | | |
|---|---|---|
| Calculé: | C 76,4, | H 8,33% |
| Trouvé: | C 76,1, | H 8,3% |

Spectre RMN ($CDCl_3$, 60 MHz)

Pic à 2,25 p. p. m. attribué aux hydrogènes en 4 du butényle.
Pics de 6,05 à 6,32 p. p. m. attribués à l'hydrogène en 2 du butényle.
Pics de 6,09 à 7,5 p. p. m. caractéristiques de l'hydrogène en 1 du butényle.
Pics de 1,25 à 1,37 p. p. m. attribués aux hydrogènes des méthyles en 2 du cycle cyclopropane.
Pics de 2,5 à 5,75 p. p. m. caractéristiques de l'hydrogène en 1 du cycle cyclopentyle.
Pic à 1,77 p. p. m. attribué aux hydrogènes du méthyle en 2 du cycle cyclopentyle.
Pics de 5,48 à 6,13 p. p. m. caractéristiques de l'hydrogène en 2 de l'allyle.
Pics de 4,75 à 5,25 p. p. m. caracteristiques des hydrogènes en 3 de l'allyle et du méthylène en 4.

Le chlorure d'acide utilisé au départ de l'exemple 31 à été préparé comme suit:

Stade A

Acide (1R,cis)2,2-diméthyl 3-/(E) 3-oxo 1-buténylicyclopropane carboxylique

On mélange 14,21 g de lactone de l'acide cis 2,2-diméthyl 3S-(dihydroxy méthyl)cyclopropane-1R-carboxylique, 63,7 g d'acétonylidène triphényl phosphorane préparé selon le mode opératoire donné ci-dessous, 1000 $cm^3$ de monoglyme et 1200 $cm^3$ de benzène. On porte au reflux pendant 4 heures, refroidit à température ambiante et évapore les solvants.

On reprend l'huile obtenue dans un mélange de 200 $cm^3$ de soude 1 N et 400 $cm^3$ de chlorure de méthylène, on agite le mélange, sépare la phase aqueuse, la lave au chlorure de méthylène, puis l'acidifie à pH 1 par addition d'acide chlorhydrique concentré. On extrait à l'éther, lave les phases éthérées à l'eau, les sèche, et les concentre à sec.

On obtient 13,5 g de produit attendu que l'on recristallise dans l'éther isopropylique.

On obtient 10,95 g des produit attendu. F = 120°C.

$[\alpha]_D^{20} = -35° \pm 2°$ (c = 0,6%, benzène)

Spectre IR (chloroforme)

Absorption à 3500 $cm^{-1}$ caractéristique de l'OH
Absorptions à 1750 et 1755 $cm^{-1}$ caractéristiques du carbonyle
Absorption a 1665 $cm^{-1}$ caractéristique de la fonction cétone
Absorptions à 1612 $cm^{-1}$ et 980 $cm^{-1}$ caracteristiques de la fonction C=C.

Stade B
Chlorure de l'acide (1R,cis)2,2-diméthyl 3-/(E) 3-oxo 1-buténył/cyclopropane carboxylique

On dissout 3 g de l'acide obtenu au stade A dans 15 $cm^3$ de chlorure de méthylène et 15 $cm^3$ d'isoprène.

On ajoute entre +5 et +7 °C 2 $cm^3$ de chlorure de thionyle.

On maintient sous agitation à 5 °C pendant 15 minutes, puis à 20 °C pendant trois heures. On élimine les solvants sous pression réduite, reprend le résidu au benzène, évapore le solvant et obtient 3,3 g de produit attendu brut utilisé tel quel pour la suite de la synthèse.

Préparation de l'acétonylidène triphényl phorphorane:

1) Chlorure d'acétonyltriphényl phosphonium

On dissout 55 g de triphényl phosphine dans 15,5 $cm^3$ de chloracétone et 165 $cm^3$ de chloroforme. On porte au reflux 45 minutes, refroidit à 20 °C, verse le mélange réactionnel dans 1,65 l d'éther éthylique, essore, lave à l'éther, sèche sous pression réduite et obtient 26,9 g de produit utilisé tel quel pour le stade suivant.

2) Acétonylidène triphényl phosphorane

On introduit le produit obtenu au stade précédent dans 270 $cm^3$ d'une solution aqueuse de carbonate de sodium à 10% agite 16 heures à température ambiante, essore, lave à l'eau, sèche sous pression réduite et obtient 20,3 g de produit brut que l'on recristallise dans du méthanol à 50% d'eau.

On obtient 16 g de produit attendu. F = 207—208 °C.

Spectre U. V. (Ethanol)

| | | |
|---|---|---|
| Infl. vers 220 nm | $E_1^1$ = 906 | |
| Max. vers 260 nm | $E_1^1$ = 201 | $\varepsilon$ = 6400 |
| Max. vers 267 nm | $E_1^1$ = 209 | $\varepsilon$ = 6650 |
| Max. vers 274 nm | $E_1^1$ = 205 | $\varepsilon$ = 6500 |
| Infl. vers 283 nm | $E_1^1$ = 179 | |

Exemple 32
(1R,trans)2,2-diméthyl 3-(E)3-oxo 1-buténył cyclopropane carboxylate de (1S)2-méthyl 3-allyl 4-méthylène cyclopent-2-èn 1-yle

On dissout 3,29 g de (1S)-hydroxy 2-méthyl 3-allyl 3-méthylène cyclopent-2-ène dans 20 $cm^3$ de benzène puis ajoute 5 $cm^3$ de pyridine. On agite à 20 °C puis ajoute en 15 minutes le chlorure de l'acide (1R,trans)2,2-diméthyl 3-/(E)3-oxo 1-butényl) cyclopropane carboxylique préparé ci-dessous, dissous dans 20 $cm^3$ de benzène. On maintient sous agitation pendant 17 heures à 20 °C, verse le mélange réactionnel dans l'eau et extrait au chlorure de méthylène. On sèche les phases organiques et évapore le solvant. On obtient 12,7 g de produit attendu que l'on chromatographie sur silice en éluant au mélange cyclohexane-acétate d'éthyle (9-1) à 1 pour 1000 de triéthylamine.

On obtient ainsi 3,39 g de produit attendu.

Analyse ($C_{20}H_{26}O_3$) (314,43):

| | | |
|---|---|---|
| Calculé: | C 76,39, | H 8,34% |
| Trouvé: | C 76,6, | H 8,6% |

Spectre RMN ($CDCl_3$, 60 MHz)

Pic à 2,2 p. p. m. attribué aux hydrogènes en position 4 du buténylе.
Pics de 6,08 à 6,35 p. p. m. caractéristiques de l'hydrogène en position 2 du buténylе.
Pics de 6,37 à 6,5 p. p. m. et de 6,63 à 6,76 p. p. m. caractéristiques de l'hydrogène en position 1 du buténylе.
Pics de 1,25 à 1,3 p. p. m. caractéristiques des hydrogènes et méthyles en 2 du cyclo cyclopropane.

Pics de 4,67 à 6,08 p. p. m. caractéristiques de l'hydrogène en position 1 du cycle cyclopentyle.
Pic à 1,78 p. p. m. caractéristique des hydrogènes du méthyle en 2 du cycle cyclopentyle.

Le chlorure d'acide utilisé au départ de l'exemple 32 a été préparé comme suit:

Stade A
Acide (1R,trans)2,2-diméthyl 3-(E)3-oxo 1-buténylcyclopropane carboxylique

On mélange 10,66 g d'acide (1R,trans)2,2-diméthyl 3-formyl cyclopropane-1-carboxylique, 47,76 g d'acétonylidène triphényl phosphorane avec 600 $cm^3$ de benzène et 60 $cm^3$ de monoglyme.
On porte le mélange réactionnel au reflux pendant 4 heures puis évapore les solvants, on reprend le résidu dans un mélange de 200 $cm^3$ de soude 1 N et 400 $cm^3$ de chlorure de méthylène, agite énergiquement, sépare la phase aqueuse, et la lave au chlorure de méthylène. On acidifie ensuite cette phase aqueuse à pH 1 par addition d'acide chlorhydrique concentré, extrait à l'éther, sèche la phase étherée et évapore le solvant. On obtient 13 g de produit attendu que l'on recristallise dans le toluène. On obtient 10,28 g de produit attendu. F = 130°C.

$[\alpha]_D^{20} = +112,5° \pm 3°$ (c = 0,4%, benzène)

Spectre IR (chloroforme)

Absorption à 3500 $cm^{-1}$ (hydroxyle)
Absorptions à 1739 $cm^{-1}$ et 1695 $cm^{-1}$ caractéristiques du carbonyle de l'acide
Absorption à 1657 $cm^{-1}$ caractéristique de la cétone
Absorptions à 1614 $cm^{-1}$ et à 975 $cm^{-1}$ caractéristiques de la double liaison C=C

Stade B
Chlorure de l'acide (1R,trans)2,2-diméthyl 3-/(E) 3-oxo 1-butényl/cyclopropane carboxylique

On mélange 4 g de l'acide obtenu au stade A avec 20 $cm^3$ d'isopropène et 3 $cm^3$ de chlorure de thionyle.
On agite le mélange à 20°C pendant 5 heures, évapore le solvant sous pression réduite et obtient un produit brut que l'on utilise tel quel pour le stade suivant.

Exemple 33
(1R,trans)2,2-diméthyl 3-/ 2-méthoxy carbonyl (E) éthényl/cyclopropane carboxylate de (1S)2-méthyl 3-allyl 4-méthylène cyclopent-2-èn 1-yle

On dissout 3,03 g de (1S)-hydroxy 2-méthyl 3-allyl 4-méthylène cyclopent-2-ène dans 20 $cm^3$ de benzène puis ajoute 4 $cm^3$ de pyridine. On agite la solution à 20°C puis ajoute en 15 minutes, le chlorure d'acide préparé ci-dessous, dissous dans 15 $cm^3$ de benzène.
On agite le mélange pendant 17 heures à 20°C puis le verse dans l'eau puis l'extrait au chlorure de méthylène. On sèche la phase organique et évapore le solvant.
On obtient 9,6 g de produit brut que l'on chromatographie sur silice en éluant au mélange cyclohexane-acétate d'éthyle (9-1) à 1 pour 1000 de triéthylamine.
On obtient ainsi 3,08 g de produit attendu.

$[\alpha]_D^{20} = -20° 5 \pm 1° 5$ (c = 0,8%, chloroforme)

Spectre RMN ($CDCl_3$, 60 MHz)

Pic à 3,73 p. p. m. caractéristique des hydrogènes du méthoxy Pics de 5,82 à 6,08 p. p. m. caractéristiques de l'hydrogène en 2 de l'éthényle.
Pics de 6,46 à 6,63 et de 6,73 à 6,9 p. p. m. caractéristiques de l'hydrogène en 1 de L-éthényle.
Pics de 1,23 à 1,28 p. p. m. caractéristiques des hydrogènes des méthyles en position 2 du cycle cyclopropane.
Pic à 1,77 p. p. m. caractéristique des hydrogènes du méthyle en 2 du cycle cyclopentyle.
Pics de 4,67 à 6,08 p. p. m. caractéristiques des autres hydrogènes des substituants du cycle cyclopentyle et de l'hydrogène en 1 du cycle cyclopentyle.

Le chlorure d'acide utilisé au départ de l'exemple 33 a été préparé comme suit:

Stade A
Bromure de triphényl acétate de méthyle phosphonium

On mélange 131,14 g de triphényl phosphine avec 600 $cm^3$ de benzène puis ajoute à 20°C 76,5 g de bromoacétate de méthyle. On agite la suspension obtenue pendant une heure trente à 20°C puis pendant deux heures à 10°C. On filtre, lave au benzène puis à l'éther de pétrole. On sèche sous pression réduite et obtient 135,8 g de produit attendu. F = 200°C.

Stade B
Acide (1R,trans)2,2-diméthyl 3-/ 2-méthoxy carbonyl (E) éthényl/cyclopropane carboxylique

On mélange 7,19 g d'hydrure de sodium à 60% dans l'huile minérale à 200 $cm^3$ de tétrahydrofurane, agite la suspension à 20°C et puis ajoute 75,95 g du produit obtenu au stade A. On agite la suspension obtenue à 20°C pendant trois heures puis refroidit à 0°C et ajoute 20 g d'acide (1R,trans)2,2-diméthyl 3-formyl cyclopropane carboxylique, dissous dans 100 $cm^3$ de tétrahydrofuranne. On agite ensuite à 0°C pendant deux heures 30 puis laisse remonter la température à 20°C et agite pendant 16 heures.

On verse le mélange réactionnel dans un mélange de 20 $cm^3$ d'acide chlorhydrique concentré et de glace, extrait au chlorure de méthylène, lave la phase organique à l'eau puis la sèche et évapore le solvant. On obtient 65,3 g de produit attendu que l'on chromatographie sur silice en éluant au mélange benzène-acétate d'éthyle (8-2) à 1 pour 1000 d'acide acétique. On obtient 22,51 g de produit attendu.

$[\alpha]_D^{20} = +107° \pm 2°$ (c = 0,88%, chloroforme)

Stade C
Chlorure d'acide (1R,trans)2,2-diméthyl 3-/2-méthoxy carbonyl (E) éthényl/cyclopropane carboxylique

On mélange 4 g du produit obtenu au stade B avec 20 $cm^3$ d'isoprène et 3 $cm^3$ de chlorure de thionyle, agite la solution obtenue à 20°C pendant 5 heures, évapore à sec sous pression réduite et obtient un produit attendu brut utilisé tel quel pour la suite de la synthèse.

Exemple 34
(1R,cis)2,2-diméthyl 3-/2-méthoxy carbonyl (Z) éthényl/cyclopropane carboxylate de (1S)2-méthyl 3-allyl 4-méthylène cyclopent-2-èn 1-yle

On dissout 0,9 g de 1S-hydroxy 2-méthyl 3-allyl 4-méthylène cyclopent-2-ène dans 5 $cm^3$ de benzène puis ajoute 0,486 g de pyridine. On ajoute ensuite à 10°C sous agitation 1,08 g de chlorure d'acide (1R,cis)2,2-diméthyl 3-/2-méthoxy carbonyl (Z)éthényl/cyclopropane carboxylique dissous dans 10 $cm^3$ de benzène puis agite pendant 16 heures à 20°C, verse dans l'eau, décante, lave la phase organique à l'eau, réextrait au benzène, sèche les phases organiques réunies puis évapore le solvant. On obtient 2,2 g de produit attendu que l'on chromatographie sur silice en éluant au benzène à 1 pour 1000 de triéthylamine.

On obtient 0,54 g de produit attendu.

$[\alpha]_D^{20} = +45° \pm 1°$ (c = 0,9%, chloroforme)

Analyse ($C_{20}H_{26}O_4$) (330,428):

| | | |
|---|---|---|
| Calculé: | C 72,70, | H 7,93% |
| Trouvé: | C 72,8, | H 7,6% |

Spectre RMN ($CDCl_3$, 60 MHz)

Pic à 3,75 p. p. m. caractéristique des hydrogènes du méthoxy.
Pics de 6,52 à 6,68 p. p. m. et de 6,71 à 6,86 p. p. m. caractéristiques de l'hydrogène en 1 de l'éthényle.
Pics de 5,83 à 6,02 p. p. m. caractéristiques de l'hydrogène en 2 de l'éthényle.
Pics de 1,27 à 1,32 p. p. m. caractéristiques des hydrogènes des méthyles en 2 du cycle cyclopropane.
Pics de 5,5 à 5,83 p. p. m. caractéristiques de l'hydrogène en 1 du cycle cyclopentyle.

Pic à 1,78 p. p. m. caractéristique des hydrogènes du méthyle en 2 du cycle cyclopentyle.
Pics de 2,95 à 3,07 p. p. m. caractéristiques des hydrogènes en 2 de l'allyle.
Pics de 4,75 à 5,17 p. p. m. caractéristiques des hydrogènes du méthylène et des hydrogènes en 3 de l'allyle.

Le chlorure d'acide utilisé au départ de l'exemple 34 à été préparé comme suit:

Stade A

Acide (1R,cis)2,2-diméthyl 3-/2-méthoxy carbonyl (Z) et (E)éthényl/cyclopropane carboxylique

On mélange 11,15 g d'hydrure de sodium (à 60% dans l'huile minérale) dans 300 $cm^3$ de tétrahydrofuranne, puis ajoute 116,8 g de bromure de triphényl acétate de méthyle phosphonium.

On agite la suspension obtenue pendant trois heures à 20°C, refroidit à 0°C, puis ajoute en une heure 20 g de lactone de l'acide cis 2,2-diméthyl 3S(dihydroxyméthyl) cyclopropane-1R-carboxylique en solution dans 100 $cm^3$ de tétrahydrofuranne.

On agite pendant deux heures à 0°C puis pendant 16 heures à 20°C. On verse le milieu réactionnel dans un mélange de 30 $cm^3$ d'acide chlorhydrique concentré et de glace, agite puis ajoute du chlorure de méthylène, décante, lave la phase organique à l'eau puis la sèche et évapore le solvant.

On obtient 82,2 g de produit attendu que l'on chromatographie sur silice en éluant au mélange benzène-acétate d'éthyle (6-4) à 1 pour 1000 d'acide acétique. On obtient ainsi 2 g d'isomère Z attendu et 6,5 g de l'isomère E correspondant.

Isomère Z

$[\alpha]_{20}^{D} = +75,5° \pm 2°$ (c = 1%, chloroforme)

Spectre IR (chloroforme)

Absorptions à 1712 et 1695 $cm^{-1}$ caractéristiques du carbonyle
Absorptions à 1637 $cm^{-1}$ et vers 1625 $cm^{-1}$ caractéristiques de la double liaisaon C=C
Pas d'absorption vers 980 $cm^{-1}$.

Isomère E

$[\alpha]_{20}^{D} = +9,5° \pm 1°$ (c = 1%, chloroforme)

Spectre IR

Absorptions a 1700 et 1710 $cm^{-1}$ caractéristiques du carbonyle
Absorptions à 1647 $cm^{-1}$ et vers 1635 $cm^{-1}$ caractéristiques de la double liaison C=C
Absorption à 980 $cm^{-1}$ caractéristique de la double liaison E.

Stade B

Chlorure d'acide (1R,cis)2,2-diméthyl 3-/2-méthoxy carbonyl (Z)éthényl/cyclopropane carboxylique

On mélange 1,8 g de l'acide (isomère Z) obtenu au stade A avec 10 $cm^3$ d'isoprène et 1 $cm^3$ de chlorure de thionyle, laisse sous agitation à 0°C pendant 30 minutes, puis à 20°C pendant 4 heures, chasse le solvant sous pression réduite et obtient un produit brut que l'on utilise tel quel pour le stade suivant.

Exemple 35

(1R,cis)2,2-diméthyl 3-/2-méthoxy carbonyl (E) éthényl/cyclopropane carboxylate de (1S)2-méthyl 3-allyl 4-méthylène cyclopent-2-èn 1-yle

On mélange 1,36 g de (1S)-hydroxy 2-méthyl 3-allyl 4-méthylène cyclopent-2-ène avec 10 $cm^3$ de benzène et 0,7 $cm^3$ de pyridine.

On refroidit à 0°C puis ajoute en 15 minutes 1,65 g de chlorure de l'acide (1R,cis)2,2-diméthyl 3-/2-méthoxy carbonyl (E)éthényl/cyclopropane carboxylique dissous dans le benzène.

On maintient sous agitation à 20°C pendant 16 heures, verse dans l'eau, décante, lave la phase organique à l'eau, la sèche et évapore le solvant. On obtient 3,2 g de produit attendu brut que l'on chromatographie sur silice en éluant au mélange benzène-acétate d'éthyle (95-5) à 1 pour 1000 de triéthylamine.

On obtient 0,564 g de produit attendu.

$[\alpha]_{20}^{D} = -40° \pm 2{,}5°$ (c = 0,5%, chloroforme)

Analyse ($C_{20}H_{26}O_4$):

| | | |
|---|---|---|
| Calculé: | C 72,70, | H 7,93% |
| Trouvé: | C 72,5, | H 8,2% |

Spectre RMN ($CDCl_3$, 60 MHz)

Pic à 3,73 p. p. m. caractéristique des hydrogènes du méthoxy.
Pics de 5,83 à 6,1 p. p. m. caractéristiques de l'hydrogène en 2 de l'éthényle.
Pics de 7,08 à 7,58 p. p. m. caractéristiques de l'hydrogène en 1 de l'éthényle.
Pics de 1,23 à 1,36 p. p. m. caractéristiques de l'hydrogène des méthyles en 2 du cycle cyclopropane.
Pic à 5,58 p. p. m. caractéristique de l'hydrogène en 1 du cycle cyclopentyle.
Pic à 1,77 p. p. m. caractéristique des hydrogènes du méthyle en 2 du cycle cyclopentyle.
Pics de 4,75 à 5,25 p. p. m. caractéristiques de l'hydrogène du méthylène et des hydrogènes en 3 de l'allyle.

Le chlorure d'acide utilisé au départ de l'exemple 35 a été Préparé comme suit:

On mélange 1,8 g d'acide (1R,cis)2,2-diméthyl 3-/2-méthoxy carbonyl (E)éthényl/cyclopropane carboxylique obtenus à l'exemple 34, avec 10 cm³ d'isopropène, 1 cm³ de chlorure de thionyle. On maintient à 0°C pendant trente minutes, puis à 20°C pendant quatre heures, chasse le solvant sous pression réduite et obtient le produit attendu brut utilisé tel quel pour la suite de la synthèse.

### Exemple 36
### (1R,trans)2,2-diméthyl 3-éthényl cyclopropane carboxylate de (1S)2-méthyl 3-allyl 4-méthylène cyclopent-2-èn 1-yle

On mélange 3 g de (1S)-hydroxy 2-méthyl 3-allyl 4-méthylène cyclopent-2-ène avec 100 cm³ de chlorure de méthylène, 3 g d'acide (1R,trans)2,2-diméthyl 3-éthényl cyclopropane carboxylique et 0,3 g de 4-diméthylamino pyridine.

On refroidit à 0°C et sous agitation, introduit 4,4 g de dicyclohexyl carbodiimide.

On agite pendant 10 minutes à 0°C, laisse revenir à 20°C, et agite pendant trois heures.

On filtre la dicyclohexyl urée, lave le filtrat à l'eau, le sèche et évapore le solvant. On obtient 5,6 g de produit attendu brut que l'on chromatographie sur silice en éluant au benzène à 1 pour 1000 de triéthylamine.

On obtient 4,5 g de produit attendu.

$[\alpha]_{20}^{D} = -72°5 \pm 2°5$

Analyse ($C_{18}H_{24}O_2$) (272,37):

| | | |
|---|---|---|
| Calculé: | C 79,37, | H 8,88% |
| Trouvé: | C 79,4, | H 9,0% |

Spectre IR (chloroforme)

Absorption à 1713 $cm^{-1}$ caractéristique du carbonyle de l'ester
Absorption à 1656 $cm^{-1}$ caractéristique du système conjugué
Absorptions à 1389 et 1379 $cm^{-1}$ caractéristiques du gem diméthyle
Absorptions à 985 et 914 $cm^{-1}$ (vinyle) et à 866 $cm^{-1}$ (double liaison conjugée).

Spectre RMN ($CDCl_3$, 60 MHz)

Pics de 1,12 p. p. m. à 1,23 p. p. m. caractéristiques des hydrogènes des méthyles en 2 du cycle cyclopropane.
Absorptions de 1,52 à 1,62 p. p. m. caractéristiques de l'hydrogène en 1 du cycle cyclopropane.
Absorption à 1,78 p. p. m. caractéristique des hydrogènes du méthyle en 2 du cycle cyclopentyle.
Absorptions de 2,92 à 3,02 p. p. m. caractéristiques des hydrogènes en 1 de la chaîne allyle.
Absorptions de 4,6 à 6,17 p. p. m. caractéristiques des hydrogènes du méthylène et de l'allyle du cycle cyclopentyle ainsi que les hydrogènes en 1 et 2 de la chaîne éthényle.

L'acide (1R,trans)2,2-diméthyl -éthényl cyclopropane carboxylique utilisé au départ de l'exemple 36 a été préparé comme suit:

On mélange 70 g d'acide (1R,trans)2,2-diméthyl 3-formyl carboxylique avec 500 $cm^3$ de diméthyl formamide et 210 g de bromure de triphényl méthyl phosphonium.

On refroidit à −60°C puis ajoute une solution de 115 g de terbutylate de potassium dans 200 $cm^3$ de diméthylformamide.

Après la fin de l'introduction, on laisse remonter la température à +10°C, verse la suspension dans l'eau glacée, acidifie puis extrait au benzène, sèche la phase organique et évapore le solvant. On obtient 62,2 g de produit attendu utilisé tel quel pour le stade suivant.

## Exemple 37
## (1R,cis)2,2-diméthyl 3-éthényl cyclopropane carboxylate de (1S)2-méthyl 3-allyl 4-méthylène cyclopent-2-èn 1-yle

On opère de la manière indiquée à l'exemple 36 au départ de 3 g de (1S)-hydroxy 2-méthyl 3-allyl 4-méthylène cyclopent-2-ène et de 3 g d'acide (1R,cis)2,2-diméthyl 3-éthényl cyclopropane carboxylique et obtient après chromatographie dans le benzène à 1 pour 1000 de triéthylamine, 2,063 g de produit attendu.

$[\alpha]_{20}^{D} = -31,5° \pm 2°$ (c = 0,7%, chloroforme)

Analyse ($C_{18}H_{24}O_2$):

| | | |
|---|---|---|
| Calculé: | C 79,37, | H 8,88% |
| Trouvé: | C 79,2, | H 8,8% |

Spectre IR (chloroforme)

Absorption à 1711 $cm^{-1}$ (carbonyle de l'ester)
Absorption à 1630 $cm^{-1}$ (C=C conjugué)
Absorptions à 1387 et 1376 $cm^{-1}$ (gem diméthyle)
Absorptions à 990–910 $cm^{-1}$ (vinyle déformation).

Spectre RMN ($CDCl_3$, 60 MHz)

Pics de 1,18 à 1,3 p. p. m. attribués aux hydrogènes des méthyles en 2 du cycle cyclopropane.
Pics de 4,75 à 6,5 p. p. m. attribués aux hydrogènes de la chaîne éthényle.
Pic à 5,58 p. p. m. attribué à l'hydrogène en 1 du cycle cyclopentyle.
Pic à 1,75 p. p. m. attribué à l'hydrogène du méthyle en 2 du cycle cyclopentyle.
Pics de 2,93 à 3,03 p. p. m. attribués aux hydrogènes en 1 de la chaîne allyle.
Pics de 4,75 à 6,5 p. p. m. attribués aux hydrogènes en 3 de la chaîne allyle et du méthylène.

L'acide (1R,cis)2,2-diméthyl 3-éthényl cyclopropane carboxylique utilisé au départ de l'exemple 37 a été préparé comme suit:

On mélange à 20°C, 5 g de la lactone de l'acide cis 2,2-diméthyl 3S-(dihydroxyméthyl) cyclopropane-1R-carboxylique avec 15 g de bromure de triphényl méthyl phosphonium et 40 $cm^3$ de diméthyl formamide.

On refroidit à −60°C puis ajoute en une heure une solution de 8,2 g de terbutylate de potassium dans 10 $cm^3$ de diméthylformamide. On laisse ensuite remonter la température vers 0°C en une heure trente environ, verse la solution dans un mélange d'eau et de glace, lave au chlorure de méthylène, acidifie la phase aqueuse à pH 1 par de l'acide chlorhydrique puis extrait au benzène. On lave la phase organique à l'eau, la sèche, et évapore le solvant. On obtient 4,9 g de produit attendu.

Exemple 38
(1R,trans)2,2-diméthyl 3-(2-fluoro 2-chloroéthényl)cyclopropane carboxylate de (1S)2-méthyl 3-allyl 4-méthylène cyclopent-2-èn 1-yle

On opère comme indiqué à l'exemple 36, au départ de 3,9 g d'acide (1R,trans)2,2-diméthyl 3-(2-fluoro 2-chloroéthényl)cyclopropane carboxylique et de 3,04 g de (1S)-hydroxy 2-méthyl 3-allyl 4-méthylène cyclopent-2-ène.

On obtient après chromatographie sur silice en éluant au mélange éther de pétrole (Eb. 40—70°C-éther (97-3) à 1 pour 1000 de triéthylamine 2,08 g de produit attendu.

$[\alpha]_{20}^{D} = -56° \pm 2,5°$ (c = 1%, chloroforme)

Spectre IR (chloroforme)

Absorption à 1713 $cm^{-1}$ (carbonyle de l'ester)
Absorptions à 1670 et 1982 $cm^{-1}$ (système conjugué)
Absorptions à 1390 et 1379 $cm^{-1}$ (gem diméthyle)
Absorptions à 990 et 916 $cm^{-1}$ (vinyle déformation).

Spectre RMN ($CDCl_3$, 60 MHz)

Pics de 4,38 à 4,52 p. p. m. et de 4,75 à 5,25 p. p. m. attribués à l'hydrogène en 1 de la chaîne éthényle.
Pics de 1,48 à 1,57 p. p. m. et de 1,51 à 1,6 p. p. m. attribués à l'hydrogène en 1 du cycle cyclopropane.
Pic a 5,58 p. p. m. attribué à l'hydrogène en 1 du cycle cyclopentyle.
Pic à 1,78 p. p. m. attribué aux hydrogènes du méthyle en 2 du cycle cyclopentyle.
Pics de 5,5 à 6,17 p. p. m. attribués à l'hydrogène en 2 de la chaîne allyle.
Pics de 4,75 à 5,25 p. p. m. attribués aux hydrogènes en 3 de la chaîne allyle et du méthylène.

L'acide utilisé au départ de l'exemple 38 a été décrit par D. BROWN (Structure Activity Studies of halopyrethroids, 1974, page 27 North Texas State University).

Exemple 39
(1R,trans)2,2-diméthyl 3-cyclobutylidène méthyl cyclopropane carboxylate de (1S)2-méthyl 3-allyl 4-méthylène cyclopent-2-èn 1-yle

On mélange 6 g de (1S)-hydroxy 2-méthyl 3-allyl 4-méthylène cyclopent-2-ène avec 6 $cm^3$ de benzène et 4 $cm^3$ de pyridine, on ajoute à 10°C en 30 minutes, 7,5 g du chlorure de l'acide (1R,trans)2,2-diméthyl 3-(cyclobutylidène méthyl) cyclopropane carboxylique.

On agite le mélange pendant 17 heures à 20°C, puis le verse dans l'eau, extrait au chlorure de méthylène, sépare la phase organique, la sèche et évapore à sec.

On obtient 12,8 g de produit brut que l'on purifie par chromatographie sur silice en éluant au mélange cyclohexane-acétate d'éthyle (95-5) à 1 pour 1000 de triéthylamine.

On obtient ainsi 3,63 g de produit attendu.

$[\alpha]_{20}^{D} = -76,5° \pm 2,5°$ (c = 0,7%, chloroforme)

Analyse ($C_{21}H_{28}O_2$):

| | | |
|---|---|---|
| Calculé: | C 80,73, | H 9,03%, |
| Trouvé: | C 80,5, | H 9,0% |

Spectre RMN ($CDCl_3$, 60 MHz)

Pics de 1,38 à 1,47 p. p. m. attribués à l'hydrogène en 1 du cycle cyclopropane.
Pics de 1,13 à 1,25 p. p. m. attribués aux hydrogènes des méthyles en 2 du cycle cyclopropane.
Pic à 1,78 p. p. m. attribué aux hydrogènes du méthyle en 2 du cycle cyclopentyle.
Pics de 5,48 à 6,17 p. p. m. attribués aux hydrogènes en 2 de l'allyle et à l'hydrogène en 1 du cycle cyclopentyle.

Pics de 4,67 à 5,17 p. p. m. attribués aux hydrogènes en 3 de l'allyle, aux hydrogènes du méthylène et à l'hydrogène de la double liaison du butylidène.

Spectre IR (chloroforme)

Absorption à 1709 $cm^{-1}$ (carbonyle de l'ester)
Absorption à 1630 $cm^{-1}$ (double liaison conjuguée)
Absorption à 1377 $cm^{-1}$ (gem diméthyl)
Absorptions à 988 et 915 $cm^{-1}$ ($CH=CH_2$ déformation)

Le chlorure d'acide utilisé au départ de l'exemple 39 a été décrit dans le brevet d'addition français n° 93 112.

### Exemple 40
### (1R,trans)2,2-diméthyl 3-/2- (E) et (Z) cyanoéthényl/cyclopropane carboxylate de (1S)2-méthyl 3-allyl 4-méthylène cyclopent-2-èn 1-yle

On opère comme indique à l'exemple 39 au départ de 9,4 g de (1S)-hydroxy 2-méthyl 3-allyl 4-méthylène cyclopent 2-ène et de 10,9 g du chlorure de l'acide (1R,trans)2,2-diméthyl 3-/2- (E) + (Z) cyano éthényl/cyclopropane carboxylique et obtient après chromatographie sur silice en éluant au mélange cyclohexane-acétate d'éthyle (9-1) à 1 pour 1000 de triéthylamine, 1°) 2,2 g de l'isomère (E) de l'ester 2°) 2 g de l'isomere (Z) de l'ester F ~ 94°C.

Isomère E

$[\alpha]_{20}^{D} = -15° \pm 2°$ (c = 0,7%, benzène)

Analyse ($C_{19}H_{23}NO_2$):

| | | | |
|---|---|---|---|
| Calculé: | C 76,73, | H 7,80, | N 4,71% |
| Trouvé: | C 76,8, | H 7,7, | N 4,5% |

Spectre RMN ($CDCl_3$, 60 MHz)

Pics de 3,18 à 5,57 p. p. m. attribués à l'hydrogène en 2 de l'éthényle.
Pics de 6,15 à 6,32 et de 6,42 à 6,58 p. p. m. attribués à l'hydrogène en 1 de l'éthényle.
Pics de 1,22 à 1,28 p. p. m. attribués aux hydrogènes des méthyles en 2 du cycle cyclopropane.
Pic à 5,58 p. p. m. attribué à l'hydrogène en 1 du cycle cyclopentyle.
Pic à 1,78 p. p. m. attribué aux hydrogènes du méthyle en 2 du cycle cyclopentyle.
Pics de 2,93 à 3,03 p. p. m. attribués aux hydrogènes en 1 de la chaîne allyle.
Pics de 5,5 à 6,16 p. p. m. attribués à l'hydrogène en 2 de la chaîne allyle.
Pics de 4,75 à 5,17 p. p. m. attribués aux hydrogènes en 3 de la chaîne allyle et aux hydrogènes du méthylène.

Isomère Z

$[\alpha]_{20}^{D} = -94° \pm 3°$ (c = 0,5%, benzène)

Analyse ($C_{19}H_{23}NO_2$):

| | | | |
|---|---|---|---|
| Calculé: | C 76,73, | H 7,80, | N 4,71% |
| Trouvé: | C 76,2, | H 7,8, | N 4,5% |

Spectre RMN ($CDCl_3$, 60 MHz)

Pics de 5,23 à 5,42 p. p. m. attribues à l'hydrogène en 2 de l'éthényle.
Pics de 3,92 à 6,08 et de 6,1 à 6,27 p. p. m. attribués à l'hydrogène en 1 de l'éthényle.

Pics à 1,27 et 1,34 p. p. m. attribués à l'hydrogène des méthyles en 2 du cycle cyclopropane.
Pic à 5,58 p. p. m. attribué à l'hydrogène en 1 du cycle cyclopentyle.
Pic à 1,78 p. p. m. attribué à l'hydrogène du méthyle en 2 du cycle cyclopentyle.
Pics de 2,93 à 3,03 p. p. m. attribués aux hydrogènes en 1 de la chaîne allyle.
Pics de 5,42 à 6,25 p. p. m. attribués à l'hydrogène en 1 de la chaîne allyle.
Pics de 4,75 à 5,25 p. p. m. attribués aux hydrogènes en 3 de la chaîne allyle et aux hydrogènes du méthylène.

Le chlorure d'acide utilisé au départ de l'exemple 40 a été préparé comme suit:

### Stade A
### Acide (1R,trans)2,2-diméthyl 3-/2- (E) + (Z) cyano éthényl/cyclopropane carboxylique

On introduit 8,64 g d'hydrure de sodium (en suspension à 50% dans l'huile minérale) dans 150 $cm^3$ de glyme. On introduit à 20°C en trente minutes, une solution de 31,9 g de diéthyl cyanométhyl phosphonate dans 45 $cm^3$ de glyme. On maintient une heure sous agitation à 20°C puis refroidit à −15°C et ajoute une solution de 13 g d'acide (1R,trans)2,2-diméthyl 3-formyl cyclopropane carboxylique dans 70 $cm^3$ de glyme.

On maintient sous agitation à 0°C pendant une heure puis à 20°C pendant trois heures.

On évapore à sec, refroidit le résidu et ajoute 100 $cm^3$ de soude 1 N.

On lave la suspension obtenue au chlorure de méthylène, acidifie la phase aqueuse par environ 11 $cm^3$ d'acide chlorhydrique concentré puis extrait au chlorure de méthylène.

On sèche la phase organique et évapore le solvant. On obtient 14,5 g de produit attendu que l'on chromatographie sur silice en éluant au mélange benzène-acétate d'éthyle (60-40) à 1 pour 100 d'acide acétique.

On obtient 14 g de produit attendu.

### Stade B
### Chlorure de l'acide (1R,trans)2,2-diméthyl 3-/2-(E) + (Z)-cyano éthényl/cyclopropane carboxylique

On mélange 14 g de l'acide obtenu au stade A avec 70 $cm^3$ d'éther de pétrole (Eb. 60—80°C) et 28 $cm^3$ de chlorure de thionyle. On porte au reflux pendant 4 heures puis évapore à sec et obtient 16 g de produit attendu utilisé tel quel pour le stade suivant.

## Exemple 41
### (1R,trans)2,2-diméthyl 3-(2-méthyl 1-propényl) cyclopropane-1-carboxylate de (1R)2-méthyl 3-allyl 4-méthylène cyclopent-2-èn 1-yle

On opère comme indiqué à l'exemple 1 au départ de 2,6 g de chlorure d'acide (1R,trans)2,2-diméthyl 3-(2-méthyl 1-propényl)cyclopropane-1-carboxylique et de 2 g de (1R)-hydroxy 2-méthyl 3-allyl 4-méthylène cyclopent -2-ène et obtient après chromatographie du produit sur silice en éluant au mélange cyclohexane-acétate d'éthyle (95-5) à 1 pour 1000 de triéthylamine, 1,532 g de produit attendu.

Spectre RMN ($CDCl_3$, 60 MHz)

Pics de 1,12 à 1,27 p. p. m. attribués aux hydrogènes des méthyles en 2 du cycle cyclopropane.
Pics à 1,69 et 1,77 p. p. m. attribués aux hydrogènes du méthyle en 2 du cycle cyclopentyle.
Pics de 1,33 à 1,42 p. p. m. attribués aux hydrogènes en 1 du cycle cyclopropane.
Pics à 1,93 et 3,02 p. p. m. attribués à l'hydrogène en 3 du cycle cyclopropyle.
Pics à 4,75 et 5,17 p. p. m. attribués aux hydrogènes du méthylène du cycle cyclopentyle ainsi qu'à l'hydrogène en 1 de la chaîne propényle.
Pics à 5,5 et 6,03 p. p. m. attribués à l'hydrogène en 2 de la chaîne allyle.

## Exemple 42
### (1R,trans)2,2-diméthyl 3-(2-méthyl 1-propényl)cyclopropane-1-carboxylate de (1S)2-méthyl 3-allyl 4-cyano méthylène cyclopent-2-èn 1-yle

On mélange 3 g de (1S)-hydroxy 2-méthyl 3-allyl 4-cyano méthylène cyclopent-2-ène et 3,3 g de chlorure de l'acide (1R,trans)2,2-diméthyl 3-(2-méthyl 1-propényl) cyclopropane carboxylique avec

30 $cm^3$ de benzène. On ajoute à +5°C 2 $cm^3$ de pyridine, laisse sous agitation à 5°C pendant 15 minutes, puis à 20°C pendant deux heures, verse le mélange dans 100 $cm^3$ d'eau à +5°C renfermant 5 $cm^3$ d'acide chlorhydrique 1 N, extrait à l'éther, lave la phase organique à l'eau, la sèche, évapore le solvant. On obtient 6 g de produit attendu que l'on chromatographie sur silice en éluant au mélange chlorure de méthylène-cyclohexane (8-2). On obtient 3,5 g de produit attendu. F < à 50°C.

$[\alpha]_D = -152° \pm 3,5°$ (c = 1%, benzène)

Analyse ($C_{21}H_{27}NO_2$):

| | | | |
|---|---|---|---|
| Calculé: | C 77,5, | H 8,36, | N 4,30% |
| Trouvé: | C 77,6, | H 8,4, | N 4,0% |

Spectre IR

Absorption à 1715 $cm^{-1}$ (carbonyle)
Absorption à 2210 $cm^{-1}$ (C=N conjugué)
Absorptions à 1637 et 1614 $cm^{-1}$ (C=C conjugué).

Spectre RMN ($CDCl_3$, 60 MHz)

Pics à 1,7—1,71 p. p. m. attribués aux hydrogènes du méthyle en 2 du propényle et aux hydrogènes en 3 du propényle.
Pics à 1,14 et 1,28 p. p. m. attribués aux hydrogènes des méthyles en 2 du cycle cyclopropane.
Pic à 5,67 p. p. m. attribué à l'hydrogène en 1 du cycle cyclopentyle.
Pic à 1,9 p. p. m. attribué aux hydrogènes du méthyle en 2 du cycle cyclopentyle.

Le (1S)-hydroxy 2-méthyl 3-allyl 4-cyano méthylène cyclopent-2-ène utilisé au départ de l'exemple 42 a été préparé comme suit:

On introduit 4,8 g d'hydrure de sodium à 50% dans l'huile minérale, dans 200 $cm^3$ de monoglyme. On ajoute lentement 19,4 $cm^3$ de cyanométhyl phosphonate de O,O-diéthyle. On maintient sous agitation pendant 30 minutes, refroidit à +5°C, puis ajoute en 20 minutes 15,22 g de 1S-hydroxy 2-méthyl 3-allyl 4-oxo cyclopent-2-ène dans 20 $cm^3$ de monoglyme. On maintient ensuite sous agitation à 5°C pendant 15 minutes, puis à 20°C pendant 20 heures. On concentre à sec sous pression réduite, reprend le résidu par un mélange à 5°C, de 100 $cm^3$ d'acide chlorhydrique 1 N et 200 $cm^3$ d'eau, extrait à l'éther, lave à l'eau, sèche et évapore le solvant. On obtient 18 g de produit brut que l'on chromatographie sur silice dans le mélange chlorure de méthylène-acétate d'éthyle (95/5), puis dans le mélange toluène-acétate d'éthyle (6/4). On obtient 8,76 g de produit attendu.

$[\alpha]_D^{20} = -140° \pm 3°$ (c = 0,42%, benzène)

Analyse ($C_{11}H_{13}NO$) (175,23):

| | | | |
|---|---|---|---|
| Calculé: | C 75,39, | H 7,47, | N 7,99% |
| Trouvé: | C 75,3, | H 7,5, | N 7,8% |

Spectre IR (chloroforme)

- Absorption à 3600 $cm^{-1}$ caractéristique de OH
- Absorption à 2205 $cm^{-1}$ caractéristique CN conjugué
- Absorption 1636 et 1611 $cm^{-1}$ caractéristique C=C conjuguée
- Absorption 990 et 919 $cm^{-1}$ caractéristique $CH=CH_2$ (déformation).

Spectre RMN ($CDCl_3$, 60 MHz)

- Pics de 2,33 à 3,5 p. p. m. caractéristiques des hydrogènes en 5.
- Pic à 4,72 p. p. m. caractéristique de l'hydrogène en 1.
- Pic à 1,93 p. p. m. caractéristique des hydrogènes du méthyle en 2.
- Pics de 2,33 à 3,5 p. p. m. caractéristiques des hydrogènes en 1 de la chaîne allyle.
- Pics de 5,42 à 6,08 p. p. m. caractéristiques de l'hydrogène en 2 de la chaîne allyle.

– Pics de 4,75 à 5,33 p. p. m. caractéristiques des hydrogènes en 3 de la chaîne allyle et de l'hydrogène du cyanométhylène.

Exemple 43
(1R,cis)2,2-diméthyl 3-(2,2-dibromoéthényl)cyclopropane carboxylate de (1S)2-méthyl 3-allyl 4-cyanométhylène cyclopent-2-èn 1-yle

On opère comme indiqué à l'exemple 42, au départ de 2 g de (1S)-hydroxy 2-méthyl 3-allyl 4-cyano méthylène cyclopent-2-ène et de 3,7 g de chlorure de l'acide (1R,cis)2,2-diméthyl 3-(2,2-dibromoéthényl) cyclopropane carboxylique et obtient après purification par chromatographie sur silice en éluant au mélange chlorure de méthylène-cyclohexane (8-2), 3,9 g de produit attendu.

$[\alpha]_{20}^{D} = -23° \pm 2,5°$ (c = 0,5%, benzène)

Analyse ($C_{19}H_{21}Br_2NO_2$):

| | | | |
|---|---|---|---|
| Calculé: | C 50,13, | H 4,65, | N 3,07% |
| Trouvé: | C 50,2, | H 4,6, | N 2,8% |

Spectre IR (chloroforme)

Absorption à 1720 $cm^{-1}$ (carbonyle)
Absorption à 2210 $cm^{-1}$ (C≡N conjugué)
Absorptions à 1637 et 1615 $cm^{-1}$ (C=C conjugué).

Spectre RMN ($CDCl_3$, 60 MHz)

Pics de 6,75 à 6,88 p. p. m. attribués à l'hydrogène en 1 de l'éthényle.
Pics à 1,27 et 1,3 p. p. m. attribués aux hydrogènes des méthyles en 2 du cycle cyclopropane.
Pics de 5,67 à 5,75 p. p. m. attribués à l'hydrogène en 1 du cycle cyclopentyle.
Pic à 1,9 p. p. m. attribué aux hydrogènes du méthyle en 2 du cycle cyclopentyle.
Pics de 2,95 à 3,05 p. p. m. attribués aux hydrogènes en 1 de la chaîne allyle.
Pics de 5,5 à 6,17 p. p. m. attribués à l'hydrogène en 2 de la chaîne allyle.
Pics de 4,83 à 5,17 p. p. m. attribués aux hydrogènes en 3 de la chaîne allyle et à l'hydrogène du méthylène.

Exemple 44
(1R,trans)2,2-diméthyl 3-(2-méthyl 1-propényl)cyclopropane carboxylate de (1S)2-méthyl 3-allyl 4-(E) benzylidène cyclopent-2-èn 1-yle

On opère comme indiqué à l'exemple 42, au départ de 3,4 g de (1S)-hydroxy 2-méthyl 3-allyl 4-(E) benzylidène cyclopent-2-ène et de 2,8 g de chlorure de l'acide (1R,trans)2,2-diméthyl 3-(2-méthyl 1-propényl)cyclopropane carboxylique et obtient après chromatographie sur silice en éluant au mélange cyclohexane-acétate d'éthyle (98-2) à 1‰ de triéthylamine, 1,92 g de produit attendu.

$[\alpha]_{20}^{D} = -171° \pm 3,5°$ (c = 0,5%, chloroforme)

Analyse ($C_{26}H_{32}O_2$):

| | | |
|---|---|---|
| Calculé: | C 82,9, | H 8,56% |
| Trouvé: | C 83,0, | H 8,7% |

Spectre IR (chloroforme)

Absorption à 1709 $cm^{-1}$ (carbonyle de l'ester)
Absorptions à 1634–1625 $cm^{-1}$ (C=C conjugué)
Absorptions à 1594–1570–1487 $cm^{-1}$ (bandes aromatiques)
Absorptions à 985–913 $cm^{-1}$ (allyle).

Spectre RMN ($CDCl_3$, 60 MHz)

Pics de 1,7 à 1,72 p. p. m. attribués aux hydrogènes du méthyle en 2 du propényle et aux hydrogènes en 3 du propényle.
Pics de 4,75 à 5,25 p. p. m. attribués à l'hydrogène en 1 du propényle.
Pics à 1,12 et à 1,29 p. p. m. attribués aux hydrogènes des méthyles en 2 du cycle cyclopropane.
Pics de 1,37 à 1,45 p. p. m. attribués à l'hydrogène en 1 du cycle cyclopropane.
Pic à 5,75 p. p. m. attribué à l'hydrogène en 1 du cycle cyclopentyle.
Pic à 1,83 p. p. m. attribué aux hydrogènes du méthyle en 2 du cycle cyclopentyle.
Pics à 3,05 et 3,15 p. p. m. attribués aux hydrogènes en 1 de la chaîne allyle.
Pics de 4,75 à 5,25 p. p. m. attribués aux hydrogènes en 3 de la chaîne allyle.
Pic à 6,32 p. p. m. attribué à l'hydrogène porté par le carbone du benzylidène.
Pic à environ 7,30 p. p. m. attribué aux hydrogènes du cycle phényle.

Le 1S-hydroxy 2-méthyl 3-allyl 4-(E)-benzylidène cyclopent-2-ène utilisé au départ de l'exemple 44 a été préparé comme suit:

On mélange 22 g de ter-butylate de potassium à 250 $cm^3$ de tétrahydrofuranne, puis ajoute 90 $cm^3$ de ter-butanol. On ajoute ensuite lentement 76,4 g de chlorure de triphényl benzyl phosphonium, agite pendant 1 heure à 23°C, puis ajoute lentement une solution de 29,8 g de (1S)-hydroxy 2-méthyl 3-allyl 4-oxo cyclopent-2-ène dans 10 $cm^3$ de tétrahydrofuranne. On porte au reflux pendant 24 heures, refroidit à 20°C, verse dans une solution aqueuse glacée de phosphate monosodique, extrait à l'éther, lave la phase éthérée à l'eau, sèche et évapore le solvant. On obtient 81,5 g de produit brut que l'on chromatographie sur silice en éluant au mélange cyclohexane-acétate d'éthyle (1/1). On obtient 25,5 g de produit attendu. F. $< 50$°C.

Spectre IR (chloroforme)

- Absorption à 3600 $cm^{-1}$ caractéristique de OH
- Absorption à 1635—1625 $cm^{-1}$ caractéristique du système conjugué
- Absorption à 1597—1488 $cm^{-1}$ caractéristique des bandes aromatiques
- Absorption à 999—914 $cm^{-1}$ caractéristique de l'allyle.

Spectre RMN ($CDCl_3$, 60 MHz)

- Pic à 4,66 p. p. m. caractéristique de l'hydrogène en 1.
- Pic à 1,89 p. p. m. caractéristique des hydrogènes du méthyle en 2.
- Pics à 3,03 et 3,12 p. p. m. caractéristiques des hydrogènes en 1 de la chaîne allyle.
- Pics de 4,83 à 5,25 p. p. m. caractéristiques des hydrogènes en 3 de la chaîne allyle.
- Pics de 5,55 à 6,16 p. p. m. caractéristiques des hydrogènes en 2 de la chaîne allyle.
- Pic à 6,28 p. p. m. caractéristique de l'hydrogène du benzylidène.
- Pic à environ 7,32 p. p. m. caractéristique des hydrogènes aromatiques.

## Exemple 45

### (1R,trans)2,2-diméthyl 3-(2-méthyl 1-propényl)cyclopropane carboxylate de (1S)2-méthyl 3-allyl 4-(cyano éthoxy carbonyl)méthylène cyclopent-2-èn 1-yle

On opère comme indiqué à l'exemple 42 au départ de 2,5 g de (1S)-hydroxy 2-méthyl 3-allyl 4-(cyano éthoxy carbonyl)méthylène cyclopent-2-ène et de 1,9 g de chlorure d'acide (1R,trans)2,2-diméthyl 3-(2-méthyl 1-propényl)cyclopropane carboxylique et obtient après chromatographie sur silice en éluant au mélange toluène-acétate d'éthyle (97-3), 2,37 g de produit attendu.

$[\alpha]_{20}^{D} = -93° \pm 2°$ (c = 1%, chloroforme)

Analyse ($C_{24}H_{31}O_4N$):

| | | | |
|---|---|---|---|
| Calculé: | C 72,52, | H 7,86, | N 3,52% |
| Trouvé: | C 72,8, | H 7,7, | N 3,5% |

Spectre IR (chloroforme)

Absorption à 2218 $cm^{-1}$ (C=N conjugué)
Absorption à 1719 $cm^{-1}$ (carbonyle de l'ester)
Absorptions à 1637—1618—1570 $cm^{-1}$ (système conjugué)
Absorptions à 991—998 $cm^{-1}$ ($CH=CH_2$ déformation).

Spectre RMN ($CDCl_3$, 60 MHz)

Pic à 1,7 p. p. m. attribué aux hydrogènes du méthyle en 2 de la chaîne propényle et aux hydrogènes en 3 de la chaîne propényle.
Pics de 4,75 à 5,25 p. p. m. attribués à l'hydrogène en 1 de la chaîne propényle.
Pics de 1,13 à 1,27 p. p. m. attribués aux hydrogènes des méthyles en 2 du cycle cyclopropane.
Pics de 5,67 à 5,75 p. p. m. attribués à l'hydrogène en 1 du cycle cyclopentyle.
Pic à 1,98 p. p. m. attribué aux hydrogènes du méthyle en 2 du cycle cyclopentyle.
Pics de 3,43 à 3,55 p. p. m. attribués aux hydrogènes en 1 de la chaîne allyle.
Pics de 4,75 à 5,25 p. p. m. attribués aux hydrogènes en 3 de la chaîne allyle.
Pics à 1,22—1,33—1,45—4,1—4,22—4,33— et 4,45 p. p. m. attribués aux hydrogènes de l-éthyle.

Le (1S)-hydroxy 2-méthyl 3-allyl 4-(cyano éthoxy carbonyl) méthylène cyclopent-2-ène utilisé au départ de l'exemple 45 a été préparé comme suit:

On mélange 3,1 g de (1S)-hydroxy 2-méthyl 3-allyl 4-oxo méthylène cyclopent-2-ène, 4,52 g de cyanoacétate d'éthyle, 0,7 g d'acétate d'ammonium, 1 $cm^3$ d'acide acétique et 50 $cm^3$ de toluène. On porte au reflux pendant 6 heures, puis élimine l'eau par distillation et refroidit à 20°C. On ajoute 0,6 g d'acétate d'ammonium dans 1 $cm^3$ d'acide acétique, porte à nouveau au reflux pendant 6 heures, puis élimine à nouveau l'eau formée par distillation. On refroidit à 20°C, dilue à l'éther, décante, lave la phase éthérée à l'eau, sèche, évapore à sec et obtient 4,15 g de produit brut. On chromatographie ce produit brut sur silice, en éluant au mélange benzène-acétate d'éthyle (6/4) et obtient 1,06 g de produit attendu.

$[\alpha]_D = -87° \pm 2°$ (c = 0,9%, chloroforme)

Spectre IR (chloroforme)

— Absorption à 3600 $cm^{-1}$ caractéristique de OH
— Absorption à 2220 $cm^{-1}$ caractéristique de C≡N
— Absorption à 1718 $cm^{-1}$ caractéristique de C=O ester
— Absorptions à 1637, 1616 et 1507 $cm^{-1}$ caractéristiques de C=C conjuguée
— Absorptions à 990—918 $cm^{-1}$ caractéristiques de $CH=CH_2$ (Déformation).

Spectre RMN ($CDCl_3$, 60 MHz)

— Pics à 1,22, 1,33, 1,45, 4,11, 4,23, 4,35 et 4,47 p. p. m. caractéristiques du —COO Et.
— Pic à 2,06 p. p. m. caractéristique des hydrogènes du méthyle en 2.
— Pic à 2,58 p. p. m. caractéristique de l'hydrogène du OH.
— Pics de 2,75 à 4 p. p. m. caractéristiques des hydrogènes en 5.
— Pics de 3,42 à 3,53 p. p. m. caractéristiques des hydrogènes en 1 de la chaîne allyle.
— Pic à 4,75 p. p. m. caractéristique de l'hydrogène en 1.
— Pics de 4,66 à 5,25 p. p. m. caractéristiques des hydrogènes en 3 de la chaîne allyle.
— Pics de 5,67 à 6,25 p. p. m. caractéristiques de l'hydrogène en 2 de la chaîne allyle.

## Exemple 46
### (1R,trans)2,2-diméthyl 3-(2-méthyl 1-propényl)cyclopropane carboxylate de (1S)2-méthyl 3-allyl 4-fluoro chlorométhylène cyclopent-2-èn 1-yle

On mélange à 20°C sous agitation 70 $cm^3$ de N-heptane, 3,4 g de terbutylate de potassium et 2,85 g d'alcool terbutylique.

On porte le mélange pendant quinze minutes à 50°C puis refroidit à 20°C et ajoute 7,86 g de triphényl phosphine dans 70 $cm^3$ de N-heptane.

On refroidit à 0°C puis ajoute en trente minutes, une solution de 3,9 g de difluorodichlorométhane dans 40 $cm^3$ de N-heptane puis ajoute à 0°C, en vingt minutes, 6,28 g de (1R,trans)2,2-diméthyl

3-(2-méthyl 1-propényl)cyclopropane carboxylate de (1S)-hydroxy 2-méthyl 3-allyl 4-oxocyclopent-2-èn 1-yle dans 10 $cm^3$ de tétrahydrofuranne.

On maintient sous agitation à 20°C pendant vingt-quatre heures puis à 60°C pendant une heure. On refroidit à 20°C, concentre sous pression réduite, empâte le résidu dans 200 $cm^3$ d'éther de pétrole (Eb. 40—70°C) filtre et rince à l'éther de pétrole. On recueille le filtrat, le lave par une solution aqueuse saturée de phosphate monosodique puis à l'eau, sèche et concentre à sec. On obtient 15 g de produit brut que l'on chromatographie une première fois en éluant au mélange cyclohexane-acétate d'éthyle (95-5) à 1 pour 1000 de triéthylamine puis une seconde fois en éluant au mélange toluène-cyclohexane (5-5), on obtient finalement 0,8 g de produit attendu.

$[\alpha]_D = -98° \pm 3°$ (c = 0,5%, benzène)

Analyse ($C_{20}H_{26}ClFO_2$):

| | | | | |
|---|---|---|---|---|
| Calculé: | C 68,07, | H 7,42, | Cl 10,04, | F 5,38% |
| Trouvé: | C 68,1, | H 7,3, | Cl 10,2, | F 5,3% |

Spectre RMN ($CDCl_3$, 60 MHz)

Pics à 1,7 et 1,72 p. p. m. attribués aux hydrogènes du méthyle en 2 du propényle et des hydrogènes en 3 du propényle.
Pics à 4,85—4,97 p. p. m. attribués à l'hydrogène en 1 du propényle.
Pics de 1,13 à 1,27 p. p. m. attribués aux hydrogènes des méthyles en 2 du cycle cyclopropane.
Pics à 1,34—1,43 p. p. m. attribués à l'hydrogène en 1 du cycle cyclopropane.
Pics à de 5,53 à 5,65 p. p. m. attribués à l'hydrogène en 1 des cycles cyclopentyles.
Pic à 1,7 p. p. m. attribué aux hydrogènes du méthyle en 2 du cycle cyclopentyle.
Pics de 5,5 à 6,18 p. p. m. attribués à l'hydrogène en 2 de la chaîne allyle et,
Pics de 4,83 à 5,17 p. p. m. attribués aux hydrogènes en 3 de la chaîne allyle.

### Exemple 47

### (1R,cis)2,2-diméthyl 3-(2-méthyl 1-propényl)cyclopropane carboxylate de (1S)2-méthyl 3-allyl 4-dichlorométhylène cyclopent-2-èn 1-yle

On opère comme indiqué à l'exemple 42 au départ de 2,3 g de (1S)-hydroxy 2-méthyl 3-allyl 4-dichlorométhylène cyclopent-2-ène et de 2 g de chlorure de l'acide (1R,cis)2,2-diméthyl 3-(2-méthyl 1-propényl)cyclopropane carboxylique et obtient après chromatographie sur silice en éluant au mélange benzène-cyclohexane (4-6) à 1 pour 1000 de triéthylamine, 1,5 g de produit attendu.

$[\alpha]_D = -16,5° \pm 1,5°$ (c = 0,7%, benzène)

Analyse ($C_{20}H_{26}Cl_2O_2$):

| | | | |
|---|---|---|---|
| Calculé: | C 65,04, | H 7,10, | Cl 19,20% |
| Trouvé: | C 64,9, | H 7,0, | Cl 19,3% |

Spectre IR

Absorption à 1719 $cm^{-1}$ (carbonyle-ester)
Absorptions à 1634 et 1600 $cm^{-1}$ (C=C conjuguée)
Absorption à 1384 $cm^{-1}$ (gem diméthyle)
Absorptions à 995—917 $cm^{-1}$ ($CH=CH_2$).

Spectre RMN ($CDCl_3$, 60 MHz)

Pics à 1,7 et 1,77 p. p. m. aux hydrogènes du méthyle en 2 du propényle et aux hydrogènes en 3 du propényle.
Pics à 5,32—5,45 p. p. m. attribués à l'hydrogène en 1 du propényle.
Pics à 1,2—1,26 p. p. m. attribués aux hydrogènes des méthyles en 2 du cycle cyclopentyle.
Pics à 5,45—5,58 p. p. m. attribués à l'hydrogène en 1 du cycle cyclopentyle.

Pics à 1,7—1,77 p. p. m. attribué aux hydrogènes du méthyle en 2 du cycle cyclopentyle.
Pics de 5,5 à 6,33 p. p. m. attribués à l'hydrogène en 2 de la chaîne allyle et,
Pics de 4,75 à 5,17 p. p. m. attribués aux hydrogènes en 3 de la chaîne allyle.

Exemple 48
(1R,cis)2,2-diméthyl 3-(2-oxo 2,3,4,5-tétrahydro-3-thiophénylidène méthyl)cyclopropane carboxylate de (1S)2-méthyl 3-allyl 4-dichlorométhylène cyclopent-2-èn 1-yle

On opère comme indiqué à l'exemple 42 au départ de 2,3 g de (1S)-hydroxy 2-méthyl 3-allyl 4-dichlorométhylène cyclopent-2-ène et de 2,6 g du chlorure de l'acide (1R,cis)2,2-diméthyl 3-(2-oxo 2,3,4,5-tétrahydro-3-thiophénylidène méthyl) cyclopropane carboxylique et obtient après chromatographie sur silice en éluant au mélange éther de pétrole-acétate d'éthyle (25-75) à 1 pour 1000 de triéthylamine, 3,2 g de produit attendu.

$[\alpha]^D_{20} = +9° \pm 1°$ (c = 1%, benzène)

Analyse ($C_{21}H_{24}Cl_2O_3S$):

| | | | | |
|---|---|---|---|---|
| Calculé: | C 59,01, | H 5,66, | Cl 16,60, | F 7,50%, |
| Trouvé: | C 58,7, | H 5,8, | Cl 16,4, | F 7,3%. |

Spectre IR (chloroforme)

Absorption à 1703 $cm^{-1}$ (C=O ester)
Absorption à 1678 $cm^{-1}$ (C=O thiolactone)
Absorption à 1630 $cm^{-1}$ (C=C).

Spectre RMN ($CDCl_3$, 60 MHz)

Pics de 2.83 à 3,5 p. p. m. attribués aux hydrogènes du cycle thiophénylidène.
Pics à 6,75—6,92 p. p. m. attribués à l'hydrogène du thiophénylidène méthyle.
Pics à 1,27 et 1,35 p. p. m. attribués aux hydrogènes des méthyles en 2 du cycle cyclopropane.
Pics à 5,48—5,93 p. p. m. attribués à l'hydrogène en 1 du cycle cyclopentyle.
Pic à 1,77 p. p. m. attribué aux hydrogènes du méthyle en 2 du cycle cyclopentyle.
Pics de 2,83 à 3,5 p. p. m. attribués aux hydrogènes en 1 de la chaîne allyle.
Pics de 4,75 à 5,17 p. p. m. attribués aux hydrogènes en 3 de la chaîne allyle.
Pics de 5,5 à 6,25 p. p. m. attribués à l'hydrogène en 1 de la chaîne allyle.

Le chlorure d'acide utilisé au départ de l'exemple 48 a été décrit dans le brevet français 2.097.244.

Exemple 49
(1R,trans)2,2-diméthyl 3-(2-fluoro 2-chloro) (E) + (Z) éthényl/cyclopropane carboxylate de (1S)2-méthyl 3-allyl 4-dichlorométhylène cyclopent-2-èn 1-yle

On opère comme indiqué à l'exemple 42 au départ de 2,5 g de (1S)-hydroxy 2-méthyl 3-allyl 4-dichlorométhylène cyclopent-2-ène et de 2,4 g de chlorure de l'acide (1R,trans)2,2-diméthyl 3-/2-fluoro 2-chloro (E) + (Z) éthényl/cyclopropane carboxylique, on obtient après chromatographie sur silice en éluant au mélange benzène-acétate d'éthyle (9-1) à 1 pour 1000 de triéthylamine, 1,92 g de produit attendu.

$[\alpha]^D_{20} = -64° \pm 2,5°$ (c = 0,6%, chloroforme)

Analyse ($C_{18}H_{20}Cl_3FO_2$):

| | | | | |
|---|---|---|---|---|
| Calculé: | C 54,91, | H 5,12, | Cl 27,01, | F 4,82%, |
| Trouvé: | C 55,1, | H 5,0, | Cl 27,0, | F 4,9%. |

Spectre IR (chloroforme)

Absorption à 1716 $cm^{-1}$ (C=O ester)
Absorption à 1670 $cm^{-1}$ (C=C)
Absorptions à 1632—1600 $cm^{-1}$ (système conjugué)
Absorption à 1380 $cm^{-1}$ (gem diméthyle)
Absorptions à 900 et 990 $cm^{-1}$ ($CH=CH_2$ déformation).

Spectre RMN ($CDCl_3$, 60 MHz)

Pics à 4,35—4,48 p. p. m. et 4,8—4,93 p. p. m. et de 4,95 à 5,25 p. p. m. attribués à l'hydrogène en 1 de l'éthényle.
Pics à 1,15—1,27 p. p. m. attribués aux hydrogènes des méthyles en 2 du cycle cyclopropane.
Pic à 5,58 p. p. m. attribué à l'hydrogène en 1 du cycle cyclopentyle.
Pic à 1,77 p. p. m. attribué aux hydrogènes du méthyle en 2 du cycle cyclopentyle.
Pics de 5,42 à 6,25 p. p. m. attribués à l'hydrogène en 2 de la chaîne allyle.
Pics de 4,75 à 5,16 p. p. m. attribués aux hydrogènes en 3 da la chaîne allyle.

### Exemple 50
### (1R,trans)2,2-diméthyl 3-/2-(E + Z)cyano éthényl/cyclopropane carboxylate de (1S)2-méthyl 3-allyl 4-dichlorméthylène cyclopent-2-èn 1-yle

On opère comme indiqué à l'exemple 42 au départ de 2,5 g de (1S)-hydroxy 2-méthyl 3-allyl 4-dichlorométhylène cyclopent-2-ène et de 2,1 g de chlorure de l'acide (1R,trans)2,2-diméthyl 3-/2-(E + Z) cyano éthényl/cyclopropane carboxylique et obtient après chromatographie sur silice en éluant au mélange benzène-acétate d'éthyle (9-1) à 1 pour 1000 de triéthylamine 0,981 g de produit attendu.

$[\alpha]_D^{20} = -50° \pm 1,5°$ (c = 1%, chloroforme)

Spectre IR (chloroforme)

Absorption à 2218 $cm^{-1}$ (C≡N)
Absorption à 1720 $cm^{-1}$ (C=O ester)
Absorptions à 1628—1618—1602 $cm^{-1}$ (C=C conjugué)
Absorptions à 1390—1380 $cm^{-1}$ (gem diméthyle)
Absorptions à 990—900 $cm^{-1}$ (allyle)
Absorptions à 968 $cm^{-1}$ (CH=CH trans).

Spectre RMN ($CDCl_3$, 60 MHz)

Pics de 4,83 à 6,67 p. p. m. attribués aux hydrogènes en 1 et en 2 de l'éthényle.
Pics à 1,25—1,32—1,37 p. p. m. attribués aux hydrogènes des méthyles en 2 du cycle cyclopropane.
Absorption à 5,58 p. p. m. attribuée à l'hydrogène en 1 du cycle cyclopentyle.
Absorption à 1,8 p. p. m. attribuée aux hydrogènes du méthyle en 2 du cycle cyclopentyle.
Absorptions de 5,5 à 6,33 p. p. m. attribuées à l'hydrogène en 1 de la chaîne allyle.
Absorptions de 4,83 à 5,17 p. p. m. attribuées aux hydrogènes en 3 de la chaîne allyle.

### Exemple 51
### (1R,cis)2,2-diméthyl 3-/2-(E + Z)cyano éthényl/cyclopropane carboxylate de (1S)2-méthyl 3-allyl 4-dichlorométhylène cyclopent-2-èn 1-yle

On opère comme indiqué à l'exemple 42, au départ de 2,5 g de (1S)-hydroxy 2-méthyl 3-allyl 4-dichlorométhylène cyclopent-2-ène et de 2,1 g de chlorure de l'acide (1R,cis)2,2-diméthyl 3-/2-(E + Z)cyano éthényl/cyclopropane carboxylique et obtient après chromatographie sur silice en éluant au mélange benzène-acétate d'éthyle (9-1) à 1 pour 1000 de triéthylamine, 1,692 g de produit attendu.

$[\alpha]_D^{20} = -6° \pm 2°$ (c = 0,5%, chloroforme)

Spectre IR (chloroforme)

Absorption à 2210 $cm^{-1}$ (C≡N conjugué)
Absorption à 1718 $cm^{-1}$ (C=O ester)
Absorptions à 1340–1621–1600 $cm^{-1}$ (C=C)
Absorptions à 1390–1380 $cm^{-1}$ (gem diméthyle)
Absorptions à 990–900 $cm^{-1}$ (allyle)
Absorption à 970 $cm^{-1}$ (CH=CH trans).

Spectre RMN ($CDCl_3$, 60 MHz)

Pics de 5,16 à 5,66 p. p. m. attribués à l'hydrogène en 2 de l'éthényle.
Pics de 5,72 à 7,22 p. p. m. attribués à l'hydrogène en 1 de l'éthényle.
Pic à 1,24–1,3–1,32 p. p. m. attribués aux hydrogènes des méthyles en 2 du cycle cyclopropane.
Pic à 5,58 p. p. m. attribué à l'hydrogène en 1 du cycle cyclopentyle.
Pic à 1,76 p. p. m. attribué aux hydrogènes du méthyle du cycle cyclopentyle.
Pics de 5,5 à 6,17 p. p. m. attribués à l'hydrogène en 2 de la chaîne allyle.
Pics de 4,75 à 5,17 p. p. m. attribués aux hydrogènes en e de la chaîne allyle.

### Exemple 52
### (1R,trans)2,2-diméthyl 3-/2-méthoxy carbonyl(E)éthényl/cyclopropane carboxylate de (1S)2-méthyl 3-allyl 4-dichlorométhylène cyclopent-2-èn 1-yle

On agite pendant une heure à 20°C un mélange de 0,5 g d'acide (1R,trans)2,2-diméthyl 3-/2-méthoxy carbonyl (E) éthényl/cyclopropane carboxylique préparé dans l'exemple 33, 10 $cm^3$ de chlorure de méthylène, 20 mg de 4-diméthylamino pyridine et 0,52 g de dicyclohexylcarbodiimide. On ajoute ensuite 0,54 g de (1S)-hydroxy 2-méthyl 3-allyl 4-dichlorométhylène cyclopent-2-ène, agite pendant deux heures à 20°C, filtre, lave le filtrat à l'eau, décante, sèche la phase organique et évapore le solvant. On obtient 1,7 g de produit brut que l'on chromatographie sur silice en éluant au mélange benzène-acétate d'éthyle (9-1) à 1 pour 1000 de triéthylamine et obtient 0,8 g de produit attendu.

$[\alpha]_{20}^{D} = -27,5° \pm 2°$ (c = 0,4%, chloroforme)

Spectre IR (chloroforme)

Absorption à 1715 $cm^{-1}$ (C=O ester)
Absorption à 1649–1635–1602 $cm^{-1}$ (C=C conjugué)
Absorptions à 1391–1380 $cm^{-1}$ (gem diméthyle)
Absorption à 916 $cm^{-1}$ ($CH=CH_2$ déformation)
Absorption à 980 $cm^{-1}$ (CH=CH trans).

Spectre RMN ($CDCl_3$, 60 MHz)

Pic à 3,69 p. p. m. attribué aux hydrogènes du méthoxy.
Pics à 6,05–5,8 p. p. m. attribués à l'hydrogène en 2 de l'éthényle.
Pics à 6,43–6,6 p. p. m. et 6,68–6,85 p. p. m. attribués à l'hydrogène en 1 de l'éthényle.
Pics à 1,23 et à1,29 p. p. m. attribués aux hydrogènes des méthyles en 2 du cycle cyclopropane.
Pic à 5,58 p. p. m. attribué à l'hydrogène en 1 du cycle cyclopentyle.
Pic à 1,77 p. p. m. attribué aux hydrogènes du méthyle en 2 du cycle cyclopentyle.
Pics de 4,75 à 5,17 p. p. m. attribués aux hydrogènes en 3 de la chaîne allyle.

### Exemple 53
### (1R,trans)2,2-diméthyl 3-(2-méthyl 1-propényl) cyclopropane carboxylate de (1S)2-méthyl 3-allyl 4-dichlorométhylène cyclopent-2-èn 1-yle

On mélange à 20°C 170 $cm^3$ de N-heptane et 9,8 g de terbutylate de potassium puis ajoute 8,2 $cm^3$ d'alcool terbutylique.

On ajoute à la suspension abtenue, 23 g de triphénylphosphine. On refroidit à −20°C puis ajoute lentement, 8 $cm^3$ de chloroforme dans 20 $cm^3$ de N-heptane.

On laisse une heure sous agitation à −20°C, puis à cette même température, ajoute lentement, 7,86 g

de (1R,trans)2,2-diméthyl 3-(2-méthyl 1-propenyl)cyclopropane carboxylate de (1S)2-méthyl 3-allyl 4-oxo cyclopent-2-èn 1-yle en solution dans 20 $cm^3$ de tétrahydrofuranne.

On laisse sous agitation pendant une heure à −20°C puis pendant une heure à 0°C douze heures à 20°C.

On évapore le solvant sous pression réduite et obtient 21 g de produit brut que l'on empâte dans l'éther de pétrole. (Eb. 40—70°C).

On filtre, lave le filtrat par une solution aqueuse saturée de phosphate monosodique puis à l'eau, enfin sèche et évapore à sec. On obtient 11 g de produit brut que l'on chromatographie une première fois sur silice en éluant au mélange cyclohexane-acétate d'éthyle (98-2) à 1 pour 1000 de triéthylamine puis une seconde fois par un mélange cyclohexane-toluène (6-4) et obtient 1,95 g de produit attendu.

$[\alpha]_D$ = −95,5° ± 2,5° (c = 0,7%, benzène)

Analyse ($C_{20}H_{26}Cl_2O_2$):

| | | | |
|---|---|---|---|
| Calculé: | C 65,04, | H 7,09, | Cl 19,19% |
| Trouvé: | C 65,0, | H 7,0, | Cl 19,0% |

Spectre IR (chloroforme)

Absorption à 1700 $cm^{-1}$ (C=O ester)
Absorptions à 1638—1605 $cm^{-1}$ (C=C conjugué)
Absorption à 1381 $cm^{-1}$ (gem diméthyle)
Absorptions à 994—918 $cm^{-1}$ (allyle)

Spectre RMN ($CDCl_3$ 60 MHz)

Pic à 1,72 p. p. m. attribué aux hydrogènes du méthyle en 2 du propényle et aux hydrogènes en 3 du propényle.
Pics de 4,83 à 5,17 p. p. m. attribués à l'hydrogène en 1 du propényle.
Pics à 1,15—1,28 p. p. m. attribués aux hydrogènes des méthyles en 2 de cycle cyclopropane.
Pics à 1—1,44 p. p. m. attribués à l'hydrogène en 1 du cycle cyclopropane.
Pics de 5,5 à 5,58 p. p. m. attribués à l'hydrogène en 1 du cycle cyclopentyle.
Pic à 1,78 p. p. m. attribué aux hydrogenes du méthyle en 2 du cycle cyclopentyle.
Pics à de 5,5 à 5,25 p. p. m. attribués à l'hydrogène en 2 de la chaîne allyle.
Pics de 4,83 à 5,17 p. p. m. attribués aux hydrogènes en 3 de la chaîne allyle.

### Exemple 54

### (1R,trans)2,2-diméthyl 3-(2-méthyl 1-propényl) cyclopropane carboxylate carboxylate de (1S)-2-méthyl 3-allyl 4-dicyanométhylène cyclopent-2-èn 1-yle

On mélange 3 g de (1S)-hydroxy 2-méthyl 3-allyl 4-dicyanométhylène cyclopent-2-ène avec 3 g de chlorure de l'acide (1R,trans)2,2-diméthyl 3-(2-méthyl 1-propényl)cyclopropane carboxylique et 50 $cm^{-1}$ de benzène puis ajoute à 5°C 1,5 $cm^3$ de pyridine et maintient pendant quinze minutes à 5°C puis pendant deux heures, à 20°C. On verse dans un mélange de 18 $cm^3$ d'acide chlorhydrique 2N et 100 $cm^3$ d'eau à + 5°C, extrait à l'éther, lave la phase organique à l'eau, la sèche et évapore le solvant.

On obtient 6,5 g de produit attendu brut que l'on purifie par chromatographie sur silice en éluant au mélange toluène-acétate d'éthyle (97-3), et obtient 4,93 g de produit attendu.

$[\alpha]_D^{20}$ = −162,5° ± 3,5° (c = 0,6% benzène)

Analyse ($C_{22}H_{26}N_2O_2$):

| | | | |
|---|---|---|---|
| Calculé: | C 75,39, | H 7,47, | N 7,99% |
| Trouvé: | C 75,3, | H 7,6, | N 7,9% |

Spectre IR ($CHCl_3$)

Absorption à 2220 $cm^{-1}$ (C≡N conjugué)

Absorption à 1712 $cm^{-1}$ (C=O ester)
Absorptions à 1640—1617—1570 $cm^{-1}$ (système conjugué)
Absorptions à 995—920 $cm^{-1}$ (allyle)

Spectre RMN ($CDCl_3$ 60 MHz)

Pic à 1,73 p. p. m. attribué aux hydrogènes du méthyle en 2 du propényle et aux hydrogènes en 3 du propényle.
Pics à 4,83—4,96 p. p. m. attribués à l'hydrogène en 1 du propényle.
Pics à 1,36—1,45 p. p. m. attribués à l'hydrogène en 1 du cycle cyclopropane.
Pics à 1,17—1,29 p. p. m. attribués aux hydrogènes des méthyles en 2 du cycle cyclopropane.
Pic à 5,67 p. p. m. attribué à l'hydrogène en 1 du cycle cyclopentyle.
Pic à 2,03 p. p. m. attribué aux hydrogènes du méthyle en 2 du cycle cyclopentyle.
Pics à 3,33—3,43 p. p. m. attribués aux hydrogènes en 1 de la chaîne allyle.
Pics de 5,58 à 6,25 p. p. m. attribués à l'hydrogène en 2 de la chaîne allyle.
Pics de 4,92 à 5,25 p. p. m. attribués aux hydrogènes en 3 de la chaîne allyle.

Le (1S)-hydroxy 2-méthyl 3-allyl 4-dicyanométhylène cyclopent-2-ène utilisé au depart de l'exemple 54 a été préparé comme suit:
On opère de manière analogue à celle décrite à l'exemple 45, au départ de 45 g de (1S)-hydroxy 2-méthyl 3-allyl 4-oxo cyclopent-2-ène et de 24 g de malonitrile. On abtient après chromatographie sur silice dans le mélange toluène-acétate d'éthyle (6/4) puis (8/2), 34,9 g de produit attendu.

$[\alpha]_D^{20} = -203° \pm 4°$ (c = 0,5% benzène)

Analyse ($C_{12}H_{12}N_2O$):

| | | | |
|---|---|---|---|
| Calculé: | C 71,98, | H 6,04, | N 13,99% |
| Trouvé: | C 71,8, | H 6,2, | N 13,8% |

Spectre IR (chloroforme)

Absorption à 3600 $cm^{-1}$ caractéristique de OH
Absorption à 2222 $cm^{-1}$ caractéristique de C≡N
Absorptions à 1637, 1611 et 1567 $cm^{-1}$, caractéristique de C=C conjuguée
Absorptions à 990 et 920 $cm^{-1}$ caratéristique de $CH=CH_2$ (déformation).

Spectre RMN ($CDCl_3$, 60 MHz)

Pic à 4,75 p. p. m. caractéristique de l'hydrogène en 1.
Pic à 2,58 p. p. m. caractéristique de l'hydrogène du OH.
Pic à 2,07 p. p. m. caractéristique des hydrogènes du méthyle en 2.
Pics de 3,25 à 3,42 p. p. m. caractéristiques des hydrogènes en 1 de la chaîne allyle.
Pics de 5,5 à 6,25 p. p. m. caractéristiques de l'hydrogène en 2 de la chaîne allyle.
Pics de 4,67 à 5,25 p. p. m. caractéristiques des hydrogènes en 3 de la chaîne allyle.

### Exemple 55
### (1R,cis)2,2-diméthyl 3-éthynyl cyclopropane carboxylate de (1S)-2-méthyl 3-allyl 4-méthylène cyclopent-2-èn 1-yle

On opère comme à l'exemple 54 au départ de 2,5 g de (1S)-hydroxy 2-méthyl 3-allyl 4-méthylène cyclopent-2-ène et de 2,5 g de chlorure d'acide (1R,cis)2,2-diméthyl 3-éthynyl cyclopropane carboxylique abtenu selon le procédé décrit cidessous et obtient après chromatographie sur silice en éluant avec le mélange éther de pétrole (Eb. 40—70°C)-éther (95-5) à 1 pour 1000 de triéthylamine 1,26 g de produit attendu.

$[\alpha]_D^{20} = -83° \pm 3°$ (c = 0,6% chloroforme)

Spectre IR (chloroforme)

Absorption à 3300 $cm^{-1}$ (CH de l'éthynyl)
Absorption à 2225 $cm^{-1}$ (C≡C)
Absorption à 1728 $cm^{-1}$ (carbonyl de l'ester)
Absorptions à 1390 et 1379 $cm^{-1}$ (gem diméthyle)
Absorption à 1692 $cm^{-1}$ (C=C).

Spectre RMN ($CDCl_3$, 60 MHz)

Pic à 2,1 p. p. m. attribué à l'hydrogène en 3 de la chaîne éthynyle pics de 1,18 à 1,38 p. p. m. attribués aux hydrogènes des méthyles en 2 du cyclopropane.
Pic vers 1,7 p. p. m. attribué aux hydrogènes en 1 et en 3 du cycle cyclopropane.
Pics de 4,6 à 5,25 p. p. m. attribués aux hydrogènes du méthylène et aux hydrogènes en 3 de l'allyle.
Pics à 2,9 et 3 p. p. m. attribués aux hydrogènes en 1 de l'allyle.
Pics de 5,48 à 6,16 p. p. m. attribués aux hydrogènes en 2 de l'allyle.
Pic à 1,78 p. p. m. attribué à l'hydrogène du méthyle en 2 du cycle cyclopentyle.
Pic à 5,66 p. p. m. attribué àl'hydrogène en 1 du cycle cyclopentyle.

Le chlorure d'acide (1R,cis)2,2-diméthyl 3-éthynyl cyclopropane carboxylique utilisé au départ de l'exemple 55 a été préparé comme suit:

A) Acide (1R,cis)3-éthynyl 2,2-diméthyl cyclopropane-1-carboxylique

On dissout 20 g de 4-butyl lithium dans 100 ml de cyclohexane, refroidit à −55 °C et ajoute lentement sous agitation un mélange de 100 $cm^3$ de tétrahydrofuranne et 100 $cm^3$ d'éther, maintient sous agitation à −62°C, ajoute 15 g d'acide (1R,cis)2,2-diméthyl 3-(2,2-dichlorovinyl) cyclopropane-1-carboxylique en solution dans 20 $cm^3$ d'éther et 20 $cm^3$ de tétrahydrofuranne et laisse revenir à température ambiante en maintenant sous agitation pendant 48 heures. On refroidit à 5 °C, ajoute 9 $cm^3$ d'isopropanol puis 100 $cm^3$ d'acide sulfurique 2N. On décante, extrait à l'éther, lave la phase organique à l'eau, la sèche et concentre sous pression réduite. On reprend le résidu par 5 $cm^3$ d'éther de pétrole, glace, filtre, sèche et obtient 4,7 g de produit attendu.

B) Chlorure de l'acide (1R,cis)2,2-diméthyl 3-éthynyl cyclopropane-1-carboxylique

On mélange 7 g d'acide (1R,cis)2,2-diméthyl 3-éthynyl cyclopropane-1-carboxylique, 45 $cm^3$ d'éther de pétrole (Eb. 40—70°C), 15 $cm^3$ de chlorure de thionyle et chauffe au reflux pendant 3 heures. On chasse le solvant sous pression réduite, rectifie le résidu, obtient 7 g de produit attendu.

Exemple 56
(1R,trans)2,2-diméthyl 3-éthynyl cyclopropane carboxylate de (1S)-2-méthyl 3-allyl 4-méthylène cyclopent-2-èn 1-yle

On opère comme à l'exemple 54 au départ de 1,4 g de (1S)-hydroxy 3-allyl 4-méthylène cyclopent-2-ène et de 1,2 g de chlorure d'acide (1R,trans)2,2-diméthyl 3-éthynyl cyclopropane carboxylique et obtient après chromatographie sur silice en éluant avec le mélange éther de pétrole (Eb. 40—70°C) éther (95-5) à 1 pour 1000 de triéthylamine 0,83 g de produit attendu.

$[\alpha]_D^{20} = -39,5° \pm 1,5°$ (c = 1,5% chloroforme)

Spectre IR (chloroforme)

Absorption à 3300 $cm^{-1}$ (CH de l'éthynyl)
Absorption à 1712 $cm^{-1}$ (carbonyl de l'ester)
Absorption à 1633 $cm^{-1}$ (C=C conjuguée)
Absorption à 2225 $cm^{-1}$ (C≡C)
Absorption à 985—915 $cm^{-1}$ (allyle).

Spectre RMN ($CDCl_3$, 60 MHz)

Pic à 1,95 p. p. m. attribué à l'hydrogène en 3 de la chaîne éthynyle.
Pics à 1,23 et 1,3 p. p. m. attribués aux hydrogènes des méthyles en 2 du cycle cyclopropane.
Pic vers 5,58 p. p. m. attribué à l'hydrogène en 1 du cycle cyclopentyle.
Pic à 1,8 p. p. m. attribué aux hydrogènes du méthyle en 2 du cycle cyclopentyle.
Pics de 4,75 à 5,16 p. p. m. attribués aux hydrogènes du méthylène et aux hydrogènes en 3 de l'allyle.
Pics de 5,52 à 6,23 p. p. m. attribués aux hydrogènes en 2 de l'allyle.
Pics de 2,94 à 3 p. p. m. attribués aux hydrogènes en 1 de l'allyle.

Le chlorure d'acide (1R,trans)2,2-diméthyl 3-éthynyl cyclopropane carboxylique utilisé au départ de l'example 56 a été préparé à partir d'acide (1R,trans)2,2-diméthyl 3-(2,2-dichlorovinyl)cyclopropane carboxylique selon un mode opératoire identique décrit dans l'exemple 55.

Exemple 57
2,2,3,3-tétraméthyl cyclopropane carboxylate de (1S)-2-méthyl 3-allyl 4-méthylène cyclopent-2-èn 1-yle

On mélange sous agitation 3,72 g d'acide 2,2,3,3-tétraméthyl cyclopropane carboxylique, 100 $cm^3$ de chlorure de méthylène, 4,33 g de (1S)-hydroxy 2-méthyl 3-allyl 4-méthylène cyclopent-2-ène et 0,4 g de diméthyl aminopyridine. On refroidit à 0°C et introduit lentement 6,54 g de dicyclohexylcarbodiimide dans 10 $cm^3$ de chlorure de méthylène, laisse 27 heures à température ambiante, filtre, concentre, reprend à l'éther de pétrole, lave à l'eau, sèche, évapore à sec. Après chromatograpie sur silice en éluant par le mélange éther de pétrole (Eb. 40—70°C)-éther (9-1) à 1 pour 1000 de triéthylamine, on obtient 4,1 g de produit attendu.

$[\alpha]_D^{20} = -56° \pm 2,5°$ (c = 0,7% éthanol)

Spectre IR (chloroforme)

Absorption à 1710 $cm^{-1}$ (C=O ester)
Absorption à 1632 $cm^{-1}$ (C=C conjugué)
Absorption à 1394—1380 $cm^{-1}$ (gem diméthyle)
Absorption à 990—915 $cm^{-1}$ (allyle)
Absorption à 866 $cm^{-1}$ (méthylène).

Spectre RMN ($CDCl_3$, 60 MHz)

Pics à 1,17 et 1,25 p. p. m. attribués aux hydrogènes des méthyles en 2 et en 3 du cyclopropane.
Pic à 5,58 p. p. m. attribué à l'hydrogène en 1 du cycle cyclopentyle.
Pics de 4,73 à 5,2 p. p. m. attribués aux hydrogènes du méthylène et aux hydrogènes en 3 de l'allyle.
Pics de 5,5 à 6,16 p. p. m. attribués aux hydrogènes en 2 de l'allyle,
Pics de 2,95 à 3,05 p. p. m. attribués aux hydrogènes en 1 de l'allyle.
Pic à 1,8 p. p. m. attribué à l'hydrogène du méthyle en 2 du cycle cyclopentyle.

Exemple 58
(1R,cis)2,2-diméthyl 3-(2-(E + Z)-cyano 2-phényl éthényl)cyclopropane carboxylate de (1S)-2-méthyl 3-allyl 4-méthylène cyclopent-2-ène 1-yle

On chauffe à 80°C pendant 15 minutes solution de 3,3 g d'acide (1R,cis)2,2-diméthyl 3-(2-(E + Z)-cyano 2-phényl éthényl)cyclopropane carboxylique dans 4 $cm^3$ de chlorure de thionyle puis évapore le solvant. On dissout le chlorure d'acide obtenu dans 50 $cm^3$ de benzène, ajoute 2,57 g de (1S)-hydroxy 2-méthyl 3-allyl 4-méthylène cyclopent-2-ène et 1,9 $cm^3$ de pyridine, agite 16 heures à température ambiante et dilue par 200 $cm^3$ de benzène. On lave la phase benzénique par 30 $cm^3$ d'une solution d'acide chlorhydrique N puis 15 $cm^3$ d'une solution de bicarbonate de potassium M, puis 30 $cm^3$ d'eau enfin sèche et évapore à sec. Après chromatographie sur silice en éluant avec le mélange cyclohexane-acétate d'éthyle (9-1) on obtient 3,4 g de produit attendu.

$[\alpha]_D^{20} = -39,5° \pm 2,5°$ (c = 0,5% benzène)

Spectre IR (chloroforme

Absorption à 2215 $cm^{-1}$ (C≡N conjuguée)
Absorption à 1711 $cm^{-1}$ (C=O ester)
Absorptions à 1631—1603 $cm^{-1}$ (C=C + conjuguée)
Absorptions à 1596—1490 $cm^{-1}$ (aromatique)
Absorptions à 990—913 $cm^{-1}$ (allyl : déformation)
Absorptions à 864 $cm^{-1}$ (C=C du méthylène de l'alléthrolone).

Spectre RMN ($CDCl_3$, 60 MHz)

Pics à 1,35 et 1,38 p. p. m attribués aux hydrogènes des méthyles en 2 du cycle cyclopropyle.
Pic à 1,78 p. p. m attribué aux hydrogènes du méthyle en 2 du cycle cyclopentyle.
Pics de 4,75 à 5,25 p. p. m attribués aux hydrogènes des méthylènes.
Pics de 5,5 à 6,16 p. p. m attribués aux hydrogènes en 2 de l'allyle.
Pic vers 5,58 p. p. m attribué à hydrogène en 1 du cycle cyclopentyle.
Pics de 7,17 à 7,66 p. p. m attribués aux hydrogènes aromatiques et hydrogènes éthyléniques de la chaîne de l'acide.

L'acide (1R,cis)2,2-diméthyl 3-(2-(E + Z)-cyano 2-phényl éthényl)cyclopropane carboxylique utilisé au départ de l'exemple 58 a été préparé comme suit:

On dissout 3,5 g de méthylate de potassium dans 70 $cm^3$ de méthanol, ajoute 5,8 g de cyanure de benzyle 7,1 g de lactone de l'acide (cis)2,2-diméthyl (3S)-dihydroxyméthyl cyclopropane-1R-carboxylique. On chauffe au reflux pendant 90 minutes, chasse le solvant, reprend le résidu dans 75 $cm^3$ d'une solution d'acide chlorhydrique N et 30 $cm^3$ d'éther, agite 15 minutes à température ambiante, extrait à l'acétate d'éthyle, sèche, concentre à sec, lave le produit brut à l'éther de pétrole (Eb. 60—80°C) et obtient 9,5 g de produit attendu.

Spectre IR (chloroforme)

Absorption à 3500 $cm^{-1}$ (OH acide)
Absorption à 2215 $cm^{-1}$ (C≡N conjugué)
Absorptions à 1730—1695 $cm^{-1}$ (C=O)
Absorptions à 1607 $cm^{-1}$, (épaulement) 1598 $cm^{-1}$ 1493 $cm^{-1}$ (aromatiques)
Absorption à 690 $cm^{-1}$ (phényl).

Etude de l'activité insecticide des composés de l'invention

I) Protocoledes tests utilisés

a) Etude de l'activité Knock-down sur mouches domestiques

Les insectes utilisés sont des mouches femelles âgées de 3 jours. On opère par pulvérisation directe en chambre de Kearns et March en utilisant comme solvant un mélange en volumes égaux d'acétone et de Kérosène (quantité de solution utilisée 2 × 0,2 $cm^3$ ). On utilise environ 50 insectes par traitement. On effectue les contrôles deux, quatre, six, huit, dix et quinze minutes après pulvérisation. Voir tableau ci-après.

b) Etude de l'activité létale sur mouche domestique

Les insectes test sont des mouches domestiques femelles âgées de 4 jours. On opère par application topique de 1 µl de solution acétonique sur le thorax dorsal des insectes à l'aide du micromanipulateur d'Arnold. On utilise 50 individus par traitement. On effectue le contrôle de mortalité vingt-quatre heures après traitement.

Les essais sont effectués sans synergiste ou avec addition de butoxyde de pipéronyle (10 parties de synergiste pour 1 partie de composé à tester).

Les résultats expérimentaux, résumés dans un tableau, sont exprimés en DL 50 ou dose (en nanogrammes) nécessaire pour tuer 50% des insectes.

c) Etude de l'activité insecticide sur chenilles de Spodoptera littoralis

Les essais sont effectués par application topique. On dépose 1 µl d'une solution acétonique du produit à tester sur le thorax dorsal de chaque individu sauf mention particulière. On utilise 15 chenilles de Spodoptera littoralis au 4ème stade larvaire, pour chaque dose employée.

Après traitement, les individus sont placés sur un milieu nutritif artificiel (milieu de Poitout). On effectue le contrôle d'efficacité (pourcentage de mortalité compte tenu d'un témoin non traité) 24 heures puis 48 heures et éventuellement 6 jours après traitement et l'on détermine la dose létale 50 ($DL_{50}$) en nanogrammes par chenille.

Les résultats expérimentaux sont résumés dans un tableau.

d) Etude de l'activité insecticide sur larves d'Epilachna varivestris

Les essais sont effectués par application topique de manière analogue à celle utilisée pour les larves de Spodoptera. On utilise des larves de l'avant dernier stade larvaire et après traitement les larves sont alimentées sur des plants de haricots. On effectue le contrôle mortalité 72 h après traitement.

Les résulats expérimentaux sont résumés sous forme de tableau.

e) Etude de l'activité insecticide sur Sitophilus granarius ou Tribolium castaneum

Le test est effectué par application topique de manière analogue à celle utilisée pour larves de Spodoptera. Après traitement les insectes sont conservés sur grain de blé les observations de mortalité sont effectuées 24 h, 48 h et 6 ou 7 jours traitement.

Les résultats expérimentaux obtenus sont résumés dans un tableau.

f) Etude de l'activité insecticide sur Blatella germanica (adultes mâles)

Le test effectué est un test par film sur verre. On dépose dans des boîtes de Petri de 154 $cm^2$, 1,54 $cm^3$ de solution acétonique à 10 mg/l de produit à tester, puis laisse l'acétone s'évaporer: le film formé correspond à 0,1 mg de matière active par $m^2$.

On retire ensuite les insectes de la boîte de Petri et les transvase dans des bocaux propres. On effectue des contrôles de mortalité au bout de 24 heures, 48 heures et 6 jours.

Les résultats expérimentaux sont résumés dans un tableau.

g) Etude de l'activité insecticide sur Aphis craccivora

On opère par application topique d'une microgoutte de solution acétonique du composé à tester. Les résultats expérimentaux sont résumés dans un tableau.

Dans ce qui suit les $DL_{50}$ sont expérimés en nanogrammes par insecte.

h) Etude de l'activité létale sur Aedes aegypti

La méthode utilisée dans ce test est la méthode O. M. S. 20 adultes sont laissés 1 heure en observation dans un tube non traité, puis mis en contact avec le papier traité. Le traitement est effectué en déposant à la pipette de la solution acétonique de produit à tester sur une feuille de papier filtre de 180 $cm^2$. La contamination est faire 24 heures après le traitement du papier. Il y a deux répétitions par dose.

i) Etude de l'activité de Knock-down sur Aedes aegypti

La méthode utilisée est analogue à celle décrite précédemment pour l'effect létal mais le comptage des insectes abattus est effectué à intervalles rapprochés (2, 4, 6, 8 minutes.), jusqu'à ce qu'on ait observé que tous les insectes sont abattus. On détermine ainsi un $KT_{50}$ (Knock-down 50).

II) Etude expérimentale de l'activité insecticide de divers composés de l'invention

a) Etude de l'activité insecticide du (1R,trans)2,2-diméthyl 3-(2-méthyl 1-propényl) cyclopropane-1-carboxylate de (1S)2-méthyl 3-allyl 4-méthylène cyclopent-2-èn-1-yle (composé A)

1) Activité de Knock-down sur mouche domestique

| | Doses en mg/L | % de KD 2 mn | 4 mn | 6 mn | 8 mn | 10 mn | 15 mn | $KT_{50}$ en mn |
|---|---|---|---|---|---|---|---|---|
| non synergisé | 500 | 0 | 0 | 6,7 | 33,3 | 56,7 | 93,3 | 8,8 |
| synergisé | 500 | 3,3 | 6,7 | 30,0 | 56,7 | 73,3 | 96,7 | 7,0 |

2) Activité létale sur mouche domestique

| | Doses en ng par insecte | Nombre d'individus utilisés | % de mortalité à 24 heures | $DL_{50}$ en ng par insecte |
|---|---|---|---|---|
| sans synergiste | 500 | 30 | 100 | 32 |
| | 250 | 30 | 100 | |
| | 100 | 60 | 100 | |
| | 50 | 92 | 96,7 | |
| | 37,5 | 59 | 84,7 | |
| | 25 | 90 | 47,8 | |
| | 10 | 90 | 3,3 | |
| avec synergiste | 500 | 30 | 100 | 13 |
| | 100 | 30 | 100 | |
| | 25 | 90 | 100 | |
| | 20 | 90 | 92,2 | |
| | 15 | 89 | 68,5 | |
| | 10 | 120 | 24,2 | |

3) Activité insecticide sur Aphis craccivora

| Doses en nanogrammes par insecte | % de mortalité au bout de 1 h | 2 h | 24 h | 48 h |
|---|---|---|---|---|
| 20 | 100 | 100 | 100 | 100 |
| 2 | 100 | 100 | 95,0 | 100 |
| témoin | 0 | 0 | 0 | 0 |

4) Activité sur Spodoptera littoralis

| | Doses en nanogrammes par insecte | % de mortalité au bout de 24 h | 48 h | 6 jours |
|---|---|---|---|---|
| produit | 200 | 100 | 100 | 100 |
| synergisé | 20 | 100 | 96,1 | 96,1 |

5) Activité sur Sitophilus granarius

| | Doses en nanogrammes par insecte | % de mortalité au bout de 24 h | 48 h | 6 jours |
|---|---|---|---|---|
| produit | 200 | 100 | 100 | 100 |
| synergisé | 20 | 100 | 96,1 | 96,1 |

6) Activité sur Tribolium castaneum

| | Doses en nanogrammes par insecte | % de mortalité au bout de 24 h | 48 h | 6 jours |
|---|---|---|---|---|
| produit | 200 | 100 | 100 | 100 |
| synergisé | 20 | 30,0 | 24,0 | 30,0 |

7) Conclusion:
Le composé A est doué d'une intéressante activité insecticide.

b) Etude de l'activité insecticide du (1R,trans)2,2-diméthyl 3-cyclopentylidène méthyl cyclopropane-1-carboxylate de (1S)2-méthyl 3-allyl 4-méthylène cyclopent-2-èn 1-yle (composé B)

1) Activité de Knock-down mouches

| | Doses en mg de matière active par litre | % DE K. D. 2 mn | 4 mn | 6 mn | 8 mn | 10 mn | 15 mn | $KT_{50}$ en minutes |
|---|---|---|---|---|---|---|---|---|
| pas synergisé | 500 | 0 | 3,3 | 6,7 | 16,7 | 43,3 | 76,7 | 11,0 |
| synergisé | 500 | 0 | 3,3 | 16,7 | 33,3 | 63,3 | 100 | 9,5 |

2) Activité létale sur mouche

| Doses de matière active en nanogrammes par insecte | Nombre d'individus utilisé | % de mortalité à 24 heures | $DL_{50}$ en nanogramme par insecte |
|---|---|---|---|
| 100 | 60 | 100 | 5,19 |
| 50 | 60 | 100 | |
| 25 | 60 | 100 | |
| 15 | 60 | 100 | |
| 10 | 90 | 100 | |
| 7,5 | 60 | 93,3 | |
| 5 | 120 | 43,8 | |
| 1 | 90 | 4,0 | |

3) Activité létale sur Aedes aegypti

| Doses en mg de matière active par $m^2$ | % de mortalité au bout de | | $DL_{50}$ à 24 h |
|---|---|---|---|
| | 1 h | 24 h | |
| 10 | 100 | 100 | 0,75 |
| 5 | 100 | 100 | |
| 1 | 45 | 80 | |
| 0,5 | 8,3 | 12,5 | |

4) Activité de Knock-down sur Aedes aegypti
Activité de produits utilisés à 83,3 mg de matière active par $m^2$

| % insectes abattus au bout de | | | |
|---|---|---|---|
| 2 mn | 4 mn | 6 mn | 8 mn |
| 0 | 31,9 | 76,5 | 100 |

5) Activité sur Aphis craccivora

| Doses en nanogrammes par insecte | % de mortalité au bout de | | | |
|---|---|---|---|---|
| | 1 h | 2 h | 24 h | 48 h |
| 20 | 80,0 | 75,0 | 100 | 100 |
| 2 | 50,0 | 65,0 | 100 | 100 |

6) Activité sur Spodoptera littoralis

| Doses en nanogrammes par insecte | % de mortalité au bout de 24 h | 48 h |
|---|---|---|
| 1000 | 100 | 100 |
| 100 | 100 | 100 |
| 10 | 70,0 | 100 |

7) Activité sur Sitophilus granarius

| Doses en nanogrammes par insecte | % de mortalité au bout de 24 h | 48 h | 6 jours |
|---|---|---|---|
| 200 | 100 | 100 | 100 |
| 20 | 98,0 | 98,0 | 94,1 |

8) Activité sur Tribolium castaneum

| Doses en nanogrammes par insecte | % de mortalité au bout de 24 h | 48 h | 6 jours |
|---|---|---|---|
| 200 | 100 | 100 | 100 |
| 20 | 86,0 | 80,0 | 82,0 |

9) Conclusion:
Le composé B est doué d'une intéressante activité insecticide

c) Etude de l'activité insecticide du (1R,trans)2,2-diméthyl 3-(2,2-dichloroéthényl) cyclopropane-1-carboxylate de (1S)2-méthyl 3-allyl 4-méthylène cyclopent-2-èn-1-yle (composé C).

1) Activité de Knock-down sur mouche

| Doses en mg de matière active par litre | % DE K. D. 2 mn | 4 mn | 6 mn | 8 mn | 10 mn | 12 mn | 15 mn | $KT_{50}$ en minutes |
|---|---|---|---|---|---|---|---|---|
| 1000 | 4 | 28 | 54 | 86 | 98 | – | 100 | 8,7 |
| 500 | 1,8 | 1,8 | 8,8 | 31,6 | 70,2 | – | 91,2 | 14,5 |
| 250 | 0 | 0 | 2 | 9,8 | 17,6 | 35,3 | 60,8 | 26,0 |

2) Activité létale sur mouche

| | Doses en nanogrammes de matière active par insecte | % de mortalité à 24 h | $DL_{50}$ en ng par insecte |
|---|---|---|---|
| non synergisé | 10 | 83,3 | 6,3 |
| | 7,5 | 61 | |
| | 5 | 37,1 | |
| | 2,5 | 11 | |
| synergisé | 7,5 | 85,8 | 5,4 |
| | 5 | 40,4 | |
| | 2,5 | 6,7 | |
| | 1 | 5,6 | |

3) Activité létale sur Aphis craccivora

| Doses en nanogrammes par insecte | % de mortalité au bout de 1 h | 2 h | 24 h | 48 h |
|---|---|---|---|---|
| 20 | 100 | 100 | 100 | 100 |
| 2 | 15 | 35 | 100 | 100 |

4) Activité létale sur Spodoptera littoralis

| Doses en nanogrammes par insecte | % de mortalité au bout de 24 h | 48 h |
|---|---|---|
| 100 | 100 | 100 |
| 10 | 100 | 100 |

5) Activité sur Blatella germanica

| Doses en nanogrammes par insecte | % de mortalité au bout de 24 h | 48 h | 6 jours |
|---|---|---|---|
| 1000 | 100 | 100 | 100 |

6) Activité sur Sitophilus granarius

| Doses en mg/litre | mg de matière active par Kg de blé ( p. p. m.) | efficacité à 7 jours |
|---|---|---|
| 200 | 10 | 100 |
| 20 | 1 | 100 |

7) Activité sur Tribolium castaneum

| Doses en mg/litre | mg de matière active par Kg de blé ( p. p. m.) | efficacité à 7 jours |
|---|---|---|
| 200 | 10 | 100 |
| 20 | 1 | 100 |

8) Conclusion:
Le composé C est doué d'une intéressante activité insecticide.

d) Etude insecticide du (1R,trans)2,2-diméthyl 3-(2,2-dibromo-éthényl cyclopropane-1-carboxylate de (1S)2-méthyl 3-allyl 4-méthylène cyclopent-2-èn-1-yle (composé D).

1) Activité de Knock-down sur mouche

| | Poids en mg de solution pulvérisée | % DE K. D. 2 mn | 4 mn | 6 mn | 8 mn | 10 mn | 15 mn | $KT_{50}$ en minutes |
|---|---|---|---|---|---|---|---|---|
| non synergisé | 491 | 0 | 0 | 38 | 74 | 92 | 100 | 6,5 |
| synergisé | 507 | 0 | 18 | 74 | 98 | 100 | 100 | 5,2 |

2) Activité létale sur mouche

| | Doses en mg de matière active par litre | % de mortalité à 24 h | $DL_{50}$ en ng par insecte |
|---|---|---|---|
| non synergisé | 100 | 93,3 | 24,0 |
| | 50 | 85,0 | |
| | 25 | 55,0 | |
| | 10 | 6,7 | |
| synergisé | 100 | 100 | 8,5 |
| | 50 | 83,3 | |
| | 10 | 53,2 | |
| | 1 | 20,0 | |

3) Activité sur Aphis craccivora

| | Doses en nanogrammes par insecte | % de mortalité au bout de 1 h | 2 h | 24 h | 48 h |
|---|---|---|---|---|---|
| synergisé | 20 | 70,0 | 90,0 | 100 | 100 |
| | 2 | 25,0 | 40,0 | 65,0 | 85,0 |

4) Activité sur Spodoptera littoralis

| | Doses en nanogrammes par insecte | % de mortalité au bout de 24 h | 48 h |
|---|---|---|---|
| synergisé | 1000 | 100 | 100 |
| | 100 | 100 | 100 |
| | 50 | 80,0 | 80,0 |

5) Activité sur Blatella germanica

| | Doses en nanogrammes par insecte | % de mortalité au bout de 24 h | 48 h | 8 jours |
|---|---|---|---|---|
| synergisé | 1000 | 100 | 100 | 100 |
| | 100 | 100 | 100 | 100 |
| | 50 | 100 | 90,0 | 80,0 |

6) Activité sur Sitophilus granarius

| mg de matière active par litre | mg de matière active par Kg de blé (p. p. m.) | % efficacité à 7 jours |
|---|---|---|
| 200 | 10 | 100 |
| 20 | 1 | 100 |

7) Activité sur Tribolium castaneum

| | mg de matière active par litre | mg de matière active par Kg de blé (p. p. m.) | % efficacité à 7 jours |
|---|---|---|---|
| synergisé | 200 | 10 | 100 |
| | 20 | 1 | 100 |

8) Conclusion:
Le composé D est doué d'une intéressante activité insecticide.

e) Etude de l'activité insecticide du (1R,trans)2,2-diméthyl 3-cyclopentylidène méthyl cyclopropane-1-carboxylate de (1RS)2-méthyl 3-(3-méthyl 2-buténˇyl) 4-méthylène cyclopent-2-èn-1-yle (composé E).

1) Activité sur Spodoptera littoralis

| | Dose en nanogramme de matière active par insecte | % de mortalité au bout de 24 h | 48 h |
|---|---|---|---|
| synergisé | 1000 | 100 | 100 |
| | 100 | 60 | 60 |

2) Activité sur Blatella germanica

| | Dose en nanogramme de matière active par insecte | % de mortalité au bout de 24 h | 48 h | 6 jours |
|---|---|---|---|---|
| synergisé | 1000 | 100 | 100 | 100 |
| | 500 | 70 | 100 | 100 |

3) Activité sur Sitophilus granarius

| mg de matière active par litre | mg de matière active par Kg de blé (p. p. m.) | % efficacité à 7 jours |
|---|---|---|
| 200 | 10 | 100 |
| 20 | 1 | 98 |

4) Conclusion:
Le composé E est doué d'une intéressante activité insecticide.

f) Etude de l'activité insecticide du (1R,cis)2,2-diméthyl 3-(2,2-difluoroéthényl)cyclopropane-1-carboxylate de (1S)2-méthyl 3-allyl 4-dichlorométhylène cyclopent-2-èn 1-yle (composé F).

1) Activité létale sur mouche
La $DL_{50}$ a été trouvée égale à 36,2 sans addition de synergiste et à 26,3 avex addition de synergiste (butoxyde de pipéronyle).

2) Activité létale sur Spodoptera littoralis
La $DL_{50}$ a été trouvée égale à 37,6 sans additions de synergiste.

3) Activité létale sur Epilachna varivestris
La $DL_{50}$ a été trouvée égale à 5,4 sans addition de synergiste.

4) Conclusion:
Le composé F est doué d'une intéressante activité insecticide.

g) Etude de l'activité insecticide du (1R,trans)2,2-diméthyl 3-(2,2-difluoroéthényl)cyclopropane-1-carboxylate de (1S)2-méthyl 3-allyl 4-méthylène cyclopent-2-èn 1-yle (composé G).

1) Activité de Knock-down sur mouches
Le $KT_{50}$ a été trouvé égal à 3,6 sans addition de synergiste.

2) Activité létale sur mouches
La $DL_{50}$ a été trouvée égale à 2,7 sans synergiste et à 2,8 avex addition de butoxyde de pipéronyle.

3) Activité insecticide sur Epilachna varivestris
La $DL_{50}$ a été trouvée égale à 4,5.

4) Conclusion:
Le composé G est doué d'une intéressante activité insecticide.

h) Etude de l'activité insecticide du (1R,cis)2,2-diméthyl 3-(2,2-difluoroéthényl)cyclopropane-1-carboxylate de (1S)2-méthyl 3-allyl 4-méthylène cyclopent-2-èn 1-yle (composé H).

1) La $DL_{50}$ sur Epilachna varivestris a été trouvée égale à 3,5.

2) Conclusion:
Le composé H est doué d'une intéressante activité insecticide vis-à-vis d'Epilachna varivestris.

i) Etude de l'activité insecticide du (1R,trans)2,2-diméthyl 3-2(Z)cyanoéthényl/cyclopropane-1-carboxylate de (1S)2-méthyl 3-allyl 4-méthylène cyclopent-2-èn 1-yle (composé I).

1) Activité de Knock-down sur mouche
Le $KT_{50}$ a été trouvé égal à 2,64 sans addition de synergiste.

2) Activité létale sur Epilachna varivestris
La $DL_{50}$ a été trouvée égale à 4,77 sans addition de synergiste.

3) Conclusion:
Le composé I est doué d'une intéressante activité insecticide.

j) Etude de l'activité insecticide du (1R,trans)2,2-diméthyl 3-(2-fluoro 2-chloroéthényl)cyclopropane-1-carboxylate de (1S)2-méthyl 3-allyl 4-méthylène cyclopent-2-èn 1-yle (composé J).

1) Activité de Knock-down sur mouche
Le $KT_{50}$ a été trouvé égal à 5,2 sans addition de synergiste.

2) Activité létale sur mouche
La $DL_{50}$ a été trouvé égale à 14,5 sans addition de synergiste.

3) Activité létale sur Epilachna varivestris
La $DL_{50}$ a été trouvée égale à 2,24 sans addition de synergiste.
4) Conclusion:
Le composé J est doué d'une intéressante activité insecticide.

k) Etude de l'activité insecticide du (1R,trans)2,2-diméthyl 3-cyclobutylidène méthyl cyclopropane-1-carboxylate de (1S)2-méthyl 3-allyl 4-méthylène cyclopent-2-èn 1-yle (composé k).
1) Activité de Knock-Down sur mouche
Le $KT_{50}$ a été trouvé égal à 5 sans addition de synergiste.
2) Activité létale sur mouche
La $DL_{50}$ a été trouvé égale à 12,8 sans addition de synergiste.
La $DL_{50}$ Activité létale sur Epilachna varivestris
3) Activité létale sur Epilachna varivestris
La $DL_{50}$ a été trouvée égale à 2,16 sans addition de synergiste.
4) Conclusion:
Le composé K est doué d'une intéressante activité insecticide.

l) Etude de l'activité insecticide du (1R,cis)2,2-diméthyl 3-éthynyl cyclopropane-1-carboxylate de (1S)2-méthyl 3-allyl 4-méthylène cyclopent-2-èn 1-yle (composé L).
1) Activité de Knock-down sur mouche
Le $KT_{50}$ a été trouvé égal à 5,9 sans addition de synergiste.
2) Conclusion:
Le composé L est doué d'une intéresaante activité insecticide sur mouche.

## Etude de l'activité acaricide d'un composé de l'invention

Les tests sont effectués avec le (1R,cis)2,2-diméthyl 3-(2,2-dichloroéthényl)cyclopropane carboxylate de (S)-dichlorométhylène alléthrolone (composé M).

### I) Protocole des tests utilisés

#### a) Tests de laboratoire

L'etude est effectuée sur Tetranychus Urticae à 22°C ± 1°C et à 50% d'humidité relative sur plants de haricots. Les tests portent sur 3 séries simultanées, aux doses de 5−2,5et 1,25 g/hl, chaque série comportant 2 répétitions, indiqués dans le tableau ci-après $R_1$ et $R_2$, à raison de 50 acariens femelles par répétition.

On compte d'une part le pourcentage de mortalité à 24 heures sur feuille et d'autre part le pourcentage de répulsion à 24 heures. En effet, certains composés, dont le composé M sont doués d'un effet répulsif sur les acariens qui s'éloignent alors du support végétal. L'expérience montre qu'en général, ces acarines »repoussés« pendant 24 heures meurent rapidement (voir test en parcelles ci-après) et que l'on peut donc les incorporer dans le pourcentage d'efficacité total.

Les résultats expérimentaux sont résumés dans le tableau suivant:

| Produit | Dose utilisée (en g/hl) | % de mortalité à 24 h sur feuille | | | % de répulsion à 24 h | | | % d'efficacité totale à 24 h |
|---|---|---|---|---|---|---|---|---|
| | | $R_1$ | $R_2$ | Total $= T_1$ | $R_1$ | $R_2$ | Total $= T_2$ | $T_1 = T_2$ |
| Composée M | 5,0 | 60 | 26 | 43 | 40 | 60 | 50 | 93 |
| | 2,5 | 16 | 20 | 18 | 84 | 60 | 72 | 90 |
| | 1,25 | 16 | 0 | 8 | 84 | 70 | 77 | 85 |
| Témoin | – | – | – | – | – | – | – | 0 |

Conclusion:
Les efficacités totales du composé M au bout de 24 heures, pour des doses de 5,0−2,5−1,25 g/hl sont respectivement de 93−90−85%.
Le composé M s'avère donc être un composé acaricide intéressant.

b) Tests en parcelles

Ces tests ont porté sur haricots à l'aide de 6 séries simultanées, à raison de 50 acariens (Tetranychus urticae) par plants de haricots et par dose. Les doses de composé M utilisées ont été 10–5–2, 5–1, 25–0, 625–0, 312 g/hl. Les essais ont été effectués en parcelles à l'air libre.

Les contrôles ont été effectués 24, 48 et 72 heures après traitement par dénombrement des animaux vivants et morts sur le support végétal et hors dusupport végétal (effet répulsif).

Les résultats expérimentaux suivant ont été enregistrés:

| Doses utilisées en g/hl | % selon les contrôles à 24 h s/support végétal | | hors support | | 48 h s/support végétal | | hors support | | 72 h s/support végétal | | hors support | | % total de la mortalité 72 h |
|---|---|---|---|---|---|---|---|---|---|---|---|---|---|
| | Vivts | Morts | V | M | V | M | V | M | V | M | V | M | |
| 10 | 30 | 70 | 0 | 0 | 0 | 92 | 8 | 0 | 0 | 92 | 0 | 8 | 100 |
| 5 | 46 | 44 | 10 | 0 | 0 | 90 | 10 | 0 | 0 | 90 | 0 | 10 | 100 |
| 2,5 | 50 | 30 | 20 | 0 | 22 | 58 | 4 | 16 | 0 | 80 | 0 | 20 | 100 |
| 1,25 | 40 | 0 | 60 | 0 | 40 | 0 | 12 | 48 | 0 | 40 | 0 | 60 | 100 |
| 0,625 | 40 | 0 | 60 | 0 | 40 | 0 | 6 | 54 | 40 | 0 | 0 | 60 | 60 |
| 0,312 | 100 | 0 | 0 | 0 | 86 | 14 | 0 | 0 | 78 | 22 | 0 | 0 | 22 |

Conclusion

Les résultats des essais effectués en parcelles à l'air libre ont confirmé les résultats de laboratoire. L'activité globale apparaît même comme plus élevée en plein champ qu'au laboratoire. Le pourcentage total de mortalité est égal à 100 pour 2,5 g/hl au lieu de 85 dans l'essai de laboratoire. Cela est dû en partie au fait que la mortalité (ou l'effet répulsif) est lue au bout d'un temps plus long (48 et 72 heures).

Le composé M confirme son intérêt acaricide.

c) Conclusion

Dans les deux tests précédents le composé M s'avère être doué d'une activité acaricide intéressante vis-à-vis de Tetranychus urticae. Le composé M est doué d'un effet répulsif vis-à-vis de cet acarien.

Exemple de préparation d'un concentré émulsifiable

On effectue un mélange homogène de:

- (1R,trans)2,2-diméthyl 3-(2,2-difluoroéthényl)cyclopropane-1-carboxylate de (1S)-2-méthyl 3-allyl 4-méthylene cyclopent-2-èn 1-yle — 1,25 g
- Butoxyde de pipéronyle — 1 g
- Tween 80 — 0,25 g
- 2,4-diméthyl 6-tertbutyl phénol — 0,1 g
- Eau — 97,4 g

Exemple de préparation d'une composition fumigène

On mélange de façon homogène:

- (1R,trans)2,2-diméthyl 3-(2,2-difluoroéthényl)cyclopropane-1-carboxylate de (1S)-2-méthyl 3-allyl 4-méthylène cyclopent-2-èn 1-yle — 1,25 g
- Poudre de feuilles de Macillus Thumbergii — 25 g

- Poudre de feuilles de cèdre 40 g
- Poudre de bois de pin 32,75 g
- Vert brillant 0,5 g
- p-nitrophénol 0,5 g

Exemple de préparation d'un concentré émulsifiable

On effectue un mélange homogène de:

- (1R,trans)2,2-diméthyl 3-(2,2-dichloroéthényl)cyclopropane-1-carboxylate de (1S)-2-méthyl 3-allyl 4-méthylène cyclopent-2-èn 1-yle 2 g
- Tween 80 20 g
- 2,4-diméthyl 6-tert butylphénol 0,1 g
- Xylène 77,9 g

Exemple de préparation d'une composition vétérinaire à usage ixodicide

- (1R,trans)2,2-diméthyl 3-(2,2-difluoroéthényl)cyclopropane-1-carboxylate de (1S)-2-méthyl 3-allyl 4-méthylène cyclopent-2-èn 1-yle 4 g
- Butoxyde de pipéronyle 2,5 g
- Polysorbate 80 10 g
- Triton X 100 25 g
- Acétate de tocophérol 1 g
- Alcool éthylique 100 $cm^3$

**Revendications pour les Etats contractants: BE, CH/LI, DE, FR, GB, IT, LU, NL, SE**

1. Sous toutes leurs formes isomères possibles, les composés de formule générale (I'):

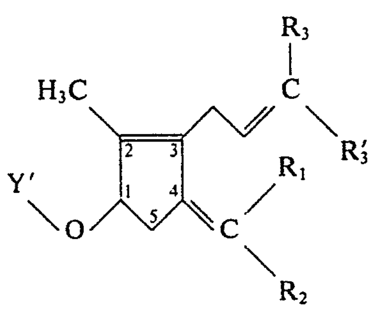

(I')

dans laquelle $R_1$ et $R_2$, identiques ou différents, représentent un groupement carbomoyle ou bien des groupements $R'_1$ et $R'_2$, lesquels, identiques ou différents, représentent un atome d'hydrogène, un atome d'halogène, un radical alcoyle comportant de 1 à 6 atomes de carbone, un radical aryle comportant de 6 à 10 atomes de carbone, un radical aralcoyle comportant de 7 à 13 atomes de carbone, un groupement alcoyloxycarbonyle comportant de 2 à 5 atomes de carbone ou un groupement cyano, $R_3$ et $R'_3$, identiques ou différents, représentent un atome d'hydrogène, un halogène ou un radical alcoyle saturé ou insaturé, comportant de 1 à 3 atomes de carbone, Y' représente ou bien un groupement:

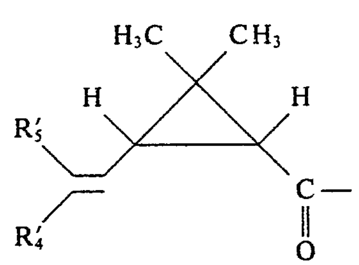

(A')

dans lequel soit $R'_4$ et $R'_5$, identiques ou différents, représentent un atome d'hydrogène, un radical alcoyle comportant de 1 à 4 atomes de carbone, un atome de fluor, de chlore ou de brome, soit $R'_4$ et $R'_5$ représentent ensemble avec l'atome de carbone auquel ils sont liés, un homocycle carboné ou un hétérocycle, comportant de 3 à 7 chaînons, ces cycles pouvant être éventuellement insaturés, ou bien

un hétérocycle de structure

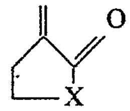

dans lequel X représente un atome d'oxygène ou de soufre, soit l'un des groupes $R'_4$ ou $R'_5$ représente un groupement cyano, un groupement

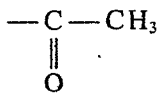 un groupement 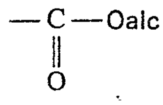

alc représentant un radical alcoyle renfermant de 1 à 4 atomes de carbone, lorsque l'autre représente un atome d'hydrogène, soit $R'_4$ représente un groupement cyano et $R'_5$ un radical phényle, les substituants du cycle cyclopropanique pouvant être de configuration cis ou trans, racémique ou optiquement active, la double liaison en position 1 de la chaîne latérale vinylique pouvant être de configuration (E) ou (Z), ou bien Y' représente un groupement:

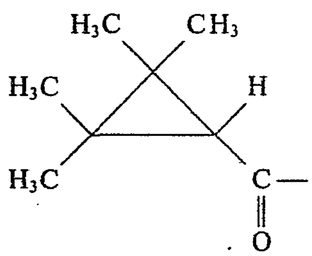

(B)

ou bien Y' représente un groupement:

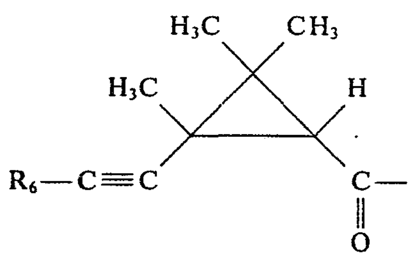

(C)

dans lequel $R_6$ représente un atome d'hydrogène, un radical alcoyle comportant de 1 à 4 atomes de carbone ou un groupenment alcoxycarbonyle comportant de 2 à 5 atomes de carbone, les substituants du cycle cyclopropanique pouvant être de configuration cis ou trans, racémique ou optiquement active, ou bien Y' représente un groupement:

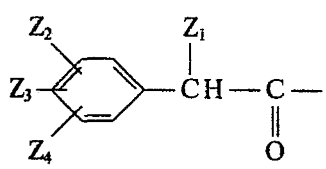

(D)

dans lequel $Z_1$ représente un radical alcoyle comportant de 1 à 4 atomes de carbone, $Z_2$, $Z_3$ et $Z_4$, identiques ou différents, représentent un atome d'hydrogène, un radical comportant de 1 à 4 atomes de carbone, un radical alcoyloxyle comportant de 1 à 4 atomes de carbone, un radical alcoyl thio comportant de 1 à 4 atomes de carbone, ou un atome d'halogène, le carbone asymétrique du groupement (D) pouvant être de configuration S, R ou racémique et le carbone asymétrique en position 1 du dérivé de l'alléthrolone pouvant être de configuration R, S ou racémique.

2. Sous toutes leurs formes isomères possibles les composés selon la revendication 1, de formule générale (I):

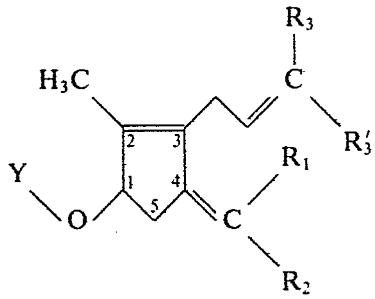

dans laquelle $R_1$, $R_2$, $R_3$ et $R'_3$ sont définis comme à la revendication 1, et Y représente ou bien un groupement:

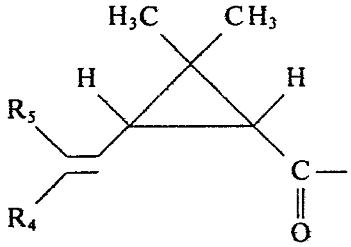

(A)

dans lequel soit $R_4$ et $R_5$, identiques ou différents, représentent un atome d'hydrogène, un radical alcoyle comportant de 1 à 4 atomes de carbone, un atome de fluor, de chlore ou de brome, soit $R_4$ et $R_5$ représentent ensemble avec l'atome de carbone auquel ils sont liés, un homocycle carboné ou un hétérocycle comportant de 3 à 7 chaînons, ces cycles pouvant être éventuellement insaturés, ou bien un hétérocycle de structure:

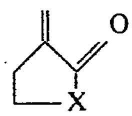

dans lequel X représente un atome d'oxygène ou de soufre, soit l'un des groupes $R_4$ ou $R_5$ représente un groupement cyano, un groupement

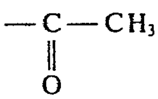 un groupement 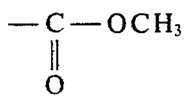

lorsque l'autre représente un atome d'hydrogène, les substituants du cycle cyclopropanique pouvant être de configuration cis ou trans, racémique ou optiquement active, la double liaison en position 1 de la chaîne latérale vinylique pouvant être de configuration (E) ou (Z), ou bien Y représente un groupement:

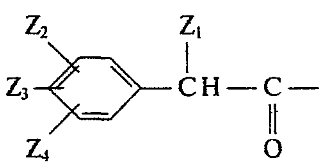

(D)

tel que défini à la revendication 1, le carbone asymétrique en position 1 du dérivé de l'alléthrolone pouvant être de configuration R, S ou racémique.

3. Les composés de formule générale I; tels que définis à la revendication 2, dans lesquels Y représente un groupement:

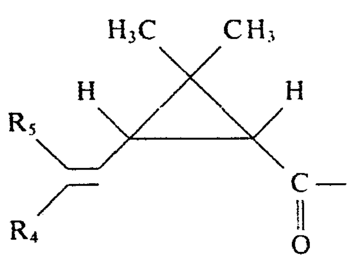

(A)

tel que défini à la revendication 2.

4. Les composés de formule générale I, tels que définis à la revendication 2, dans lesquels Y représente un groupement:

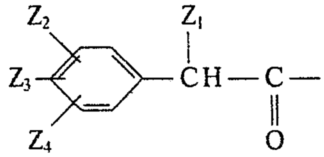

(D)

tel que défini à la revendication 2.

5. Les composés de formule générale I, tels que définis à la revendication 2 ou 3, dans lesquels Y représente un groupement A, $R_1$ et $R_2$ représentent un atome d'halogène $R_3$ et $R'_3$ représentent un atome d'hydrogène et $R_4$ et $R_5$ représentent un atome d'halogène.

6. Les composés de formule générale I; tels que définis à la revendication 2 ou 3, dans lesquels Y représente un groupement A, $R_1$, $R_2$, $R_3$ et $R'_3$ représentent de l'hydrogène et $R_4$et $R_5$, représentent avec l'atome de carbone auquel ils sont liés, un homocycle carboné.

7. Les composés de formule générale I, tels que définis à la revendication 2 ou 3, dans lesquels Y représente un groupement A, $R_1$, $R_2$, $R_3$ et $R'_3$ représentent de l'hydrogène et $R_4$ et $R_5$ représentent un atome d'halogène.

8. L'un quelconque des composés de formule générale I', dont les noms suivent:

le (1R,trans)2,2-diméthyl 3-cyclopentylidène méthyl cyclopropane 1-carboxylate de (1S) 2-méthyl 3-allyl 4-méthylène cyclopent 2-ène-1-yle,

le (1R,trans)2,2-diméthyl 3-(2,2-difluoroéthényl) cyclopropane 1-carboxylate de (1S)2-méthyl 3-allyl 4-méthylène cyclopent 2-ène-1-yle,

le (1R,trans)2,2-diméthyl 3-(2,2-dichloroéthenyl)cyclopropane 1-carboxylate de (1S)2-méthyl 3-allyl 4-méthylène cyclopent 2-ène-1-yle,

le (1R,trans)2,2-diméthyl 3-/2(Z)-cyano éthényl/ cyclopropane-1-carboxylate de (1S)2-méthyl 3-allyl 4-méthylène cyclopent-2-ène 1-yle,

le (1R,trans)2,2-diméthyl 3-/2-fluoro 2-chloro éthenyl/ cyclopropane-1-carboxylate de (1S)2-méthyl 3-allyl 4-méthylène cyclopent-2-ène-1-yle,

le (1R,trans)2,2-diméthyl 3-cyclobutylidène méthyl cyclopropane-1-carboxylate de (1S)2-méthyl 3-allyl 4-méthylène cyclopent-2-ène-1-yle et

le (1R,cis)2,2-diméthyl 3-éthynyl cyclopropane-1-carboxylate de (1S)2-méthyl 3-allyl 4-méthylène cyclopent-2-ène-1-yle.

9. Procédé de préparation des composés de formule générale (I') tels que définis à la revendication 1, caractérisé en ce que l'on fait réagir un composé de formule générale II, de configuration (S), (R) ou racémique:

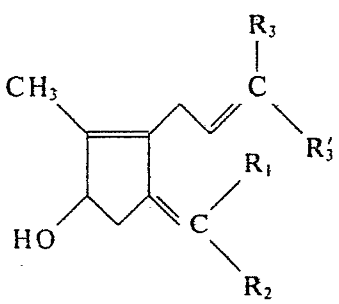

(II)

dans laquelle $R_1$, $R_2$, $R_3$, et $R'_3$ conservent les significations de la précitées, ou un dérivé fonctionnel de ce composé (II), avec un acide carboxylique de formule générale (III):

$$Y'-OH \qquad (III)$$

dans laquelle Y' conserve la signification de la revendication 1, ou avec un dérivé fonctionnel de cet acide (III).

10. Procédé de préparation des composés de formule générale (I') tels que définis à la revendication 1, caractérisé en ce que l'on soumet un composé de formule générale IV:

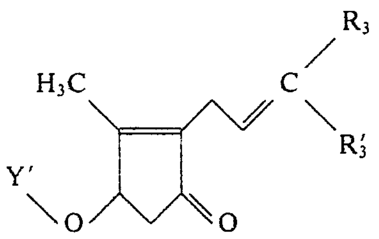

(IV)

dans laquelle $R_3$, $R'_3$ et Y' conservent les significations de revendication 1, à l'action d'un réactif de formule:

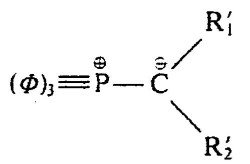

dans laquelle $R'_1$ et $R'_2$ conservent les signisfications de la revendication 1, puis, le cas échéant fait réagir, lorsque $R'_1$ et/ou $R'_2$ représentent un alcoyloxycarbonyle, le composé I' correspondant, comportant un ou deux groupements esters, avec de l'ammoniac, pour obtenir le composé de formule I' correspondant, comportant un ou deux groupements carbomoyles.

11. Compositions insecticides, caractérisées en ce qu'elles contiennent comme matière active un au moins des composés de formule générale (I') telle que définie à la revendication 1.

12. Compositions insecticides telles que définies à la revendication 11, caractérisées en ce qu'elles contiennent, outre le ou les principes actifs, au moins un agent synergisant.

13. Compositions insecticides selon la revendication 11 ou 12, caractérisées en ce qu'elles contiennent comme matière active au moins un des composés de formule générale (I), telle que définie à la revendication 2.

14. Compositions insecticides selon la revendication 11 ou 12, caractérisées en ce qu'elles contiennent comme matière active au moins un des composés définis à l'une quelconque des revendications 3, 4, 5, 6 ou 7.

15. Compositions insecticides selon la revendication 11 ou 12, caractérisées en ce qu'elles contiennent comme matière active au moins un des composés définis à la revendication 8.

16. Compositions acaricides, caractérisées en ce qu'elles contiennent comme matière active, un au moins des composés de formule générale (I') telle que définie à la revendication 1.

17. Compositions nématicides, caractérisées en ce qu'elles contiennent, comme matière active, un au moins des composés de formule générale (I'), telle que définie à la revendication 1.

18. Compositions antifongiques, caractérisées en ce qu'elles contiennent, comme matière active, un au moins des composés de formule générale (I'), telle que définie à la revendication 1.

19. Compositions pharmaceutiques à usage vétérinaire utilisées dans la lutte contre les affections provoquées par les acariens, caractérisées en ce qu'elles contiennent, comme matière active, un au moins des composés de formule générale (I'), telle que définie à la revendication 1.

20. Compositions destinées à l'alimentation animale, caractérisées en ce qu'elles sont constituées par un aliment composé, équilibré, pour animal et qu'elles renferment en outre un au moins des composés de formule générale (I'), telle que définie à la revendication 1.

21. Compositions répulsives vis-à-vis des acariens parasites des végétaux, caractérisées en ce qu'elles contiennent comme principe actif, un au moins des composés de formule générale (I') telle que définie à la revendication 1.

22. Associations douées d'activité insecticide, acaricide, fongicide ou nématicide, caractérisées en ce qûelles contiennent, comme matière active, d'une part un au moins des composés de formule générale (I'), selon la revendication 1, et d'autre part un au moins des esters pyréthrinoïdes choisis dans le groupe constitué par les esters d'alléthrolones, d'alcool 3, 4, 5, 6-tétrahydrophtalimido méthylique, d'alcool 5-benzyle 3-furyle méthylique, d'alcool 3-phénoxy benzylique et d'alcools$\alpha$-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidèneméthyl) cyclopropane-1-carboxyliques, par les esters d'alcool 3-phénoxy benzylique et d'alcool$\alpha$-cyano 3-phénoxy benzyliques des acides 2,2-diméthyl 3-(2,2-dichlorovinyl) cyclopropane-1-carboxyliques, par les esters d'alcool$\alpha$-cyano 3-phénoxy benzylique d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3, 4, 5, 6-tétrahydrophtalimidométhylique, d'alcool 5-benzyl 3-furyl méthylique, d'alcool 3-phénoxy benzylique et d'alcool$\alpha$-cyano 3-phénoxy benzyliques des acides 2,2-diméthyl 3-(1, 2, 2, 2-tétrahalo) cyclopropane-1-carboxyliques, dans lesquels »halo« représente un atome de fuor, de chlore ou de brome, étant entendu que les composés I' peuvent exister sous toutes leurs formes stéréoisomères possibles, de même que les copules acides et alcools des esters pyréthrinoïdes ci-dessus.

**Revendications pour l'Etat contractant: AT**

1. Procédé de préparation des composés, sous toutes leurs formes isomères possibles, de formule générale (I'):

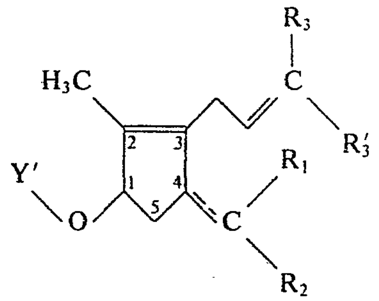

(I')

dans laquelle $R_1$ et $R_2$, identiques ou différents, représentent un groupement carbomoyle ou bien des groupements $R'_1$ et $R'_2$, lesquels, identiques ou différents, représentent un atome d'hydrogène, un atome d'halogène, un radical alcoyle comportant de 1 à 6 atomes de carbone, un radical aryle comportant de 6 à 10 atomes de carbone, un radical aralcoyle comportant de 7 à 13 atomes de carbone, un groupement alcoyloxycarbonyle comportant de 2 à 5 atomes de carbone ou un groupement cyano, $R_3$ et $R'_3$, identiques ou différents, représentent un atome d'hydrogène, un halogène ou un radical alcoyle saturé ou insaturé, comportant de 1 à 3 atomes de carbone, Y' représente ou bien un groupement:

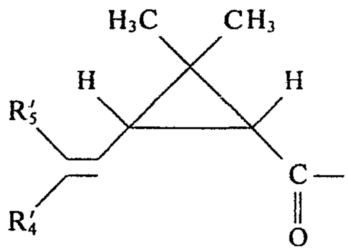

(A')

dans lequel soit $R'_4$ et $R'_5$, identiques ou différents, représentent un atome d'hydrogène, un radical alcoyle comportant de 1 à 4 atomes de carbone, un atome de fluor, de chlore ou de brome, soit $R'_4$ et $R'_5$ représentent ensemble avec l'atome de carbone auquel ils sont liés, un homocycle carboné ou un hétérocycle, comportant de 3 à 7 chaînons, ces cycles pouvant être éventuellement insaturés, ou bien un hétérocycle de structure

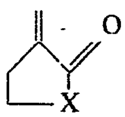

dans lequel X représente un atome d'oxygène ou de soufre, soit l'un des groupes $R'_4$ ou $R'_5$ représente un groupement cyano, un groupement

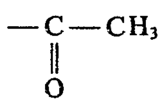 un groupement 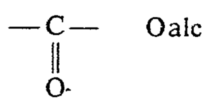

alc représentant un radical alcoyle renfermant de 1 à 4 atomes de carbone, lorsque l'autre représente un atome d'hydrogène, soit $R'_4$ représente un groupement cyano et $R'_5$ un radical phényle, les substituants du cycle cyclopropanique pouvant être de configuration cis ou trans, racémique ou optiquement active, la double liaison en position 1 de la chaîne latérale vinylique pouvant être de configuration (E) ou (Z), ou bien Y' représente un groupement:

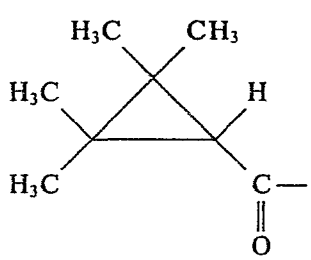

(B)

ou bien Y' représente un groupement:

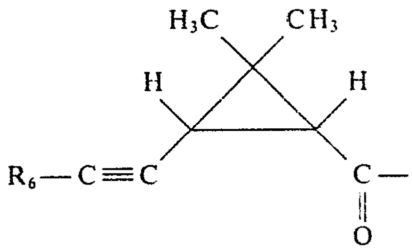

(C)

dans lequel $R_6$ représente un atome d'hydrogène, un radical alcoyle comportant de 1 à 4 atomes de carbone ou un groupement alcoxycarbonyle comportant de 2 à 5 atomes de carbone, les substituants du cycle cyclopropanique pouvant être de configuration cis ou trans, racémique ou optiquement active, ou bien Y' représente un groupement:

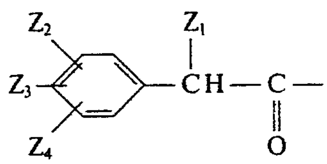

(D)

dans lequel $Z_1$ représente un radical alcoyle comportant de 1 à 4 atomes de carbone, $Z_2$, $Z_3$ et $Z_4$, identiques ou différents, représentent un atome d'hydrogène, un radical comportant de 1 à 4 atomes de carbone, un radical alcoyloxyle comportant de 1 à 4 atomes de carbone, un radical alcoyl thio comportant de 1 à 4 atomes de carbone, ou un atome d'halogène, le carbone asymétrique du groupement (D) pouvant être de configuration S, R ou racémique et le carbone asymétrique en position 1 du dérivé de l'aléthrolone pouvant être de configuration R, S ou racémique:

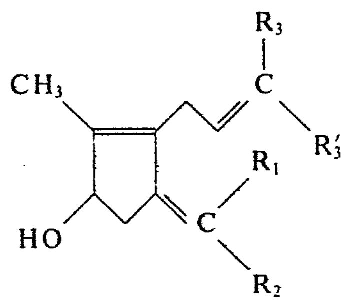

(II)

dans laquelle $R_1$, $R_2$, $R_3$ et $R'_3$ conservent les significations précitées, ou un dérivé fonctionnel de ce composé (II), avec un acide carboxylique de formule générale (III):

$$Y'—OH \qquad (III)$$

dans laquelle Y' conserve la signification précitée ou avec un dérivé fonctionnel de cet acide (III).

2. Variante du procédé de préparation des composés de formule générale (I'), tel que défini à la revendication 1, caractérisée en ce que l'on soumet un composé de formule générale IV:

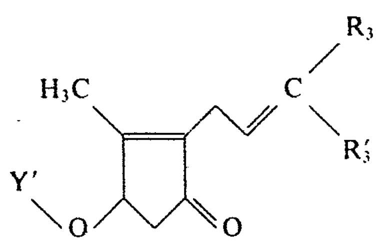

(IV)

dans laquelle $R_3$, $R'_3$ et Y' conservent les significations de la revendication 1, à l'action d'un réactif de formule:

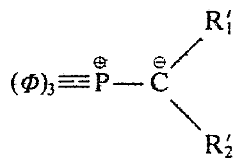

dans laquelle R'$_1$ et R'$_2$ conservent les significations de la revendication 1, puis, le cas échéant fait réagir, lorsque R'$_1$ et/ou R'$_2$ représentent un alcoyloxycarbonyle, le composé I' correspondant, comportant un ou deux groupements esters, avec de l'ammoniac, pour obtenir le composé de formule I' correspondant, comportant un ou deux groupements carbamoyles.

3. Procédé selon la revendication 1 ou 2, pour la préparation des composés, sous toutes leurs formes isomères possibles, de formule générale (I):

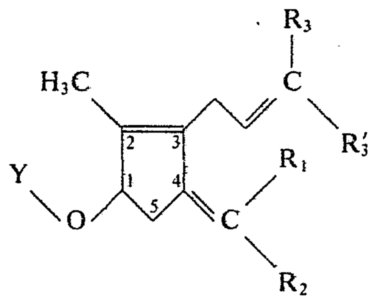

dans laquelle $R_1$, $R_2$, $R_3$ et $R'_3$ sont définis comme à la revendication 1, et Y représente ou bien un groupement:

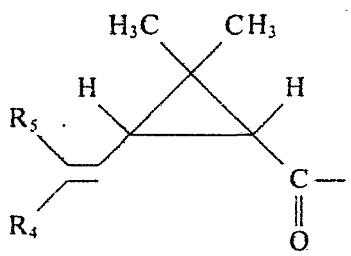

(A)

dans lequel soit $R_4$ et $R_5$, identiques ou différents, représentent un atome d'hydrogène, un radical alcoyle comportant de 1 à 4 atomes de carbone, un atome de fluor, de chlore ou de brome, soit $R_4$ et $R_5$ représentent ensemble avec l'atome de carbone auquel ils sont liés, un homocycle carboné ou un hétérocycle comportant de 3 à 7 chaînons, ces cycles pouvant être éventuellement insaturés, ou bien un hétérocycle de structure:

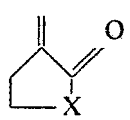

dans lequel X représente un atome d'oxygène ou de soufre, soit l'un des groupes $R_4$ ou $R_5$ représente un groupement cyano, un groupement

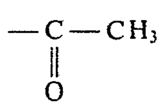 un groupement 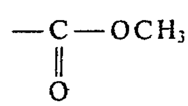

lorsque l'autre représente un atome d'hydrogène, les substituants du cycle cyclopropanique pouvant être de configuration cis ou trans, racémique ou optiquement active, la double liaison en position 1 de la chaîne latérale vinylique pouvant être de configuration (E) ou (Z), ou bien Y représente un groupement:

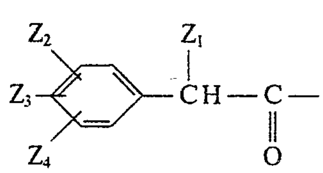

(D)

tel que défini à la revendication 1, le carbone asymétrique en position 1 du dérivé de l'alléthrolone pouvant être de configuration R, S ou racémique, caractérisé en ce que l'on utilise au départ un composé de formule III ou de formule IV, dans laquelle Y' représente un groupement Y, tel que défini ci-dessus.

4. Procédé selon la revendication 3, caractérisé en ce que Y représente un groupement:

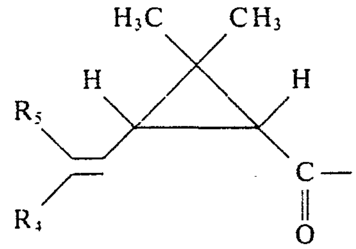

(A)

tel que défini à la revendication 3.

5. Procédé selon la revendication 3, caractérisé en ce que Y représente un groupement:

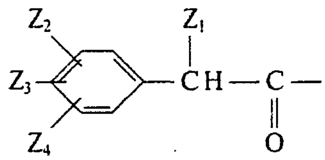

(D)

tel que défini à la revendication 3.

6. Procédé selon la revendication 4, caractérisé en ce que dans le groupement A, $R_4$ et $R_5$ représentent un atome d'halogène et en ce que dans le reste alcool du composé de formule IV ou l'alcool de formule II, $R_1$ et $R_2$ représentent un atome d'halogène et $R_3$ et $R'_3$ représentent un atome d'hydrogène.

7. Procédé selon la revendication 4, caractérisé en ce que dans le groupement A, $R_4$ et $R_5$ représentent ensemble avec l'atome de carbone auquel ils sont liés, un homocycle carboné et en ce que dans le reste alcool du composé de formule IV ou l'alcool de formule II, $R_1$, $R_2$, $R_3$ et $R'_3$ représentent un atome d'hydrogène.

8. Procédé selon la revendication 4, caractérisé en ce que dans le groupement A, $R_4$ et $R_5$ représentent un atome d'halogène et en ce que dans le reste alcool du composé de formule IV ou dans l'alcool de formule II, $R_1$, $R_2$, $R_3$ et $R'_3$ représentent un atome d'hydrogène.

9. Procédé selon la revendication 3, caractérisé en ce que les produits de départ sont choisis de manière telle que l'on prépare l'un quelconque des composés de formule générale I dont les noms suivent:

le (1R,trans)2,2-diméthyl 3-cyclopentylidène méthyl cyclopropane 1-carboxylate de (1S)-2-méthyl 3-allyl 4-méthylène cyclopent 2-ène-1-yle,

le (1R,trans)2,2-diméthyl 3-(2,2-difluoroéthènyl) cyclopropane 1-carboxylate de (1S)2-méthyl 3-allyl 4-méthylène cyclopent 2-ène-1-yle,

le (1R,trans)2,2-diméthyl 3-(2,2-dichloroéthenyl)cyclopropane 1-carboxylate de (1S)2-méthyl 3-allyl 4-méthylène cyclopent 2-ène-1-yle,

le (1R,trans)2,2-diméthyl 3-/2(Z)-cyano éthényl/ cyclopropane-1-carboxylate de (1S)2-méthyl 3-allyl 4-méthylène cyclopent-2-ène 1-yle,

le (1R,trans)2,2-diméthyl 3-/2-fluoro 2-chloro éthenyl/ cyclopropane-1-carboxylate de (1S)2-méthyl 3-allyl 4-méthylène cyclopent-2-ène-1-yle,

le (1R,trans)2,2-diméthyl 3-cyclobutylidène méthyl cyclopropane-1-carboxylate de (1S)2-méthyl 3-allyl 4-méthylène cyclopent-2-ène-1-yle.

10. Procédé selon la revendication 1 ou 2, caractérisé en ce que les produits de départ sont choisis de manière telle que l'on prépare le (1R,cis)2,2-diméthyl 3-éthynyl cyclopropane-1-carboxylate de (1S)2-méthyl 3-allyl 4-méthylene cyclopent-2-èn 1-yle.

11. Compositions insecticides, caractérisées en ce qu'elles contiennent comme matière active un au moins des composés de formule générale (I') telle que définie à la revendication 1.

12. Compositions insecticides telles que définies à la revendication 11, caractérisées en ce qu'elles contiennent, outre le ou les principes actifs, au moins un agent synergisant.

13. Compositions insecticides selon la revendication 11 ou 12, caractérisées en ce qu'elles contiennent comme matière active au moins un des composés de formule générale (I), telle que définie à la revendication 3.

14. Compositions insecticides selon la revandication 11 ou 12, caractérisées en ce qu'elles contiennent comme matière active au moins un des composés définis à l'une quelconque des revendications 4, 5, 6, 7 ou 8.

15. Compositions insecticides selon la revendication 11 ou 12, caractérisées en ce qu'elles contiennent comme matière active au moins un des composés définis à la revendication 9.

16. Compositions insecticides selon la revendication 11 ou 12, caractérisées en ce qu'elles contiennent comme matière active le (1R,cis)2,2-diméthyl 3-éthynyl cyclopropane-1-carboxylate de (1S)2-méthyl 3-allyl 4-méthylene cyclopent-2-èn 1-yle.

17. Compositions acaricides, caractérisées en ce qu'elles contiennent comme matière active, un au moins des composés de formule générale (I') telle que définie à la revendication 1.

18. Compositions nématicides, caractérisées en ce qu'elles contiennent, comme matière active, un au moins des composés de formule générale (I'), telle que définie à la revendication 1.

19. Compositions antifongiques, caractérisées en ce qúelles contiennent, comme matière active, un au moins des composés de formule générale (I'), telle que définie à la revendication 1.

20. Compositions répulsives vis-à-vis des acariens parasites des végétaux, caractérisées en ce qu'elles contiennent comme principe actif, un au moins des composés de formule générale (I') telle que définie à la revendication 1.

21. Compositions douées d'activité insecticide, acaricide, fongicide ou nématicide, caractérisées en ce qu'elles contiennent, comme matière active, d'une part un au moins des composés de formule générale (I') selon la revendication 1, et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phenoxy benzylique et d'alcools $\alpha$-cyano 3-phénoxy benzyliques des acides chrysanthémiques, par les esters d'alcool 5-benzyl 3-furyl méthylique des acides 2,2-diméthyl 3-(2-oxo-3-tétrahydrothiophénylidèneméthyl)cyclopropane-1-carboxyliques, par les esters d'alcool 3-phénoxy benzylique et d'alcools $\alpha$-cyano 3-phénoxy benzyliques des acides 2,2-diméthyl 3-(2,2-dichlorovinyl)cyclopropane-1-carboxyliques, par les esters d'alcools $\alpha$-cyano 3-phénoxy benzylique d'acides 2,2-diméthyl 3-(2,2-dibromovinyl)cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcools $\alpha$-cyano 3-phénoxy benzyliques des acides 2,2-diméthyl 3-(1,2,2,2-tétrahalo)cyclopropane-1-carboxyliques, dans lesquels »halo« représente un atome de fluor, de chlore ou de brome, étant entendu que les composés I' peuvent exister sous toutes leurs formes stéréoisomères possibles, de même que les copules acides et alcools des esters pyréthrinoïdes ci-dessus.

22. Compositions utilisées dans la lutte contre les affections provoquées par les acariens parasites des animaux, caractérisées en ce qu'elles contiennent, comme matière active, un au moins des composés de formule générale (I') telle que définie à la revendication 1.

23. Utilisation des composés de formule générale (I') telle que définie à la revendication 1, à titre d'insecticides, d'acaricides, de nématicides et d'antifongiques.

24. Utilisation des composés de formule générale (I') telle que définie à la revendication 1, dans la lutte contre les affections provoquées par les acariens parasites des animaux.

**Patentansprüche für die Vertragsstaaten: BE, CH/LI, DE, FR, GB, IT, LU, NL, SE**

1. In sämtlichen ihrer möglichen isomeren Formen die Verbindungen der allgemeinen Formel I'

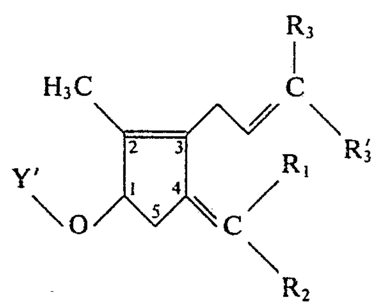

(I')

worin $R_1$ und $R_2$, die gleich oder verschieden sein können, eine Carbamoylgruppe oder Gruppen $R'_1$ und $R'_2$, die gleich oder verschieden sein können und ein Wasserstoffatom, ein Halogenatom, einen Alkylrest mit 1 bis 6 Kohlenstoffatomen, einen Arylrest mit 6 bis 10 Kohlenstoffatomen, einen Aralkylrest mit 7 bis 13 Kohlenstoffatomen, eine Alkoxycarbonylgruppe mit 2 bis 5 Kohlenstoffatomen oder eine Cyanogruppe darstellen, bedeuten, $R_3$ und $R'_3$, die gleich oder verschieden sein können, ein Wasserstoffatom, ein Halogenatom oder einen gesättigten oder ungesättigten Alkylrest mit 1 bis 3 Kohlenstoffatomen bedeuten, Y' entweder eine Gruppe

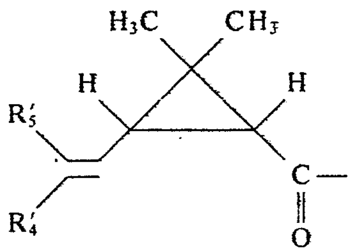

(A')

worin $R'_4$ und $R'_5$, die gleich oder verschieden sein können, ein Wasserstoffatom, einen Alkylrest mit 1 bis 4 Kohlenstoffatomen, ein Fluor-, Chlor- oder Bromatom bedeuten oder $R'_4$ und $R'_5$ gemeinsam mit dem Kohlenstoffatom, an das sie gebunden sind, einen Kohlenstoffhomocyclus oder einen Heterocyclus mit 3 bis 7 Kettengliedern, wobei diese Cyclen gegebenenfalls ungesättigt sein können, oder einen Heterocyclus der Struktur

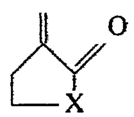

bilden, worin X ein Sauerstoff- oder Schwefelatom bedeutet oder eine Gruppe $R'_4$ oder $R'_5$ eine Cyanogruppe, eine Gruppe

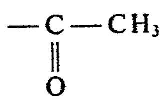 eine Gruppe 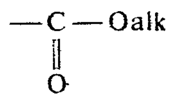

worin alk einen Alkylrest mit 1 bis 4 Kohlenstoffatomen bedeutet, wenn die andere ein Wasserstoffatom darstellt, ist oder $R'_4$ eine Cyanogruppe bedeutet und $R'_5$ einen Phenylrest bedeutet, wobei die Substituenten des Cyclopropanringes racemische oder optisch aktive cis- oder trans-Konfiguration aufweisen können, wobei die Doppelbindung in 1-Stellung der Vinylseitenkette (E)- oder (Z)-Konfiguration besitzen kann, bedeutet oder Y' eine Gruppe

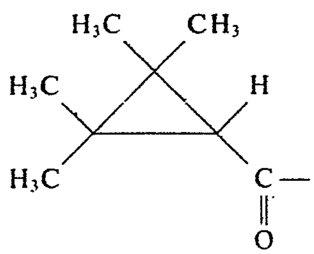

(B)

darstellt oder Y' eine Gruppe

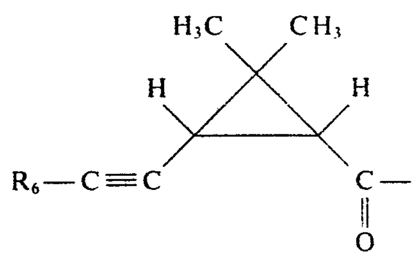

(C)

darstellt, worin $R_6$ ein Wasserstoffatom, einen Alkylrest mit 1 bis 4 Kohlenstoffatomen oder eine Alkoxycarbonylgruppe mit 2 bis 5 Kohlenstoffatomen bedeutet, wobei die Substituenten des Cyclopropanrings racemische oder optisch aktive cis- oder trans-Konfiguration aufweisen können, oder Y' eine Gruppe

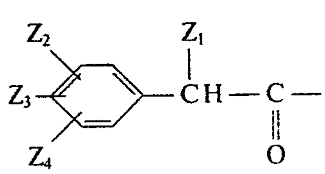

(D)

darstellt, worin $Z_1$ einen Alkylrest mit 1 bis 4 Kohlenstoffatomen bedeutet, $Z_2$, $Z_3$ und $Z_4$, die gleich oder verschieden sein können, ein Wasserstoffatom, einen Alkylrest mit 1 bis 4 Kohlenstoffatomen, einen Alkoxyrest mit 1 bis 4 Kohlenstoffatomen, einen Alkylthiorest mit 1 bis 4 Kohlenstoffatomen oder ein Halogenatom bedeuten, wobei das asymmetrische Kohlenstoffatom der Gruppe D gegebenenfalls S-, R- oder racemische Konfiguration besitzen kann und das asymmetrische Kohlenstoffatom in 1-Stellung des Allethrolonderivats R-, S- oder racemische Konfiguration aufweisen kann.

2. In sämtlichen ihrer möglichen isomeren Formen die Verbindung gemäß Anspruch 1 der allgemeinen Formel I

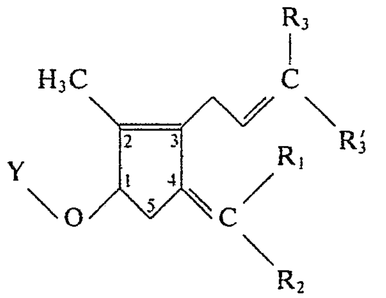

worin $R_1$, $R_2$, $R_3$ und $R'_3$ wie in Anspruch 1 definiert sind und Y entweder eine Gruppe

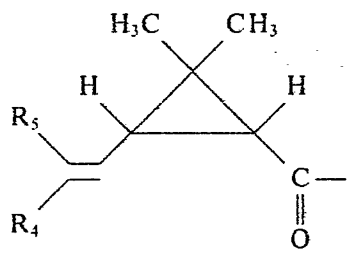

(A)

bedeutet, worin entweder $R_4$ und $R_5$, die gleich oder verschieden sein können, ein Wasserstoffatom, einen Alkylrest mit 1 bis 4 Kohlenstoffatomen, ein Fluor-, Chlor- oder Bromatom bedeuten oder $R_4$ und $R_5$ gemeinsam mit dem Kohlenstoffatom, an das sie gebunden sind, einen Kohlenstoffhomocyclus oder einen Heterocyclus mit 3 bis 7 Kettengliedern, wobei diese Cyclen gegebenenfalls ungesättigt sein können, oder einen Heterocyclus der Struktur

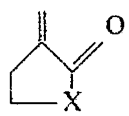

worin X ein Sauerstoff- oder Schwefelatom bedeutet, bilden oder eine der Gruppen $R_4$ oder $R_5$ eine Cyanogruppe, eine Gruppe

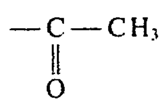 eine Gruppe 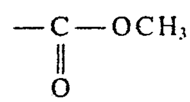

bedeutet, wenn die andere ein Wasserstoff darstellt, wobei die Substituenten des Cyclopropanringes racemische oder optisch aktive cis- oder trans-Konfiguration besitzen können, wobei die Doppelbindung in 1-Stellung der Vinylseitenkette (E)- oder (Z)-Konfiguration aufweisen kann oder Y eine Gruppe

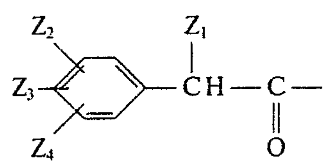

(D)

wie in Anspruch 1 definiert, bedeutet, wobei das asymmetrische Kohlenstoffatom in 1-Stellung des Allethrolonderivats R-, S- oder racemische Konfiguration besitzen kann.

3. Die Verbindungen der allgemeinen Formel 1 gemäß Anspruch 2, worin Y eine Gruppe

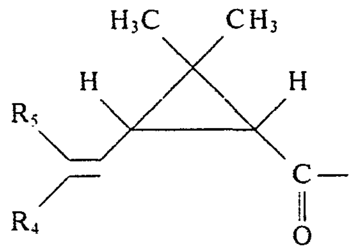

(A)

wie in Anspruch 2 definiert, bedeutet.

4. Die Verbindungen der allgemeinen Formel I gemäß Anspruch 2, worin Y eine Gruppe

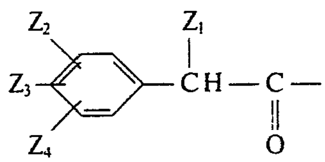

(D)

wie in Anspruch 2 definiert bedeutet.

5. Die Verbindungen der allgemeinen Formel I gemäß Anspruch 2 oder 3, worin Y eine Gruppe A bedeutet, $R_1$ und $R_2$ ein Halogenatom darstellen, $R_3$ und $R'_3$ ein Wasserstoffatom bedeuten und $R_4$ und $R_5$ ein Halogenatom bedeuten.

6. Die Verbindungen der allgemeinen Formel I gemäß Anspruch 2 oder 3, worin Y eine Gruppe A bedeutet, $R_1$, $R_2$, $R_3$ und $R'_3$ Wasserstoff bedeuten und $R_4$ und $R_5$ gemeinsam mit dem Kohlenstoffatom, an das sie gebunden sind, einen Kohlenstoffhomocyclus bilden.

7. Die Verbindungen der allgemeinen Formel I gemäß Anspruch 2 oder 3, worin Y eine Gruppe A bedeutet, $R_1$, $R_2$, $R_3$ und $R'_3$ Wasserstoff bedeuten und $R_4$ und $R_5$ ein Halogenatom bedeuten.

8. Eine der Verbindungen der allgemeinen Formel I' mit den folgenden Bezeichnungen:

(1S)-2-Methyl-3-allyl-4-methylencyclopent-2-en-1-yl-(1R,trans)-2,2-dimethyl-3-cyclopentylidenmethylcyclopropan-1-carboxylat,

(1S)-2-Methyl-3-allyl-4-methylencyclopent-2-en-1-yl-(1R,trans)-2,2-dimethyl-3-(2,2-difluoräthenyl)-cyclopropan-1-carboxylat,

(1S)-2-Methyl-3-allyl-4-methylencyclopent-2-en-1-yl-(1R,trans)-2,2-dimethyl-3-(2,2-dichloräthenyl)-cyclopropan-1-carboxylat,

(1S)-2-Methyl-3-allyl-4-methylencyclopent-2-en-1-yl-(1R,trans)-2,2-dimethyl-3-[2-(Z)-cyanoäthenyl]-cyclopropan-1-carboxylat,

(1S)-2-Methyl-3-allyl-4-methylencyclopent-2-en-1-yl-(1R,trans)-2,2-dimethyl-3-(2-fluor-2-chloräthenyl)-cyclopropan-1-carboxylat,

(1S)-2-Methyl-3-allyl-4-methylencyclopent-2-en-1-yl-(1R,trans)-2,2-dimethyl-3-cyclobutylidenmethylcyclopropan-1-carboxylat und

(1S)-2-Methyl-3-allyl-4-methylencyclopent-2-en-1-yl-(1R,cis)-2,2-dimethyl-3-äthinylcyclopropan-1-carboxylat.

9. Verfahren zur Herstellung der Verbindungen der allgemeinen Formel I' gemäß Anspruch 1, dadurch gekennzeichnet, daß man eine Verbindung der allgemeinen Formel II mit S-, R- oder racemischer Konfiguration

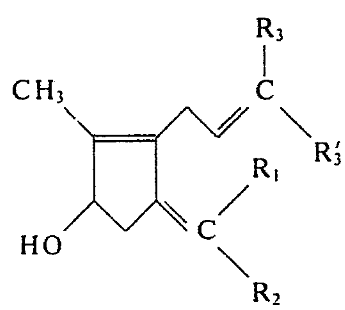

(II)

worin $R_1$, $R_2$, $R_3$, und $R'_3$ die in Anspruch 1 angegebenen Bedeutungen besitzen oder ein funktionelles Derivat dieser Verbindung II mit einer Carbonsäure der allgemeinen Formel III

$$Y'—OH \qquad \text{(III)}$$

worin Y' die in Anspruch 1 angegebene Bedeutung besitzt oder mit einem funtionellen Derivat dieser Säure III umsetzt.

10. Verfahren zur Herstellung der Verbindungen der allgemeinen Formel I' gemäß Anspruch 1, dadurch gekennzeichnet, daß man eine Verbindung der allgemeinen Formel IV

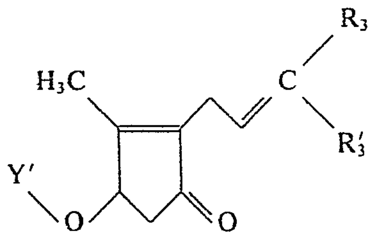

(IV)

worin $R_3$, $R'_3$ et Y' die in Anspruch 1 angegebene Bedeutung besitzen, der Einwirkung eines Reagens der Formel

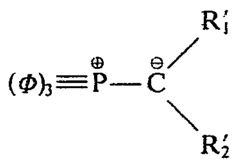

worin $R'_1$ und $R'_2$ die in Anspruch 1 angegebenen Bedeutungen besitzen, unterzieht und danach gegebenenfalls, wenn $R'_1$ und/oder $R'_2$ eine Alkoxycarbonylgruppe bedeuten, die entsprechende Verbindung I', die ein oder zwei Estergruppen enthält, mit Ammoniak umsetzt, um die entsprechende Verbindung I' zu erhalten, die ein oder zwei Carbamoylgruppen enthält.

11. Insektizide Zusammensetzungen, dadurch gekennzeichnet, daß sie als Wirkstoff zumindest eine der Verbindungen der allgemeinen Formel I' gemäß Anspruch 1 enthalten.

12. Insektizide Zusammensetzungen gemäß Anspruch 11, dadurch gekennzeichnet, daß sie außer dem oder den Wirkstoffen zumindest ein synergisierendes Mittel enthalten.

13. Insektizide Zusammensetzungen gemäß Anspruch 11 oder 12, dadurch gekennzeichnet, daß sie als Wirkstoff zumindest eine der Verbindungen der allgemeinen Formel I gemäß Anspruch 2 enthalten.

14. Insektizide Zusammensetzungen gemäß Anspruch 11 oder 12, dadurch gekennzeichnet, daß sie als Wirkstoff zumindest eine der gemäß einem der Ansprüche 3, 4, 5, 6 oder 7 definierten Verbindungen enthalten.

15. Insektizide Zusammensetzungen gemäß Anspruch 11 oder 12, dadurch gekennzeichnet, daß sie als Wirkstoff zumindest eine der in Anspruch 8 definierten Verbindungen enthalten.

16. Acarizide Zusammensetzungen, dadurch gekennzeichnet, daß sie als Wirkstoff zumindest eine der Verbindungen der allgemeinen Formel I' gemäß Anspruch 1 enthalten.

17. Nematizide Zusammensetzungen, dadurch gekennzeichnet, daß sie als Wirkstoff zumindest eine der Verbindungen der allgemeinen Formel I' gemäß Anspruch 1 enthalten.

18. Antifunguszusammensetzungen, dadurch gekennzeichnet, daß sie als Wirkstoff zumindest eine der Verbindungen der allgemeinen Formel I' gemäß Anspruch 1 enthalten.

19. Pharmazeutische Zusammensetzungen für die veterinärmedizinische Verwendung zur Bekämpfung von Erkrankungen, die durch Milben hervorgerufen werden, dadurch gekennzeichnet, daß sie als Wirkstoff zumindest eine der Verbindungen der allgemeinen Formel I' gemäß Anspruch 1 enthalten.

20. Zusammensetzungen für die tierische Ernährung, dadurch gekennzeichnet, daß sie aus einer der tierischen Ernährung angepaßten Nahrungsmittelzusammensetzung bestehen und daß sie außerdem zumindest eine der Verbindungen der allgemeinen Formel I' gemäß Anspruch 1 enthalten.

21. Milbenparasiten der Pflanzen abweisende Zusammensetzungen, dadurch gekennzeichnet, daß sie als Wirkstoff zumindest eine der Verbindungen der allgemeinen Formel I' gemäß Anspruch 1 enthalten.

22. Mit einer insektiziden, acariziden, fungiziden oder nematiziden Aktivität ausgestattete Assoziationen, dadurch gekennzeichnet, daß sie als Wirkstoff einesteils zumindest eine der Verbindungen der allgemeinen Formel I' gemäß Anspruch 1 enthalten und anderenteils zumindest einen der Pyrethrinoidester, ausgewählt unter den Estern der Allethrolone, des 3,4,5,6-Tetrahydrophthalimidomethylalkohols, des 5-Benzyl-3-furylmethylalkohols, des 3-Phenoxybenzylalkohols und der $\alpha$-Cyano-3-phenoxybenzylalkohole der Chrysanthemumsäuren, unter den Estern des 5-Benzyl-3-furylmethylalkohols der 2,2-Dimethyl-3-(2-oxo-3-tetrahydrothiophenylidenmethyl)-cyclopropan-1-carbonsäuren, unter den Estern des 3-Phenoxybenzylalkohols und der $\alpha$-Cyano-3-phenoxybenzylalkohole der 2,2-Dimethyl-3-(2,2-dichlorvinyl)-cyclopropan-1-carbonsäuren, unter den Estern der $\alpha$-Cyano-3-phenoxybenzylalkohole der 2,2-Dimethyl-3-(2,2-dibromvinyl)-cyclopropan-1-carbonsäuren, unter den Estern des 3-Phenoxybenzylalkohols der 2-p-Chlorphenyl-2-isopropylessigsäuren, unter den Estern der Allethrolone des 3,4,5,6-Tetrahydrophthalimidomethylalkohols, des 5-Benzyl-3-furylmethylalkohols, des 3-Phenoxybenzylalkohols und der $\alpha$-Cyano-3-phenoxybenzylalkohole der 2,2-Dimethyl-3-(1,2,2,2-tetrahaloäthyl)-cyclopropan-1-carbonsäuren, worin »halo« ein Fluor-, Chlor- oder Bromatome bedeutet, wobei sich versteht, daß die Verbindungen I' in sämtlichen ihrer möglichen stereoisomeren Formen vorliegen können, ebenso wie die Verknüpfungen der Säuren und Alkohole der vorstehenden Pyrethrinoidester.

**Patentansprüche für den Vertragsstaat: AT**

1. Verfahren zur Herstellung von Verbindungen der allgemeinen Formel I' in sämtlichen ihrer möglichen isomeren Formen

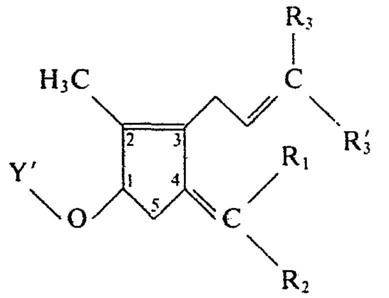

(I')

worin $R_1$ und $R_2$, die gleich oder verschieden sein können, eine Carbamoylgruppe oder Gruppen $R'_1$ und $R'_2$ bedeuten, die gleich oder verschieden sein können und ein Wasserstoffatom, ein Halogenatom, einen Alkylrest mit 1 bis 6 Kohlenstoffatomen, einen Arylrest mit 6 bis 10 Kohlenstoffatomen, einen Aralkylrest mit 7 bis 13 Kohlenstoffatomen, eine Alkoxycarbonylgruppe mit 2 bis 5 Kohlenstoffatomen oder eine Cyanogruppe bedeuten, $R_3$ und $R'_3$, die gleich oder verschieden sein können, ein Wasserstoffatom, ein Halogenatom oder einen gesättigten oder ungesättigten Alkylrest mit 1 bis 3 Kohlenstoffatomen bedeuten, Y' entweder eine Gruppe

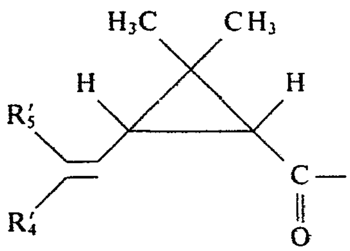

(A')

darstellt, worin $R'_4$ und $R'_5$, die gleich oder verschieden sein können, ein Wasserstoffatom, einen Alkylrest mit 1 bis 4 Kohlenstoffatomen, ein Fluor-, Chlor- oder Bromatom bedeuten oder $R'_4$ und $R'_5$ gemeinsam mit dem Kohlenstoffatom, an das sie gebunden sind, einen Kohlenstoffhomocyclus oder einen Heterocyclus mit 3 bis 7 Kettengliedern, wobei diese Cyclen gegebenenfalls ungesättigt sein können, oder einen Heterocyclus der Struktur

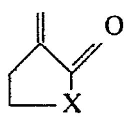

worin X ein Sauerstoff- oder Schwefelatom bedeutet, bilden oder eine der Gruppen $R'_4$ oder $R'_5$ eine Cyanogruppe, eine Gruppe

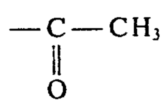 eine Gruppe 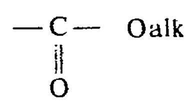

worin alk einen Alkylrest mit 1 bis 4 Kohlenstoffatomen bedeutet, darstellt, wenn die andere ein Wasserstoffatom darstellt, ist oder $R'_4$ eine Cyanogruppe bedeutet und $R'_5$ einen Phenylrest bedeutet, wobei die Substituenten des Cyclopropanringes racemische oder optisch aktive cis- oder trans-Konfiguration besitzen können, wobei die Doppelbindung in 1-Stellung der Vinylseitenkette (E)- oder (Z)-Konfiguration aufweisen kann oder Y' eine Gruppe

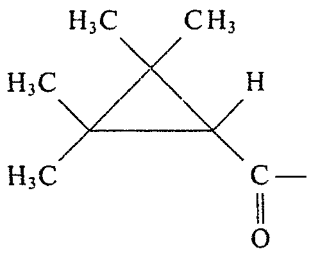

(B)

bedeutet oder Y' eine Gruppe

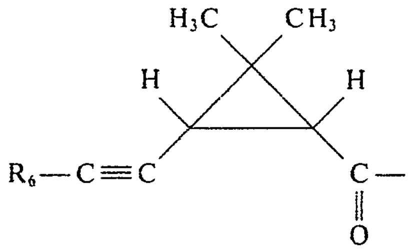

(C)

worin $R_6$ ein Wasserstoffatom, einen Alkylrest mit 1 bis 4 Kohlenstoffatomen oder eine Alkoxycarbonylgruppe mit 2 bis 5 Kohlenstoffatomen darstellt, wobei die Substituenten des Cyclopropanrings racemische oder optisch aktive cis- oder trans-Konfiguration aufweisen können oder Y' eine Gruppe

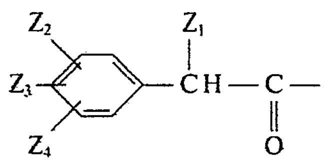

(D)

darstellt, worin $Z_1$ einen Alkylrest mit 1 bis 4 Kohlenstoffatomen bedeutet, $Z_2$, $Z_3$ und $Z_4$, die gleich oder verschieden sein können, ein Wasserstoffatom, einen Alkylrest mit 1 bis 4 Kohlenstoffatomen, einen Alkoxyrest mit 1 bis 4 Kohlenstoffatomen, einen Alkylthiorest mit 1 bis 4 Kohlenstoffatomen oder ein Halogenatom bedeuten, wobei das asymmetrische Kohlenstoffatom der Gruppe D S-, R- oder racemische Konfiguration besitzen kann und das asymmetrische Kohlenstoffatom in 1-Stellung des Allethrolonderivats R-, S- oder racemische Konfiguration aufweisen kann, dadurch gekennzeichnet, daß man eine Verbindung der allgemeinen Formel II mit S-, R- oder racemischer Konfiguration

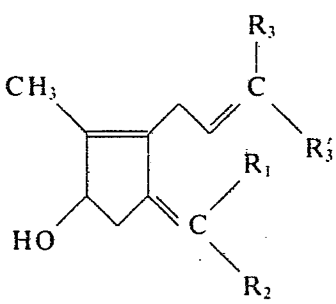

(II)

worin $R_1$, $R_2$, $R_3$, und $R'_3$ die vorstehend angegebenen Bedeutungen besitzen oder ein funktionelles Derivat dieser Verbindung II mit einer Carbonsäure der allgemeinen Formel III

$$Y'—OH \quad \text{(III)}$$

worin Y' die vorstehend angegebene Bedeutung besitzt oder mit einem funktionellen Derivat dieser Säure III umsetzt.

2. Variante des Verfahrens zur Herstellung der Verbindungen der allgemeinen Formel I' gemäß Anspruch 1, dadurch gekennzeichnet, daß man eine Verbindung der allgemeinen Formel IV

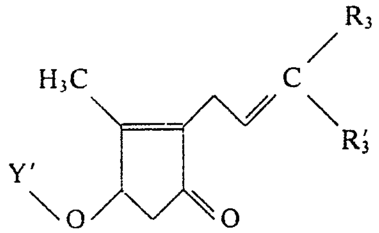

(IV)

worin $R_3$, $R'_3$ und Y' die in Anspruch 1 angegebenen Bedeutungen besitzen, der Einwirkung eines Reagens der Formel

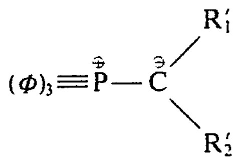

worin $R'_1$ und $R'_2$ die in Anspruch 1 angegebenen Bedeutungen besitzen, unterzieht und danach gegebenenfalls, wenn $R'_1$ und/oder $R'_2$ eine Alkoxycarbonylgruppe bedeuten, die entsprechende Verbindung I', die ein oder zwei Estergruppen enthält, mit Ammoniak umsetzt, um die entsprechende Verbindung der Formel I' zu erhalten, die ein oder zwei Carbamoylgruppen besitzt.

3. Verfahren gemäß Anspruch 1 oder 2 zur Herstellung von Verbindungen der allgemeinen Formel I in sämtlichen ihrer möglichen isomeren Formen

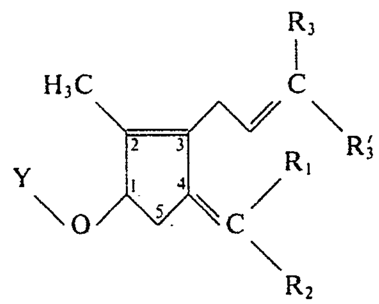

worin $R_1$, $R_2$, $R_3$ und $R'_3$ die in Anspruch 1 angegebene Bedeutung besitzen und Y entweder eine Gruppe

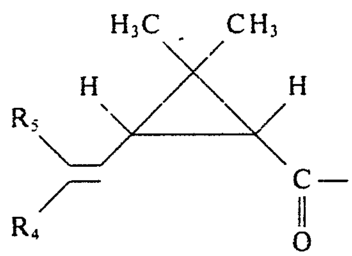

(A)

bedeutet, worin $R_4$ und $R_5$, die gleich oder verschieden sind, ein Wasserstoffatom, einen Alkylrest mit 1 bis 4 Kohlenstoffatomen, ein Fluor-, Chlor- oder Bromatom bedeuten oder $R_4$ und $R_5$ gemeinsam mit dem Kohlenstoffatom, an das sie gebunden sind, einen Kohlenstoffhomocyclus oder einen Heterocyclus mit 3 bis 7 Kettengliedern, wobei diese Cyclen gegebenenfalls ungesättigt sein können, oder einen Heterocyclus der Struktur

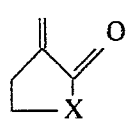

worin X ein Sauerstoff- oder Schwefelatom bedeutet, bilden oder eine der Gruppen $R_4$ oder $R_5$ eine Cyanogruppe, eine Gruppe

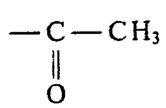 oder eine Gruppe 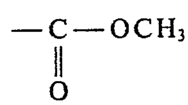

bedeutet, wenn die andere ein Wasserstoffatom darstellt, wobei die Substituenten des Cyclopropanrings racemische oder optisch aktive cis- oder trans-Konfiguration besitzen können, wobei die Doppelbindung in 1-Stellung der Vinylseitenkette (E)- oder (Z)-Konfiguration aufweisen kann oder Y eine Gruppe

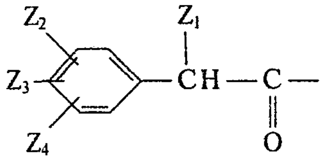

(D)

wie in Anspruch 1 definiert, bedeutet, wobei das asymmetrische Kohlenstoffatom in 1-Stellung des Allethrolonderivats R-, S- oder racemische Konfiguration aufweisen kann, dadurch gekennzeichnet, daß man zu Beginn eine Verbindung der Formel III oder der Formel IV verwendet, worin Y' eine Gruppe Y wie vorstehend definiert, bedeutet.

4. Verfahren gemäß Anspruch 3, dadurch gekennzeichnet, daß Y eine Gruppe

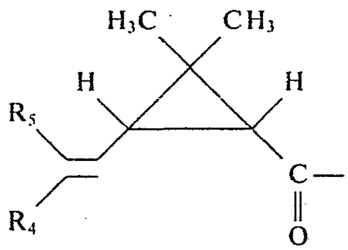

(A)

wie in Anspruch 3 definiert bedeutet.

5. Verfahren gemäß Anspruch 3, dadurch gekennzeichnet, daß Y eine Gruppe

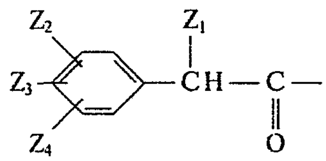

(D)

wie in Anspruch 3 definiert, bedeutet.

6. Verfahren gemäß Anspruch 4, dadurch gekennzeichnet, daß in der Gruppe A $R_4$ und $R_5$ ein Halogenatom bedeuten und daß in dem Alkoholrest der Verbindung der Formel IV oder dem Alkohol der Formel II $R_1$ und $R_2$ ein Halogenatom bedeuten und $R_3$ und $R'_3$ ein Wasserstoffatom bedeuten.

7. Verfahren gemäß Anspruch 4, dadurch gekennzeichnet, daß in der Gruppe A $R_4$ und $R_5$ gemeinsam mit dem Kohlenstoffatom, an das sie gebunden sind, einen Kohlenstoffhomocyclus bilden und daß in dem Alkoholrest der Verbindung der Formel IV oder dem Alkohol der Formel II $R_1$, $R_2$, $R_3$ und $R'_3$ ein Wasserstoffatom bedeuten.

8. Verfahren gemäß Anspruch 4, dadurch gekennzeichnet, daß in der Gruppe A $R_4$ und $R_5$ ein Halogenatom bedeuten und daß in dem Alkoholrest der Verbindung der Formel IV oder dem Alkohol der Formel II $R_1$, $R_2$, $R_3$ und $R'_3$ ein Wasserstoffatom bedeuten.

9. Verfahren gemäß Anspruch 3, dadurch gekennzeichnet, daß die Ausgangsprodukte derart ausgewählt werden, daß man eine der Verbindungen der allgemeinen Formel I mit den folgenden Bezeichnungen herstellt:

(1S)-2-Methyl-3-allyl-4-methylencyclopent-2-en-1-yl-(1R,trans)-2,2-dimethyl-3-cyclopentylidenmethylcyclopropan-1-carboxylat,

(1S)-2-Methyl-3-allyl-4-methylencyclopent-2-en-1-yl-(1R,trans)-2,2-dimethyl-3-(2,2-difluoräthenyl)-cyclopropan-1-carboxylat,

(1S)-2-Methyl-3-allyl-4-methylencyclopent-2-en-1-yl-(1R,trans)-2,2-dimethyl-3-(2,2-dichloräthenyl)-cyclopropan-1-carboxylat,

(1S)-2-Methyl-3-allyl-4-methylencyclopent-2-en-1-yl-(1R,trans)-2,2-dimethyl-3-[2-(Z)-cyanoäthenyl]-cyclopropan-1-carboxylat,

(1S)-2-Methyl-3-allyl-4-methylencyclopent-2-en-1-yl-(1R,trans)-2,2-dimethyl-3-(2-fluor-2-chloräthenyl)-cyclopropan-1-carboxylat,

(1S)-2-Methyl-3-allyl-4-methylencyclopent-2-en-1-yl-(1R,trans)-2,2-dimethyl-3-cyclobutylidenmethylcyclopropan-1-carboxylat und

(1S)-2-Methyl-3-allyl-4-methylencyclopent-2-en-1-yl-(1R,cis)-2,2-dimethyl-3-äthinylcyclopropan-1-carboxylat.

10. Verfahren gemäß Anspruch 1 oder 2, dadurch gekennzeichnet, daß die Ausgangsprodukte derart ausgewählt werden, daß man das (1S)-2-Methyl-3-allyl-4-methylencyclopent-2-en-1-yl-(1R,cis)-2,2-dimethyl-3-äthinylcyclopropan-1-carboxylat herstellt.

11. Insektizide Zusammensetzungen, dadurch gekennzeichnet, daß sie als Wirkstoff zumindest eine der Verbindungen der allgemeinen Formel I' gemäß Anspruch 1 enthalten.

12. Insektizide Zusammensetzungen gemäß Anspruch 11, dadurch gekennzeichnet, daß sie außer dem oder den Wirkstoffen zumindest ein synergisierendes Mittel enthalten.

13. Insektizide Zusammensetzungen gemäß Anspruch 11 oder 12, dadurch gekennzeichnet, daß sie als Wirkstoff zumindest eine der Verbindungen der allgemeinen Formel I gemäß Anspruch 3 enthalten.

14. Insektizide Zusammensetzungen gemäß Anspruch 11 oder 12, dadurch gekennzeichnet, daß sie als Wirkstoff zumindest eine der gemäß einem der Ansprüche 4, 5, 6, 7 oder 8 definierten Verbindungen enthalten.

15. Insektizide Zusammensetzungen gemäß Anspruch 11 oder 12, dadurch gekennzeichnet, daß sie als Wirkstoff zumindest eine der in Anspruch 9 definierten Verbindungen enthalten.

16. Insektizide Zusammensetzungen gemäß Anspruch 11 oder 12, dadurch gekennzeichnet, daß sie als Wirkstoff (1S)-2-Methyl-3-allyl-4-methylencyclopent-2-en-1-yl-(1R,cis)-2,2-dimethyl-3-äthinylcyclopropan-1-carboxylat enthalten.

17. Acarizide Zusammensetzungen, dadurch gekennzeichnet, daß sie als Wirkstoff zumindest eine der Verbindungen der allgemeinen Formel I' gemäß Anspruch 1 enthalten.

18. Nematizide Zusammensetzungen, dadurch gekennzeichnet, daß sie als Wirkstoff zumindest eine der Verbindungen der allgemeinen Formel I' gemäß Anspruch 1 enthalten.

19. Antifungus-Zusammensetzungen, dadurch gekennzeichnet, daß sie als Wirkstoff zumindest eine der Verbindungen der allgemeinen Formel I' gemäß Anspruch 1 enthalten.

20. Gegenüber Milbenparasiten der Pflanzen abweisende Zusammensetzungen, dadurch gekennzeichnet, daß sie als Wirkstoff zumindest eine der Verbindungen der allgemeinen Formel I' gemäß Anspruch 1 enthalten.

21. Mit insektizider, acarizider, fungizider oder nematizider Aktivität ausgestattete Zusammensetzungen, dadurch gekennzeichnet, daß sie als Wirkstoff einesteils zumindest eine der Verbindungen der allgemeinen Formel I' gemäß Anspruch 1 und anderenteils zumindest einen der Pyrethrinoidester enthalten, ausgewählt unter den Estern der Allethrolone, des 3,4,5,6-Tetrahydrophthalimidomethylalkohols, des 5-Benzyl-3-furylmethylalkohols, des 3-Phenoxybenzylalkohols und der $\alpha$-Cyano-3-phenoxybenzylalkohole der Chrysanthemumsäuren, unter den Estern des 5-Benzyl-3-furylmethylalkohols der 2,2-Dimethyl-3-(2-oxo-3-tetrahydrothiophenylidenmethyl)-cyclopropan-1-carbonsäuren, unter den Estern des 3-Phenoxybenzylalkohols und der $\alpha$-Cyano-3-phenoxybenzylalkohole der 2,2-Dimethyl-3-(2,2-dichlorvinyl)-cyclopropan-1-carbonsäuren, unter den Estern der $\alpha$-Cyano-3-phenoxybenzylalkohole der 2,2-Dimethyl-3-(2,2-dibromvinyl)-cyclopropan-1-carbonsäuren, unter den Estern des 3-Phenoxybenzylalkohols der 2-p-Chlorphenyl-2-isopropylessigsäuren, unter den Estern der Allethrolone des 3,4,5,6-Tetrahydrophthalimidomethylalkohols, des 5-Benzyl-3-furylmethylalkohols, des 3-Phenoxybenzylalkohols und der $\alpha$-Cyano-3-phenoxybenzylalkohole der 2,2-Dimethyl-3-(1,2,2,2-tetrahaloäthyl)-cyclopropan-1-carbonsäuren, worin »halo« ein Fluor-, Chlor- oder Bromatome bedeutet, wobei sich versteht, daß die Verbindungen I' in sämtlichen ihrer möglichen stereoisomeren Formen vorliegen können, ebenso wie die Verknüpfungen der Säuren und Alkohole der vorstehenden Pyrethrinoidester.

22. Für die Bekämpfung von Erkrankungen, die von Milbenparasiten der Tiere hervorgerufen werden, verwendete Zusammensetzungen, dadurch gekennzeichnet, daß sie als Wirkstoff zumindest eine der Verbindungen der Formel I' gemäß Anspruch 1 enthalten.

23. Verwendung der Verbindungen der allgemeinen Formel I' gemäß Anspruch 1 als insektizide, acarizide, nematizide und Antifungusmittel.

24. Verwendung der Verbindungen der allgemeinen Formel I' gemäß Anspruch 1 bei der Bekämpfung von Erkrankungen, die durch Milbenparasiten der Tiere hervorgerufen werden.

**Claims for the Contracting States: BE, CH/LI, DE, FR, GB, IT, LU, NL, SE**

1. In all their possible isomeric forms, the compounds of general formula (I'):

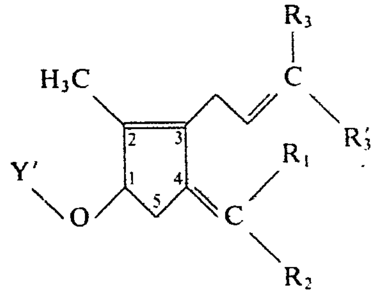

(I')

in which $R_1$ and $R_2$, being the same or different, represent a carbamoyl group or groups $R'_1$ and $R'_2$ which, being the same or different, represent a hydrogen atom, a halogen atom, an alkyl radical containing from 1 to 6 carbon atoms, an aryl radical containing from 6 to 10 carbon atoms, an aralkyl radical containing from 7 to 13 carbon atoms, an alkyloxycarbonyl group containing from 2 to 5 carbon atoms or a cyano group, $R_3$ and $R'_3$, being the same or different, represent a hydrogen atom, a halogen or a saturated or unsaturated alkyl radical, containing from 1 to 3 carbon atoms, and Y' represents either a group:

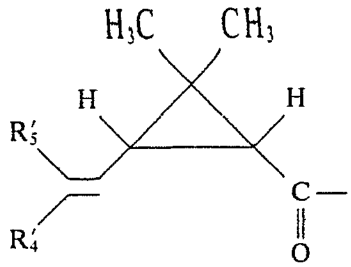

(A')

in which either $R'_4$ and $R'_5$, being the same or different, represent a hydrogen atom, an alkyl radical containing from 1 to 4 carbon atoms or a fluorine, chlorine or bromine atom, or $R'_4$ and $R'_5$ represent, together with the carbon atom to which they are bonded, a carbon homocycle or a heterocycle, containing from 3 to 7 links, these rings being possibly unsaturated, or else a heterocycle of structure:

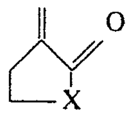

in which X represents an oxygen or sulphur atom, or one of the groups $R'_4$ or $R'_5$ represents a cyano group, a group

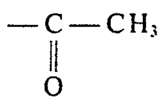 or a group 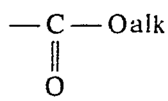

alk representing an alkyl radical containing from 1 to 4 carbon atoms when the other represents a hydrogen atom, or $R'_4$ represents a cyano group and $R'_5$ a phenyl radical, the substituents of the cyclopropane ring being capable of being of cis or trans configuration, racemic or optically-active, the double bond at position 1 of the vinyl side chain being capable of being of (E) or (Z) configuration, or Y' represents a group:

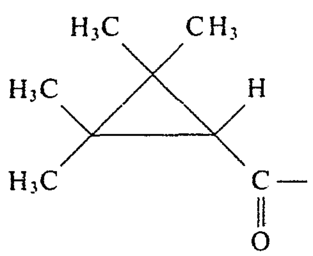

(B)

or Y' represents a group:

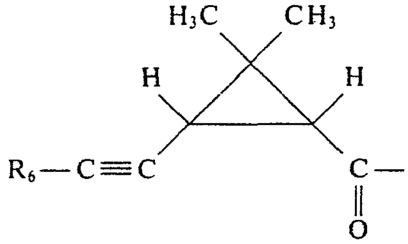

(C)

in which $R_6$ represents a hydrogen atom, an alkyl radical containing from 1 to 4 carbon atoms or an alkoxycarbonyl group containing from 2 to 5 carbon atoms, the substituents of the cyclopropane ring being capable of being of cis or trans configuration, racemic or optically-active, or Y' represents a group:

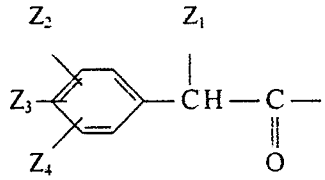

(D)

in which $Z_1$ represents an alkyl radical containing from 1 to 4 carbon atoms and $Z_2$, $Z_3$ and $Z_4$, being the same or different, represent a hydrogen atom, an alkyl radical containing from 1 to 4 carbon atoms, an alkyloxyl radical containing from 1 to 4 carbon atoms, an alkyl thio radical containing from 1 to 4 carbon atoms or a halogen atom, the asymmetric carbon of the group (D) being capable of being of S or R configuration or racemic and the asymmetric carbon at position 1 of the allethrolone derivative being capable of being of R or S configuration or racemic.

2. In all their possible isomeric forms, the compounds according to Claim 1, of general formula (I):

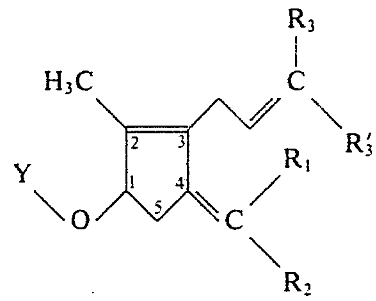

in which $R_1$, $R_2$, $R_3$ and $R'_3$ are defined as in Claim 1 and Y represents either a group:

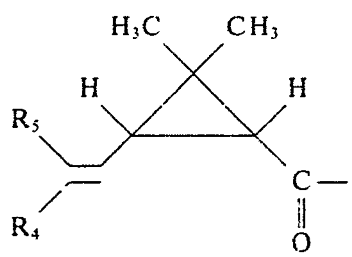

(A)

in which either $R_4$ and $R_5$, being the same or different, represent a hydrogen atom, an alkyl radical containing from 1 to 4 carbon atoms or a fluorine, chlorine or bromine atom, or $R_4$ and $R_5$ represent, together with the carbon atom to which they are bonded, a carbon homocycle or a heterocycle containing from 3 to 7 links, these rings being possibly unsaturated, or else a heterocycle of structure:

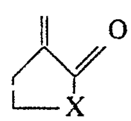

in which X represents an oxygen or sulphur atom, or one of the groups $R_4$ or $R_5$ represents a cyano group, a group

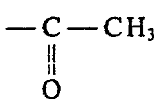 or a group 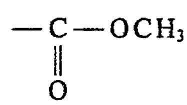

when the other represents a hydrogen atom, the substituents of the cyclopropane ring being of cis or trans configuration, racemic or optically-active, and the double bond at position 1 of the vinyl side chain being of (E) or (Z) configuration, or Y represents a group:

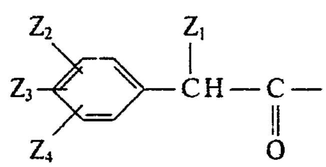

(D)

as defined in Claim 1, the asymmetric carbon at position 1 of the allethrolone derivative being capable of being of R or S configuration or racemic.

3. The compounds of general formula (I); as defined in Claim 2, in which Y represents a group:

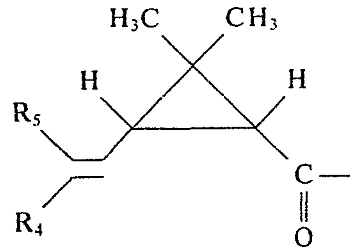

(A)

as defined in Claim 2.

4. The compounds of general formula (I), as defined in Claim 2, in which Y represents a group:

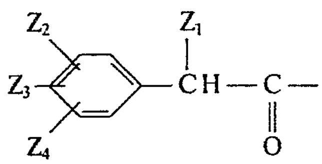

(D)

as defined in Claim 2.

5. The compounds of general formula (I), as defined in Claim 2 or 3, in which Y represents a group A, $R_1$ and $R_2$ represent a halogen atom, $R_3$ and $R'_3$ represent a hydrogen atom and $R_4$ and $R_5$ represent a halogen atom.

6. The compounds of general formula (I), as defined in Claim 2 or 3, in which Y represents a group A, $R_1$, $R_2$, $R_3$ and $R'_3$ represent hydrogen and $R_4$ and $R_5$ represent, together with the carbon atom to which they are bonded, a carbon homocycle.

7. The compounds of general formula (I), as defined in Claim 2 or 3, in which Y represents a group A, $R_1$, $R_2$, $R_3$ and $R'_3$ represent hydrogen and $R_4$ and $R_5$ represent a halogen atom.

8. Any one of the compounds of general formula (I'), of which the names are as follows:

(1S) 2-methyl 3-allyl 4-methylene cyclopent-2-en-1-yl (1R, trans) 2,2-dimethyl 3-cyclopentylidene methyl cyclopropane-1-carboxylate,

(1S) 2-methyl 3-allyl 4-methylene cyclopent-2-en-1-yl (1R, trans) 2,2-dimethyl 3-(2,2-difluoroethenyl) cyclopropane-1-carboxylate,

(1S) 2-methyl 3-allyl 4-methylene cyclopent-2-en-1-yl (1R, trans) 2,2-dimethyl 3-(2,2-dichloroethenyl) cyclopropane-1-carboxylate,

(1S) 2-methyl 3-allyl 4-methylene cyclopent-2-en-1-yl (1R, trans) 2,2-dimethyl 3-[2(Z)-cyano ethenyl] cyclopropane-1-carboxylate,

(1S) 2-methyl 3-allyl 4-methylene cyclopent-2-en-1-yl (1R, trans) 2,2-dimethyl 3-[2-fluoro 2-chloro ethenyl] cyclopropane-1-carboxylate,

(1S) 2-methyl 3-allyl 4-methylene cyclopent-2-en-1-yl (1R, trans) 2,2-dimethyl 3-cyclobutylidene methyl cyclopropane-1-carboxylate and

(1S) 2-methyl 3-allyl 4-methylene cyclopent-2-en-1-yl (1R, cis) 2,2-dimethyl 3-ethynyl cyclopropane-1-carboxylate.

9. Process for preparing the compounds of general formula (I'), as defined in Claim 1, characterized in that a compound of general formula (II), of (S) or (R) configuration or racemic:

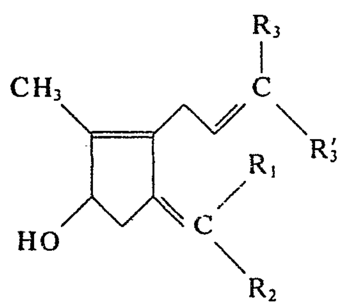

(II)

in which $R_1$, $R_2$, $R_3$ and $R'_3$ keep the meanings of Claim 1, or a functional derivative of this compound (II) is reacted with a carboxylic acid of general formula (III):

$$Y'—OH \qquad (III)$$

in which Y' keeps the meaning of Claim 1, or with a functional derivative of this acid (III).

10. Process for preparing the compounds of general formula (I'), as defined in Claim 1, characterized in that a compound of general formula (IV):

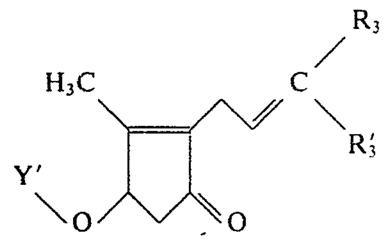

(IV)

in which $R_3$, $R'_3$ and Y' keep the meanings of Claim 1, is subjected to the action of a reagent of formula:

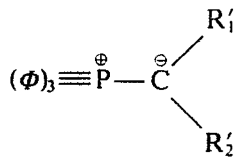

in which $R'_1$ and $R'_2$ keep the meanings of Claim 1, then, if applicable, when $R'_1$ and/or $R'_2$ represent(s) an alkyloxycarbonyl, the corresponding compound (I'), containing one or two ester groups, is reacted with ammonia, to obtain the corresponding compound of formula (I'), containing one or two carbamoyl groups.

11. Insecticidal compositions, characterized in that they contain as active substance one at least of the compounds of general formula (I') as defined in Claim 1.

12. Insecticidal compositions as defined in Claim 11, characterized in that they contain, in addition to the active principle or principles, at least one synergising agent.

13. Insecticidal compositions according to Claim 11 or 12, characterized in that they contain as active substance at least one of the compounds of general formula (I) as defined in Claim 2.

14. Insecticidal compositions according to Claim 11 or 12, characterized in that they contain as active substance at least one of the compounds defined in any one of Claims 3, 4, 5, 6 or 7.

15. Insecticidal compositions according to Claim 11 or 12, characterized in that they contain as active substance at least one of the compounds defined in Claim 8.

16. Acaricidal compositions, characterized in that they contain as active substance one at least of the compounds of general formula (I') as defined in Claim 1.

17. Nematocidal compositions, characterized in that they contain as active substance one at least of the compounds of general formula (I') as defined in Claim 1.

18. Antifungal compositions, characterized in that they contain as active substance one at least of the compounds of general formula (I') as defined in Claim 1.

19. Pharmaceutical compositions for veterinary use, used in the control of diseases caused by acaridae, characterized in that they contain as active substance one at least of the compounds of general formula (I') as defined in Claim 1.

20. Compositions intended for animal feeding, characterized in that they are constituted by a balanced, compound food for animals and in that they contain, in addition, one at least of the compounds of general formula (I') as defined in Claim 1.

21. Compositions repellent with respect to acaridae which are parasites of plants, characterized in that they contain as active principle one at least of the compounds of general formula (I') as defined in Claim 1.

22. Associations endowed with insecticidal, acaricidal, fungicidal or nematocidal activity, characterized in that they contain, as active substance, on the one hand one at least of the compounds of general formula (I'), according to Claim 1, and, on the other hand, one at least of the pyrethrinoid esters selected from the group constituted by the esters of allethrolones, of 3,4,5,6-tetrahydrophthalimido methyl alcohol, of 5-benzyl 3-furyl methyl alcohol, of 3-phenoxy benzyl alcohol and of $\alpha$-cyano 3-phenoxy benzyl alcohols of chrysanthemic acids, by the esters of 5-benzyl 3-furyl methyl alcohol of 2,2-dimethyl 3-(2-oxo 3-tetrahydrothiophenylidenmethyl) cyclopropane-1-carboxylic acids, by the esters of 3-phenoxy benzyl alcohol and of $\alpha$-cyano 3-phenoxy benzyl alcohols 2,2-dimethyl 3-(2,2-dichlorovinyl) cyclopropane-1-carboxylic acids, by the esters of $\alpha$-cyano 3-phenoxy benzyl alcohols of 2,2-dimethyl 3-(2,2-dibromovinyl) cyclopropane-1-carboxylic acids, by the esters of 3-phenoxy benzyl alcohol of 2-parachlorophenyl 2-isopropyl acetic acids, by the esters of allethrolones, of 3,4,5,6-tetrahydrophthalimidomethyl alcohol, of 5-benzyl 3-furyl methyl alcohol, of 3-phenoxy benzyl alcohol and of $\alpha$-cyano 3-phenoxy benzyl alcohols of 2,2-dimethyl 3-(1,2,2,2-tetrahaloethyl) cyclopropane-1-carboxylic acids, in which »halo« represents a fluorine, chlorine or bromine atom, it being

understood that the compounds (I') can exist in all their possible stereoisomeric forms, the same as the acid and alcohol moieties of the pyrethrinoid esters above.

**Claims for the Contracting State: AT**

1. Process for preparing the compounds, in all their possible isomeric forms, of general formula (I'):

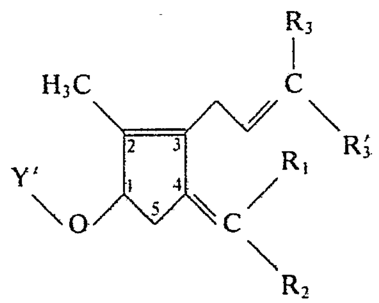

(I')

in which $R_1$ and $R_2$, being the same or different, represent a carbamoyl group or groups $R'_1$ and $R'_2$ which, being the same or different, represent a hydrogen atom, a halogen atom, an alkyl radical containing from 1 to 6 carbon atoms, an aryl radical containing from 6 to 10 carbon atoms, an aralkyl radical containing from 7 to 13 carbon atoms, an alkyloxycarbonyl group containing from 2 to 5 carbon atoms or a cyano group, $R_3$ and $R'_3$, being the same or different, represent a hydrogen atom, a halogen or a saturated or unsaturated alkyl radical, containing from 1 to 3 carbon atoms, and Y' represents either a group:

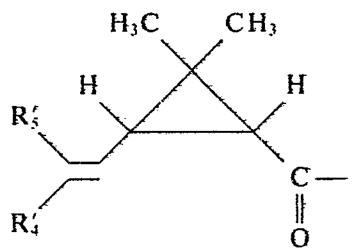

(A')

in which either $R'_4$ and $R'_5$, being the same or different, represent a hydrogen atom, an alkyl radical containing from 1 to 4 carbon atoms or a fluorine, chlorine or bromine atom, or $R'_4$ and $R'_5$ represent, together with the carbon atom to which they are bonded, a carbon homocycle or a heterocycle, containing from 3 to 7 links, these rings of being possibly unsaturated, or else a heterocycle of structure:

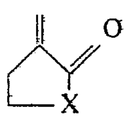

in which X represents an oxygen or sulphur atom, or one of the groups $R'_4$ or $R'_5$ represents a cyano group, a group

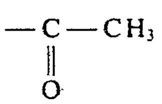 or a group 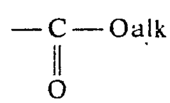

alk representing an alkyl radical containing from 1 to 4 carbon atoms when the other represents a hydrogen atom, or $R'_4$ represents a cyano group and $R'_5$ a phenyl radical, the substituents of the cyclopropane ring being capable of being of cis or trans configuration, racemic or optically-active, the double bond at position 1 of the vinyl side chain being capable of being of (E) or (Z) configuration, or Y' represents a group:

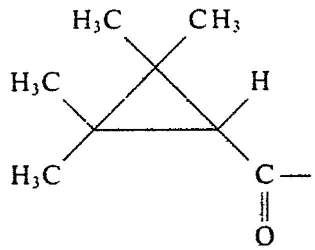

(B)

or Y' represents a group:

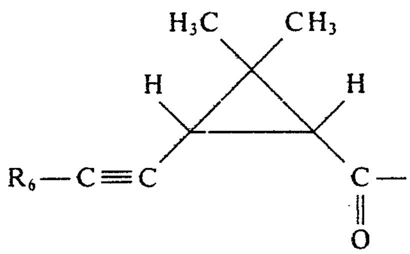

(C)

in which $R_6$ represents a hydrogen atom, an alkyl radical containing from 1 to 4 carbon atoms or an alkoxycarbonyl group containing from 2 to 5 carbon atoms, the substituents of the cyclopropane ring being capable of being of cis or trans configuration, racemic or optically-active, or Y' represents a group:

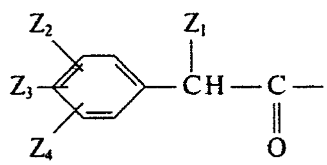

(D)

in which $Z_1$ represents an alkyl radical containing from 1 to 4 carbon atoms and $Z_2$, $Z_3$ and $Z_4$, being the same or different, represent a hydrogen atom, an alkyl radical containing from 1 to 4 carbon atoms, an alkyloxyl radical containing from 1 to 4 carbon atoms, an alkyl thio radical containing from 1 to 4 carbon atoms or a halogen atom, the asymmetric carbon of the group (D) being capable of being of S or R configuration or racemic and the asymmetric carbon at position 1 of the allethrolone derivative being capable of being of R or S configuration or racemic, characterized in that a compound of general formula (II), of (S) or (R) configuration or racemic:

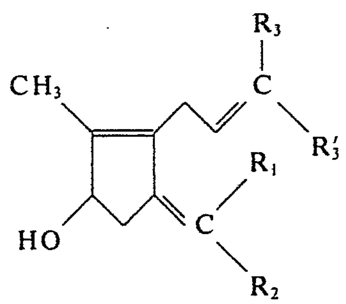

(II)

in which $R_1$, $R_2$, $R_3$ and $R'_3$ keep the aforementioned meanings or a functional derivative of this compound (II) is reacted with a carboxylic acid of general formula (III):

$$Y'—OH \qquad (III)$$

in which Y' keeps the aforementioned meaning or with a functional derivative of this acid (III).

2. Variant of the process for preparing the compounds of general formula (I'), as defined in Claim 1, characterized in that a compound of general formula (IV):

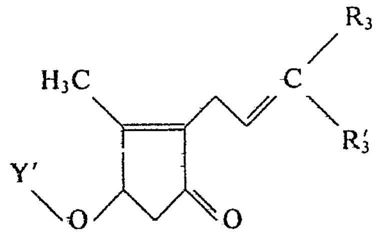

(IV)

in which $R_3$, $R'_3$ and Y' keep the meanings of Claim 1, is subjected to the action of a reagent of formula:

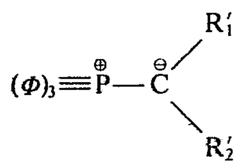

in which $R'_1$ and $R'_2$ keep the meanings of Claim 1, then, if applicable, when $R'_1$ and/or $R'_2$ represent(s) an alkyloxycarbonyl, the corresponding compound (I'), containing one or two ester groups, is reacted with ammonia, to obtain the corresponding compound of formula (I'), containing one or two carbamoyl groups.

3. Process according to Claim 1 or 2, for the preparation of the compounds, in all their possible isomeric forms, of general formula (I):

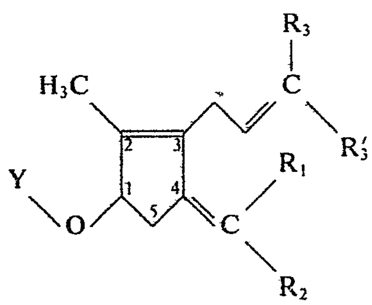

in which $R_1$, $R_2$, $R_3$ and $R'_3$ are defined as in Claim 1 and Y represents either a group:

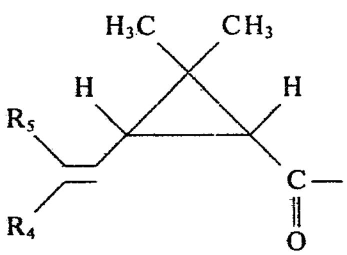

(A)

in which either $R_4$ and $R_5$, being the same or different, represent a hydrogen atom, an alkyl radical containing from 1 to 4 carbon atoms or a fluorine, chlorine or bromine atom, or $R_4$ and $R_5$ represent, together with the carbon atom to which they are bonded, a carbon homocycle or a heterocycle containing from 3 to 7 links, these rings being possibly unsaturated, or else a heterocycle of structure:

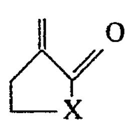

in which X represents an oxygen or sulphur atom, or one of the groups $R_4$ or $R_5$ represents a cyano group, a group

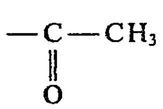 or a group 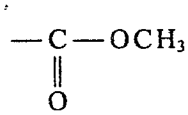

when the other represents a hydrogen atom, the substituents of the cyclopropane ring being of cis or

trans configuration, racemic or optically-active, and the double bond at position 1 of the vinyl side chain being of (E) or (Z) configuration, or Y represents a group:

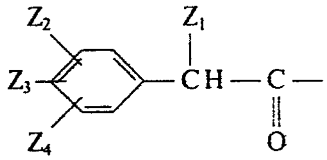

(D)

as defined in Claim 1, the asymmetric carbon at position 1 of the allethrolone derivative being capable of being of R or S configuration or racemic, characterized in that there is used to start with a compound of formula (III) or of formula (IV), in which Y' represents a group Y as defined above.

4. Process according to Claim 3, characterized in that Y represents a group:

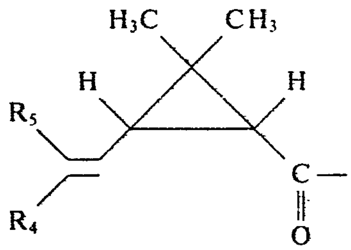

(A)

as defined in Claim 3.

5. Process according to Claim 3, characterized in that Y represents a group:

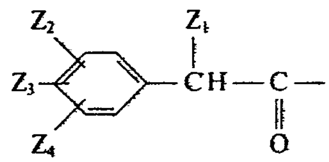

(D)

as defined in Claim 3.

6. Process according to Claim 4, characterized in that, in the group A, $R_4$ and $R_5$ represent a halogen atom and in that in the alcohol residue of the compound of formula (IV) or in the alcohol of formula (II), $R_1$ and $R_2$ represent a halogen atom and $R_3$ and $R'_3$ represent a hydrogen atom.

7. Process according to Claim 4, characterized in that, in the group A, $R_4$ and $R_5$ represent, together with the carbon atom to which they are bonded, a carbon homocycle and in that, in the alcohol residue of the compound of formula (IV) or in the alcohol of formula (II), $R_1$, $R_2$, $R_3$ and $R'_3$ represent a hydrogen atom.

8. Process according to Claim 4, characterized in that, in the group A, $R_4$ and $R_5$ represent a halogen atom and in that, in the alcohol residue of the compound of formula (IV) or in the alcohol of formula (II), $R_1$, $R_2$, $R_3$ and $R'_3$ represent a hydrogen atom.

9. Process according to Claim 3, characterized in that the starting products are selected in such a manner that any one of the compounds of general formula (I) are prepared, of which the names are as follows:

(1S) 2-methyl 3-allyl 4-methylene cyclopent-2-en-1-yl (1R, trans) 2,2-dimethyl 3-cyclopentylidene methyl cyclopropane-1-carboxylate,

(1S) 2-methyl 3-allyl 4-methylene cyclopent-2-en-1-yl (1R, trans) 2,2-dimethyl 3-(2,2-difluoroethenyl) cyclopropane-1-carboxylate,

(1S) 2-methyl 3-allyl 4-methylene cyclopent-2-en-1-yl (1R, trans) 2,2-dimethyl 3-(2,2-dichloroethenyl) cyclopropane-1-carboxylate,

(1S) 2-methyl 3-allyl 4-methylene cyclopent-2-en-1-yl (1R, trans) 2,2-dimethyl 3-[2(Z)-cyano ethenyl] cyclopropane-1-carboxylate,

(1S) 2-methyl 3-allyl 4-methylene cyclopent-2-en-1-yl (1R, trans) 2,2-dimethyl 3-[2-fluoro 2-chloro ethenyl] cyclopropane-1-carboxylate and

(1S) 2-methyl 3-allyl 4-methylene cyclopent-2-en-1-yl (1R, trans) 2,2-dimethyl 3-cyclobutylidene methyl cyclopropane-1-carboxylate.

10. Process according to Claim 1 or 2, characterized in that the starting products are selected in such a manner that (1S) 2-methyl 3-allyl 4-methylene cyclopent-2-en-1-yl (1R, cis) 2,2-dimethyl 3-ethynyl cyclopropane-1-carboxylate is prepared.

11. Insecticidal compositions, characterized in that they contain as active substanze one at least of the compounds of general formula (I') as defined in Claim 1.

12. Insecticidal compositions as defined in Claim 11, characterized in that they contain, in addition to the active principle or principles, at least one synergising agent.

13. Insecticidal compositions according to Claim 11 or 12, characterized in that they contain as active substance at least one of the compounds of general formula (I) as defined in Claim 3.

14. Insecticidal compositions according to Claim 11 or 12, characterized in that they contain as active substance at least one of the compounds defined in any one of Claims 4, 5, 6, 7 or 8.

15. Insecticidal compositions according to Claim 11 or 12, characterized in that they contain as active substance at least one of the compounds defined in Claim 9.

16. Insecticidal compositions according to Claim 11 or 12, characterized in that they contain as active substance (1S) 2-methyl 3-allyl 4-methylene cyclopent-2-en-1-yl (1R, cis) 2,2-dimethyl 3-ethynyl cyclopropane-1-carboxylate.

17. Acaricidal compositions, characterized in that they contain as active substance one at least of the compounds of general formula (I') as defined in Claim 1.

18. Nematocidal compositions, characterized in that they contain as active substance one at least of the compounds of general formula (I') as defined in Claim 1.

19. Antifungal compositions, characterized in that they contain as active substance one at least of the compounds of general formula (I') as defined in Claim 1.

20. Compositions repellent with respect to acaridae which are parasites of plants, characterized in that they contain as active principle one at least of the compounds of general formula (I') as defined in Claim 1.

21. Compositions endowed with insecticidal, acaricidal, fungicidal or nematocidal activity, characterized in that they contain, as active substance, on the one hand one at least of the compounds of general formula (I'), according to Claim 1, and, on the other hand, one at least of the pyrethrinoid esters selected from the group constituted by the esters of allethrolones, of 3,4,5,6-tetrahydrophthalimido methyl alcohol, of 5-benzyl 3-furyl methyl alcohol, of 3-phenoxy benzyl alcohol and of $\alpha$-cyano 3-phenoxy benzyl alcohols of chrystanthemic acids, by the esters of 5-benzyl 3-furyl methyl alcohol of 2,2-dimethyl 3-(2-oxo 3-tetrahydrothiophenylidenemethyl) cyclopropane-1-carboxylic acids, by the esters of 3-phenoxy benzyl alcohol and of $\alpha$-cyano 3-phenoxy benzyl alcohols 2,2-dimethyl 3-(2,2-dichlorovinyl) cyclopropane-1-carboxylic acids, by the esters of $\alpha$-cyano 3-phenoxy benzyl alcohols of 2,2-dimethyl 3-(2,2-dibromovinyl) cyclopropane-1-carboxylic acids, by the esters of 3-phenoxy benzyl alcohol of 2-parachlorophenyl 2-isopropyl acetic acids, by the esters of allethrolones, of 3,4,5,6-tetrahydrophthalimidomethyl alcohol, of 5-benzyl 3-furyl methyl alcohol, of 3-phenoxy benzyl alcohol and of $\alpha$-cyano 3-phenoxy benzyl alcohols of 2,2-dimethyl 3-(1,2,2,2-tetrahaloethyl) cyclopropane-1-carboxylic acids, in which »halo« represents a fluorine, chlorine or bromine atom, it being understood that the compounds (I') can exist in all their possible stereoisomeric forms, the same as the acid and alcohol moieties of the pyrethrinoid esters above.

22. Compositions used in the control of diseases caused by acaridae which are parasites of animals, characterized in that they contain, as active substance, at least one of the compounds of general formula (I') as defined in Claim 1.

23. Use of the compounds of general formula (I') as defined in Claim 1, as insecticidal, acaricidal, nematocidal and antifungal agents.

24. Use of the compounds of general formula (I') as defined in Claim 1 in the control of diseases caused by acaridae which are parasites of animals.